(19) Europäisches Patentamt
European Patent Office
Office européen des brevets

(11) EP 4 393 924 A1

(12) EUROPEAN PATENT APPLICATION
published in accordance with Art. 153(4) EPC

(43) Date of publication:
03.07.2024 Bulletin 2024/27

(21) Application number: 22933129.3

(22) Date of filing: 29.11.2022

(51) International Patent Classification (IPC):
*C07D 487/04 (2006.01)* *A61K 31/52 (2006.01)*
*A61P 35/00 (2006.01)*

(52) Cooperative Patent Classification (CPC):
C07D 487/04; A61K 31/4985; A61K 31/52;
A61P 35/00; C07D 519/00

(86) International application number:
PCT/CN2022/135152

(87) International publication number:
WO 2023/179078 (28.09.2023 Gazette 2023/39)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 25.03.2022 CN 202210307006

(71) Applicant: Tencent Technology (Shenzhen) Company Limited
Shenzhen, Guangdong 518000 (CN)

(72) Inventors:
- XUE, Ding
Shenzhen, Guangdong 518057 (CN)
- XU, Tingyang
Shenzhen, Guangdong 518057 (CN)
- HUANG, Junhong
Shenzhen, Guangdong 518057 (CN)
- LIU, Wei
Shenzhen, Guangdong 518057 (CN)

(74) Representative: Nederlandsch Octrooibureau
P.O. Box 29720
2502 LS The Hague (NL)

# (54) IMIDAZO [1,2-A] PYRAZINE OR PYRAZOLO [1,5-A] PYRIMIDINE DERIVATIVE AND USE THEREOF

(57) This application provides a compound. The compound is a compound as shown in Formula (I) or a stereoisomer, tautomer, deuterated compound, oxynitride, solvate, metabolite, pharmaceutically acceptable salt or prodrug of the compound having a structure as shown in Formula (I).

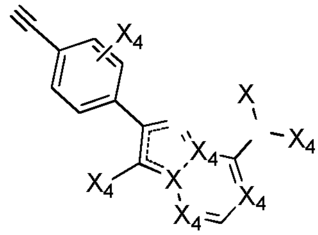

(I)

EP 4 393 924 A1

## Description

FIELD OF THE TECHNOLOGY

This application relates to the field of medicines, and in particular, this application relates to an imidazo[1,2-a]pyrazine or pyrazolo[1,5-a]pyrimidine derivative as an LSD1 inhibitor and use thereof.

BACKGROUND OF THE DISCLOSURE

Lysine specific demethylase 1 (LSD1) is a member of the monoammonia oxidase family, which utilizes flavin adenine dinucleotide (FAD) as a cofactor for catalysis of a demethylation reaction. LSD1 can be used for catalyzing demethylation of H3K4me1/2 and H3K9me1/2, so as to regulate a gene transcription process.

Under normal physiological conditions, an epigenetic system in cells normally regulates self-renewal, differentiation, proliferation and other physiological processes of the cells. However, in physiological processes of a variety of cancers, the system has dysfunction, and as a result, proliferation of cancer cells and other physiological processes are promoted.

It has been found that LSD1 has dysfunction and overexpression in a variety of cancers, including acute myeloid leukemia, small cell lung cancer, breast cancer, colorectal cancer and the like. In particular, the cancer types with differentiation retardation are involved. However, when LSD1 is knocked out, the effects of inducing differentiation and inhibiting proliferation in a variety of cancer subtypes are achieved. Therefore, LSD1 inhibitors have good clinical potential for the treatment of a variety of cancers.

The LSD1 inhibitors in early study generally have a phenylcyclopropylamine structure, and can undergo covalent binding to the flavin adenine dinucleotide (FAD) in an amine oxidase domain (AO) of LSD1, so as to irreversibly inhibit functions of the enzyme. However, due to critical effects of LSD1 in hematopoietic and embryonic stem cells, the irreversible LSD1 inhibitors may have certain target related toxicity. At present, clinical study has been carried out on at least 5 irreversible LSD1 inhibitors (including INCB-59872, Bomedemstat, GSK-2879552, Iadademstat, and Vafidemstat), which are used for treating cancers such as small cell lung cancer and acute myeloid leukemia alone or in combination with other medicines. As mentioned above, the covalent binding of the irreversible LSD1 inhibitors to the FAD is likely to affect the embryonic stem cells and hematopoietic functions, thereby resulting in target related side effects.

Therefore, the current LSD1 inhibitors still need to be further improved.

SUMMARY

This application is accomplished based on the findings and understandings of the following facts and problems by the inventor.

This application provides a reversible LSD1 inhibitor having a novel structure, which has higher LSD1 inhibitory activity and lower hERG inhibitory activity.

This application provides a compound. The compound is a compound as shown in Formula (I) or a stereoisomer, tautomer, deuterated compound, oxynitride, solvate, metabolite, pharmaceutically acceptable salt or prodrug of the compound having a structure as shown in Formula (I).

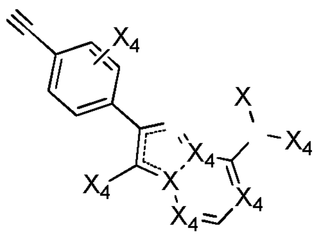

(I)

where X is C, N, or O;

$X_1$, $X_2$, $X_3$, and $X_4$ are respectively selected from C or N, provided that $X_1$ is different from $X_2$, $X_1$ is different from $X_4$, and $X_2$ is different from $X_3$;

$R_1$ and $R_2$ are independently H, deuterium, $C_{1-6}$ alkyl, $C_{3-12}$ carbocyclyl, $C_{3-12}$ carbocyclyl-$C_{1-4}$ alkylene, heterocyclyl composed of 3-12 atoms, (heterocyclyl composed of 3-12 atoms)-$C_{1-4}$ alkylene, $C_{6-10}$ aryl, $C_{6-10}$ aryl-$C_{1-4}$ alkylene, or heteroaryl composed of 5-14 atoms, (heteroaryl composed of 5-14 atoms)-$C_{1-4}$ alkylene, or $R_1$, $R_2$ and X connected thereto form a $C_{3-12}$ carbon ring or a heterocyclic ring composed of 3-12 atoms, where the $C_{1-6}$ alkyl, the $C_{3-12}$ carbocyclyl, the $C_{3-12}$ carbocyclyl-$C_{1-4}$ alkylene, the heterocyclyl composed of 3-12 atoms, the (heterocyclyl composed of 3-12 atoms)-$C_{1-4}$ alkylene, the $C_{6-10}$ aryl, the $C_{6-10}$ aryl-$C_{1-4}$ alkylene, the heteroaryl composed of 5-14 atoms, the (heteroaryl composed of 5-14 atoms)-$C_{1-4}$ alkylene, or the $C_{3-12}$ carbon ring or the heterocyclic ring composed of 3-12 atoms formed by $R_1$, $R_2$ and X connected thereto are independently unsubstituted or substituted with 1, 2, 3, 4, or 5 R';

$R_3$ is $C_{6-10}$ aryl or heteroaryl composed of 5-10 atoms, where the $C_{6-10}$ aryl and the heteroaryl composed of 5-10 atoms are independently unsubstituted or substituted with 1, 2, 3, or 4 R";

$R_4$ is H, deuterium, F, Cl, Br, CN, $NO_2$, $-OR^a$, $-NR^bR^c$, or $C_{1-6}$ alkyl;

R' and R" are independently H, deuterium, F, Cl, Br, CN, $NO_2$, =O, $-OR^a$, $-NR^bR^c$, $-S(=O)_2R^a$, $C_{1-6}$ alkyl, $C_{3-8}$ cycloalkyl, $C_{3-8}$ cycloalkenyl, or heterocyclyl composed of 3-12 atoms, where the $C_{1-6}$ alkyl, the $C_{3-8}$ cycloalkyl, the $C_{3-8}$ cycloalkenyl and the heterocyclyl composed of 3-12 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-C0-NR^bR^c$; and

$R^a$, $R^b$, and $R^c$ are independently H, deuterium, $C_{1-6}$ alkyl, $C_{1-6}$ haloalkyl, or heterocyclyl composed of 3-6 atoms, or $R^b$, $R^c$ and nitrogen atoms connected thereto form a heterocyclic ring composed of 3-6 atoms, where the $C_{1-6}$ alkyl and the heterocyclic ring composed of 3-6 atoms are independently unsubstituted or substituted with 1, 2, 3, or 4 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

In some other embodiments, the compound is a compound as shown in Formula (I) or a stereoisomer, tautomer, deuterated compound, oxynitride, solvate, metabolite, pharmaceutically acceptable salt or prodrug of the compound having a structure as shown in Formula (I):

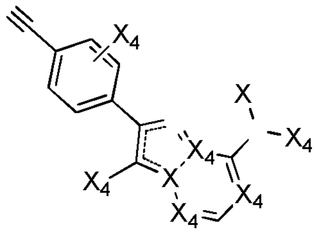

(I)

where X is C, N, or O;

$X_1$, $X_2$, $X_3$, and $X_4$ are respectively selected from C or N, provided that $X_1$ is different from $X_2$, $X_1$ is different from $X_4$, and $X_2$ is different from $X_3$;

$R_1$ and $R_2$ are independently H, deuterium, $C_{1-6}$ alkyl, $C_{3-12}$ carbocyclyl, $C_{3-12}$ carbocyclyl-$C_{1-4}$ alkylene, heterocyclyl composed of 3-12 atoms, (heterocyclyl composed of 3-12 atoms)-$C_{1-4}$ alkylene, $C_{6-10}$ aryl, $C_{6-10}$ aryl-$C_{1-4}$ alkylene, heteroaryl composed of 5-14 atoms, (heteroaryl composed of 5-14 atoms)-$C_{1-4}$ alkylene, or $R_1$, $R_1$ and X connected thereto form a $C_{3-12}$ carbon ring or a heterocyclic ring composed of 3-12 atoms, where the $C_{1-6}$ alkyl, the $C_{3-12}$ carbocyclyl, the $C_{3-12}$ carbocyclyl-$C_{1-4}$ alkylene, the heterocyclyl composed of 3-12 atoms, the (heterocyclyl composed of 3-12 atoms)-$C_{1-4}$ alkylene, the $C_{6-10}$ aryl, the $C_{6-10}$ aryl-$C_{1-4}$ alkylene, the heteroaryl composed of 5-14 atoms, the (heteroaryl composed of 5-14 atoms)-$C_{1-4}$ alkylene, or the $C_{3-12}$ carbon ring or the heterocyclic ring composed of 3-12 atoms formed by $R_1$, $R_2$ and X connected thereto are independently unsubstituted or substituted with 1, 2, 3, 4, or 5 R';

$R_3$ is $C_{6-10}$ aryl or heteroaryl composed of 5-10 atoms, where the $C_{6-10}$ aryl and the heteroaryl composed of 5-10 atoms are independently unsubstituted or substituted with 1, 2, 3, or 4 R";

$R_4$ is H, deuterium, F, Cl, Br, CN, $NO_2$, $-OR^a$, $-NR^bR^c$, or $C_{1-6}$ alkyl;

R' and R" are independently H, deuterium, F, Cl, Br, CN, $NO_2$, =O, $-OR^a$, $-NR^bR^c$, $-S(=O)_2R^a$, $C_{1-6}$ alkyl, $C_{3-8}$ cycloalkyl, $C_{3-8}$ cycloalkenyl, heterocyclyl composed of 3-12 atoms, where the $C_{1-6}$ alkyl, the $C_{3-8}$ cycloalkyl, the $C_{3-8}$ cycloalkenyl and the heterocyclyl composed of 3-12 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$; and

$R^a$, $R^b$, and $R^c$ are independently H, deuterium, $C_{1-6}$ alkyl, $C_{1-6}$ haloalkyl, heterocyclyl composed of 3-6 atoms, or $R^b$, $R^c$ and nitrogen atoms connected thereto form a heterocyclic ring composed of 3-6 atoms, where the $C_{1-6}$ alkyl and the heterocyclic ring composed of 3-6 atoms are independently unsubstituted or substituted with 1, 2, 3, or 4 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

In some other embodiments, this application relates to a stereoisomer, tautomer, deuterated compound, oxynitride, solvate, metabolite, pharmaceutically acceptable salt or prodrug of a compound having a structure as shown in Formula (II) or (III):

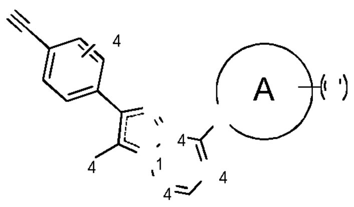

(II),

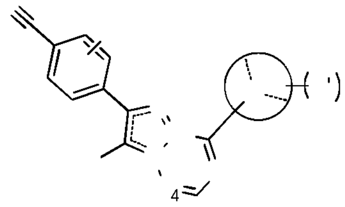

(III),

where ring A is a $C_{3-6}$ carbon ring or a heterocyclic ring composed of 3-6 atoms;
ring B is a bridge ring composed of 6-8 atoms;
m is 0, 1, 2, 3, 4, or 5; and
X, $X_1$, $X_2$, $X_3$, $X_4$, $R_3$, $R_4$, and R' are defined as described in this application.

In some other embodiments, this application relates to a stereoisomer, tautomer, deuterated compound, oxynitride, solvate, metabolite, pharmaceutically acceptable salt or prodrug of a compound having a structure as shown in Formula (IV), Formula (V), Formula (VI), or Formula (VII):

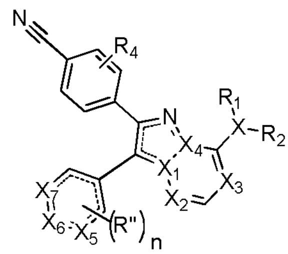

(IV),

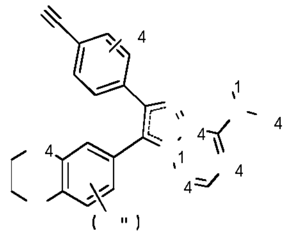

(V),

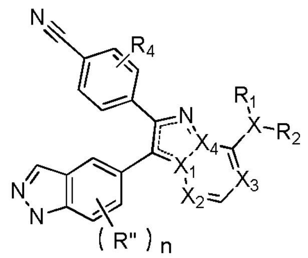

(VI),

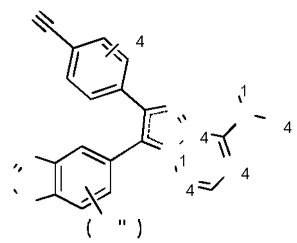

(VII),

where $X_5$ is empty or C; $X_6$ and $X_7$ are independently selected from C or N respectively; $X_8$ is O, S, C, or N;
n is 0, 1, 2, 3, or 4; and
X, $X_1$, $X_2$, $X_3$, $X_4$, $R_1$, $R_2$, $R_4$, and R" are defined as described in this application.

In some other embodiments, this application relates to a stereoisomer, tautomer, deuterated compound, oxynitride, solvate, metabolite, pharmaceutically acceptable salt or prodrug of a compound having a structure as shown in Formula (VIII) or Formula (IX):

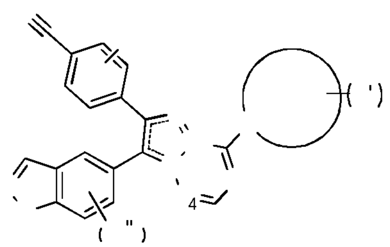

(VIII),

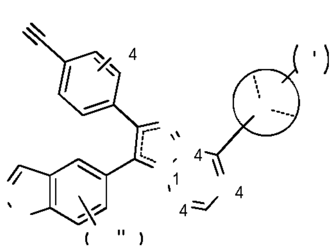

(IX),

where ring A is a $C_{3-6}$ carbon ring or a heterocyclic ring composed of 3-6 atoms;
ring B is a bridge ring composed of 6-8 atoms;
m is 0, 1, 2, 3, 4, or 5;
n is 1, 2, 3, or 4;
R" is $C_{1-6}$ alkyl or $C_{3-8}$ cycloalkyl, where the $C_{1-6}$ alkyl and the $C_{3-8}$ cycloalkyl are independently unsubstituted or substituted with 1, 2, 3, or 4 substituents, and the substituents are independently deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$; and
X, $X_1$, $X_2$, $X_3$, $X_4$, $R_4$, and R' are defined as described in this application.

In some other embodiments, this application relates to a stereoisomer, tautomer, deuterated compound, oxynitride, solvate, metabolite, pharmaceutically acceptable salt or prodrug of a compound having a structure as shown in Formula (VIII) or Formula (IX):

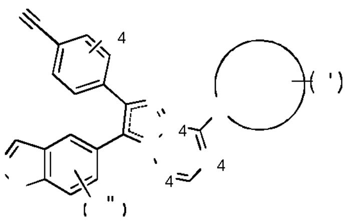 (VIII), 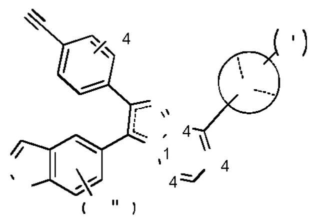 (IX),

where ring A is a $C_{3-6}$ carbon ring or a heterocyclic ring composed of 3-6 atoms;
ring B is a bridge ring composed of 6-8 atoms;
m is 0, 1, 2, 3, 4, or 5;
n is 1, 2, 3, or 4;
R" is $C_{1-6}$ alkyl or $C_{3-8}$ cycloalkyl, where the $C_{1-6}$ alkyl and the $C_{3-8}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$; and
X, $X_1$, $X_2$, $X_3$, $X_4$, $R_4$, and R' are defined as described in this application.

In some other embodiments, this application relates to a stereoisomer, tautomer, deuterated compound, oxynitride, solvate, metabolite, pharmaceutically acceptable salt or prodrug of a compound having a structure as shown in Formula (X) or Formula (XI):

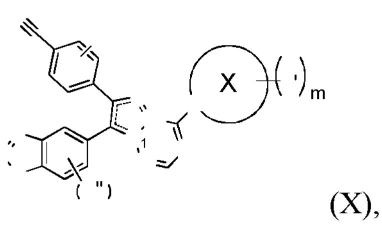 (X), 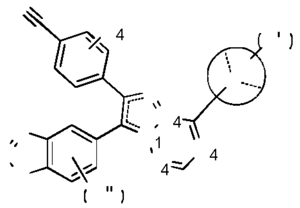 (XI),

where ring A is a $C_{3-6}$ carbon ring or a heterocyclic ring composed of 3-6 atoms;
ring B is a bridge ring composed of 6-8 atoms;
m is 0, 1, 2, 3, 4, or 5; and
n is 0, 1, 2, 3, or 4.
X, $X_1$, $X_2$, $X_3$, $X_4$, $R_4$, R', and R" are defined as described in this application.

In some other embodiments, X is N.

In some other embodiments, $R_1$ and $R_2$ are independently H, deuterium, $C_{1-6}$ alkyl, $C_{3-6}$ carbocyclyl, $C_{3-6}$ carbocyclyl-$C_{1-4}$ alkylene, heterocyclyl composed of 3-6 atoms, (heterocyclyl composed of 3-6 atoms)-$C_{1-4}$ alkylene, $C_{6-8}$ aryl, $C_{6-8}$ aryl-$C_{1-4}$ alkylene, heteroaryl composed of 5-8 atoms, or (heteroaryl composed of 5-8 atoms)-$C_{1-4}$ alkylene, or $R_1$, $R_2$ and X connected thereto form a $C_{3-8}$ carbon ring or a heterocyclic ring composed of 3-8 atoms; and where the $C_{1-6}$ alkyl, the $C_{3-6}$ carbocyclyl, the $C_{3-6}$ carbocyclyl-$C_{1-4}$ alkylene, the heterocyclyl composed of 3-6 atoms, the (heterocyclyl composed of 3-6 atoms)-$C_{1-4}$ alkylene, the $C_{6-8}$ aryl, the $C_{6-8}$ aryl-$C_{1-4}$ alkylene, the heteroaryl composed of 5-8 atoms, the (heteroaryl composed of 5-8 atoms)-$C_{1-4}$ alkylene, or the $C_{3-8}$ carbon ring or the heterocyclic ring composed

of 3-8 atoms formed by $R_1$, $R_2$ and X connected thereto are independently unsubstituted or substituted with 1, 2, or 3 R'.

In some other embodiments, $R_3$ is $C_{6-9}$ aryl or heteroaryl composed of 5-9 atoms, where the $C_{6-9}$ aryl and the heteroaryl composed of 5-9 atoms are independently unsubstituted or substituted with 1 or 2 R".

In some other embodiments, $R_4$ is H, deuterium, F, Cl, Br, CN, $NO_2$, -OH, $-NH_2$, or $C_{1-3}$ alkyl.

In some other embodiments, R' is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, or heterocyclyl composed of 3-6 atoms, where the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

In some other embodiments, R" is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, or heterocyclyl composed of 3-6 atoms, where the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

In some other embodiments, R" is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, or heterocyclyl composed of 3-6 atoms, where the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, R" is $C_{1-3}$ alkyl or $C_{3-6}$ cycloalkyl, where the $C_{1-3}$ alkyl and the $C_{3-6}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

In some other embodiments, R" is $C_{1-3}$ alkyl or $C_{3-6}$ cycloalkyl. where the $C_{1-3}$ alkyl and the $C_{3-6}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, $R_1$ and $R_2$ are independently H, deuterium, $C_{1-6}$ alkyl, $C_{3-6}$ carbocyclyl, or heterocyclyl composed of 3-6 atoms, or $R_1$, $R_2$ and X connected thereto form a $C_{3-8}$ carbon ring or a heterocyclic ring composed of 3-8 atoms, where the $C_{1-6}$ alkyl, the $C_{3-6}$ carbocyclyl, the heterocyclyl composed of 3-6 atoms, or the $C_{3-8}$ carbon ring or the heterocyclic ring composed of 3-8 atoms formed by $R_1$, $R_2$ and X connected thereto are independently unsubstituted or substituted with 1, 2, or 3 R'.

In some other embodiments, $R_3$ is phenyl, naphthyl, pyrrolyl, pyridyl, pyrazolyl, imidazolyl, triazolyl, tetrazolyl, oxazolyl, oxadiazolyl, 1,3,5-triazinyl, thiazolyl, thienyl, pyrazinyl, pyridazinyl, pyrimidinyl, indolyl, purinyl, quinolinyl, isoquinolinyl, or phenothianyl, where the phenyl, the naphthyl, the pyrrolyl, the pyridyl, the pyrazolyl, the imidazolyl, the triazolyl, the tetrazolyl, the oxazolyl, the oxadiazolyl, the 1,3,5-triazinyl, the thiazolyl, the thienyl, the pyrazinyl, the pyridazinyl, the pyrimidinyl, the indolyl, the purinyl, the quinolinyl, the isoquinolinyl and the phenothianyl are independently unsubstituted or substituted with 1 or 2 R".

In some other embodiments, $R_4$ is F, Cl, or Br.

In some other embodiments, R' is independently H, deuterium, F, Cl, Br, or $-NR^bR^c$.

In some other embodiments, R" is independently H, deuterium, F, Cl, Br, CN, $NO_2$, =O, $-OR^a$, $-NR^bR^c$, $C_{1-3}$ alkyl, or $C_{3-6}$ cycloalkyl, where the $C_{1-3}$ alkyl and the $C_{3-6}$ cycloalkyl are independently unsubstituted or substituted with 1, 2, or 3 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

In some other embodiments, R" is independently H, deuterium, F, Cl, Br, CN, $NO_2$, =O, $-OR^a$, $-NR^bR^c$, $C_{1-3}$ alkyl, or $C_{3-6}$ cycloalkyl, where the $C_{1-3}$ alkyl and the $C_{3-6}$ cycloalkyl are independently unsubstituted or substituted with 1, 2, or 3 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, R" is $C_{1-3}$ alkyl or $C_{3-6}$ cycloalkyl, where the $C_{1-3}$ alkyl and the $C_{3-6}$ cycloalkyl are independently unsubstituted or substituted with 1 or 2 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

In some other embodiments, R" is $C_{1-3}$ alkyl or $C_{3-6}$ cycloalkyl, where the $C_{1-3}$ alkyl and the $C_{3-6}$ cycloalkyl are independently unsubstituted or substituted with 1 or 2 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, $R^a$, $R^b$, and $R^c$ are independently H, deuterium, methyl, ethyl, isopropyl, n-propyl, n-butyl, tert-butyl, $C_{1-3}$ haloalkyl, or heterocyclyl composed of 3-6 atoms, or $R^b$, $R^c$ and nitrogen atoms connected thereto form a heterocyclic ring composed of 3-6 atoms, where the methyl, the ethyl, the isopropyl, the n-propyl, the n-butyl, the tert-butyl and the heterocyclic ring composed of 3-6 atoms are independently unsubstituted or substituted with 1, 2, or 3 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

In some other embodiments, this application relates to a pharmaceutical composition, which includes an effective amount of the compound as described above.

In some other embodiments, the pharmaceutical composition further includes: a pharmaceutically acceptable carrier, an adjuvant, a medium or a combination thereof.

In some other embodiments, the pharmaceutical composition further includes one or more therapeutic agents, and the therapeutic agent is selected from other anti-tumor drugs.

In some other embodiments, the therapeutic agent is an anti-mitotic drug, an alkylating agent, an anti-metabolic drug, a topoisomerase inhibitor, an estrogen receptor regulator, an androgen receptor regulator, a small-molecule inhibitor targeting protein kinase, and an antibody drug targeting protein kinase.

In some other embodiments, the anti-mitotic drug is paclitaxel, vincristine.

In some other embodiments, the alkylating agent is cisplatin, oxaliplatin, carboplatin, or cyclophosphamide.

In some other embodiments, the anti-metabolic drug is gemcitabine, 5-fluorouracil, or methotrexate.

In some other embodiments, the topoisomerase inhibitor is epipodophyllotoxin, etoposide, topotecan, or camptothecin.

In some other embodiments, the estrogen receptor regulator is tamoxifen or fulvestrant.

In other embodiments, the androgen receptor regulator is bicalutamide.

In some other embodiments, the small-molecule inhibitor targeting protein kinase is dasatinib, bosutinib, gefitinib, erlotinib, lapatinib, imatinib, nilotinib, sorafenib, tipifarnib, sunitinib, axitinib.

In some other embodiments, the antibody drug targeting protein kinase is trastuzumab, panitumumab, cetuximab.

In some other embodiments, this application relates to use of the compound as described above or the pharmaceutical composition as described above in preparation of a medicine, and the medicine is used for preventing, disposing, treating, or alleviating a disease associated with overexpression or hyperactivity of LSD1 in a patient.

In some other embodiments, this application relates to a compound as described above or a pharmaceutical composition as described above, which is used for preventing, disposing, treating, or alleviating a disease associated with overexpression or hyperactivity of LSD1 in a patient.

In some other embodiments, this application relates to a method for preventing, disposing, treating, or alleviating a disease associated with overexpression or hyperactivity of LSD1 in a patient. According to an embodiment of this application, the method includes administering a pharmacologically acceptable amount of the compound as described above or the pharmaceutical composition as described above to the patient.

In some other embodiments, the disease associated with overexpression or hyperactivity of LSD1 is a tumor.

In some other embodiments, the tumor is papillary thyroid carcinoma, breast cancer, gastric cancer, bronchogenic carcinoma, lung cancer, acute myeloid leukemia, small cell lung cancer, prostate cancer, or non-hodgkin lymphoma.

In some other embodiments, this application relates to use of the compound as described above or the pharmaceutical composition as described above in preparation of a medicine, and the medicine is used for inhibiting LSD1.

In some other embodiments, this application relates to a compound as described above or a pharmaceutical composition as described above, which is used for inhibiting LSD1. In some other embodiments, this application relates to a method for inhibiting LSD1. According to an embodiment of this application, the method includes administering the compound described above or the pharmaceutical composition as described above to a cell to be treated, where the cell to be treated expresses LSD1.

Unless otherwise specified, all stereoisomers, geometric isomers, tautomers, solvates, hydrates, metabolites, salts and pharmacologically acceptable prodrugs of the compound of this application are included in this application.

In some embodiments, the salt refers to a pharmaceutically acceptable salt. The term "pharmaceutically acceptable" refers to that a substance or composition is chemically and/or toxicologically compatible with other ingredients of a preparation and/or mammals receiving treatment with the same.

The compound of this application further includes a salt form thereof. The salt may not be a pharmaceutically acceptable salt, but may be used for preparing and/or purifying the compound of this application and/or used for separating an intermediate of an enantiomer of the compound of this application.

The compound of this application, including a salt thereof, may also be obtained in a hydrate form thereof, or may include other solvents for crystallization. The compound of this application may be inherently or designed to form a solvate having a medicinal solvent (including water). Therefore, this application further includes a solvated form and an unsolvated form thereof.

In another aspect, the compound of this application may include several asymmetrical centers or a racemic mixture form commonly described. The application further includes racemic mixtures, partial racemic mixtures as well as separated enantiomers and diastereomers.

The compound of this application may exist in the form of one or a mixture of possible isomers, rotamers, atropisomers and tautomers. This application may further include mixtures of isomers, rotamers, atropisomers and tautomers of the compound of this application, or partial mixtures of isomers, rotamers, atropisomers and tautomers, or

separated isomers, rotamers, atropisomers and tautomers.

In another aspect, this application relates to a method for preparing, separating and purifying the compound as shown in Formula (I).

The contents described above merely describe certain aspects of this application, but this application is not limited thereto. The contents in these aspects and other aspects will be more specifically and completely described below.

**Definitions and general terms**

Some embodiments of this application are now described in detail, and examples are illustrated by the attached structural formulas and chemical formulas. This application is intended to include all substitutions, modifications and equivalents of technical solutions, which are all included in the scope of this application as defined by the claims. It is understandable for a person skilled in the art that various methods and materials similar or equivalent to those described in this application can be used for practicing this application. This application is certainly not limited to the methods and materials described herein. This application prevails in a case that one or more of combined documents, patents and similar materials are different from or contradictory with this application (including, but not limited to, terms defined, application of the terms, technologies described and the like).

It is to be further understood that some features of this application are described in a variety of independent embodiments for clarity, and may also be provided in a combination form in individual embodiments. On the contrary, various features of this application are described in individual embodiments for clarity, and may also be provided separately or in any suitable sub-combination.

Unless otherwise indicated, all technical terms and scientific terms used in this application have the same meaning as commonly understood by a person skilled in the art to which this application belongs. Unless otherwise indicated, all patent publications referenced in the disclosure of the entire content of this application are incorporated into this application as an entirety by reference.

Unless otherwise indicated, the following definitions are applied in this application. According to purposes of this application, chemical elements are defined according to the Periodic Table of Elements (CAS edition) and the Chemical Handbook, 75, thEd, 1994. In addition, general principles of organic chemistry can refer to "Organic Chemistry", Thomas Sorrell, University Science Books, Sausalito: 1999, and "March's Advanced Organic Chemistry", by Michael B. Smith and Jerry March, John Wiley & Sons, New York: 2007. Therefore, reference documents are incorporated into the entire content of this application.

The term "subject" used in this application refers to animals. Typically, the animals are mammals. The subject also refers to primates (such as humans), cattle, sheep, goats, horses, dogs, cats, rabbits, rats, mice, fishes, birds and the like. In some embodiments, the subject is a primate. In other embodiments, the subject is a human.

The terms "curee" and "patient" used in this application may be used interchangeably. The terms "curee" and "patient" refer to animals (such as chickens, quails or turkeys and other birds or mammals), particularly are "mammals" including non-primates (such as cows, pigs, horses, sheep, rabbits, guinea pigs, rats, cats, dogs and mice) and primates (such as monkeys, chimpanzees and humans), and more specifically are humans. In one embodiment, the curee is a non-human animal, such as a domestic animal (such as horse, cow, pig or sheep) or a pet (such as dog, cat, guinea pig or rabbit). In other embodiments, the "patient" is a human.

Stereochemical definitions and conventions used in this application are generally in accordance with S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compound of this application may contain an asymmetric center or a chiral center, thus existing in different stereoisomer forms. It is expected that all the stereoisomers of the compound of this application, including but not limited to diastereoisomers, enantiomers, atropisomers and mixtures thereof such as racemic mixtures, are also included in the scope of this application. A variety of organic compounds exist as having optical activity, namely, having the ability to rotate a plane of a plane-polarized light. In response to describing a compound having optical activity, prefixes D and L or *R* and *S* are used for indicating absolute configurations of a molecule based on a chiral center (or a plurality of chiral centers) in the molecule. The prefixes *d* and *l* or (+) and (-) are symbols used for specifying a compound capable of rotating a plane-polarized light, where (-) or *l* indicates that a compound is levorotatory. The prefix (+) or *d* indicates that a compound is dextrotatory. As for a given chemical structure, the stereoisomers are identical except that they are mirror images of each other. Specific stereoisomers may also be called enantiomers, and a mixture of the isomers is usually called a mixture of the enantiomers. A 50:50 mixture of enantiomers is called a racemic mixture or a racemate, which may exist when a chemical reaction or method has no stereoselectivity or stereospecificity.

Based on the selection of raw materials and methods, the compound of this application may exist in the form of one or a mixture of possible isomers, such as a pure optical isomer, or a mixture of isomers such as a mixture of a racemate and a diastereoisomer, depending on the number of asymmetric carbon atoms. An optically active (*R*)- or (*S*)-isomer may be prepared by using a chiral synthon or chiral preparation, or separated by using conventional technologies. When the compound contains a double bond, a substituent may be in an *E* or *Z* configuration. When the compound contains a disubstituted cycloalkyl, a substituent of the cycloalkyl may be in a *cis*- or *trans*- configuration.

The compound of this application may contain an asymmetric center or a chiral center, thus existing in different stereoisomer forms. It is expected that all the stereoisomers of the compound of this application, including but not limited to diastereoisomers, enantiomers, atropisomers, geometric (or conformational) isomers and mixtures thereof such as racemic mixtures, are included in the scope of this application.

Unless otherwise indicated, a structure described in this application further includes all isomers of the structure (such as enantiomers, diastereoisomers, atropisomers and geometric (or conformational) isomers); for example, *R* and *S* configurations of various asymmetrical centers, (*Z*) and (*E*) double bond isomers, and (*Z*) and (*E*) conformational isomers. Therefore, all individual stereochemical isomers and mixtures of enantiomers, mixtures of diastereomers and mixtures of geometric isomers (or conformational isomers) of the compound of this application are included in the scope of this application.

The term "tautomers" or "tautomeric forms" refer to structural isomers which have different energies and can be converted to each other by a low energy barrier. When tautomerism is possible (such as in a solution), chemical equilibrium of the tautomers can be achieved. For example, protontautomers (also known as prototropic tautomers) are converted to each other by proton transfer, such as keto-enol isomerization and imine-enamine isomerization. Valence tautomers are converted to each other by recombination of some bonding electrons. A specific example of the keto-enol tautomerism is tautomerism of pentane-2,4-dione and 4-hydroxypent-3-en-2-one tautomers. Another example of tautomerism is phenol-keto tautomerism. A specific example of the phenol-keto tautomerism is tautomerism of pyridin-4-ol and pyridin-4(1*H*)-one tautomers. Unless otherwise indicated, all tautomeric forms of the compound of this application are included in the scope of this application.

The term "deuterated compound" used in this application refers to a compound generated after one or more hydrogens of a compound are substituted with $^2H$.

The term "oxynitride" used in this application refers to that when a compound contains several amine functional groups, 1 or more than 1 nitrogen atoms can be oxidized to form an *N*-oxide. Special examples of the *N*-oxide include *N*-oxides of tertiary amines or *N*-oxides of nitrogen-containing heterocyclic nitrogen atoms. Corresponding amines can be treated with an oxidant such as hydrogen peroxide or peracid (such as peroxycarboxylic acid) to form *N*-oxides (with reference to Advanced Organic Chemistry, Wiley Interscience, 4th edition, Jerry March, pages). In particular, an *N*-oxide can be prepared by an L. W. Deady method (Syn. Comm. 1977, 7, 509-514). For example, an amine compound reacts with m-chloroperbenzoic acid (MCPBA) in an inert solvent such as dichloromethane.

The "solvate" used in this application refers to a complex formed by one or more solvent molecules and the compound of this application. A solvent used for forming the solvate includes, but is not limited to, water, isopropanol, ethanol, methanol, dimethyl sulfoxide, ethyl acetate, acetic acid, and aminoethanol. The term "hydrate" refers to a complex formed when the solvent molecule is water.

The term "metabolite" refers to a product obtained by metabolism of a specific compound or a salt thereof in the body. The metabolite of a compound may be identified by technologies known in the art, and the activity may be characterized by a test method as described in this application. Such a product may be obtained by oxidation, reduction, hydrolysis, acylation, deacylation, esterification, degreasing and enzymatic cleavage of an administered compound and other methods. Accordingly, this application includes metabolites of the compound, including metabolites produced after adequate contact of the compound of this application with mammals for a period of time.

The term "pharmaceutically acceptable salt" used in this application refers to organic salts and inorganic salts of the compound of this application. The pharmaceutically acceptable salt is well known in the art, as recorded in the following document: S. M. Berge *et al.*, describe pharmaceutically acceptable salts in detail in J. Pharmaceutical Sciences, 1977, 66: 1-19 . A pharmaceutically acceptable salt formed by a non-toxic acid includes, but is not limited to, inorganic acid salts formed by reactions with an amino group such as hydrochloride, hydrobromate, phosphate, sulfate and perchlorate, and organic acid salts such as acetate, oxalate, maleate, tartrate, citrate, succinate and malonate, or these salts are obtained by other methods, such as ion exchange, as recorded in books and documents. Other pharmaceutically acceptable salts include adipate, alginate, ascorbate, aspartate, benzenesulfonate, benzoate, bisulfate, borate, butyrate, camphorate, camphorsulfonate, cyclopentyl propionate, disgluconate, dodecyl sulfate, ethanesulfonate, formate, fumarate, glucoheptanoate, glycerophosphate, gluconate, hemisulphate, heptanate, caproate, hydroiodate, 2-hydroxyethanesulfonate, lactobionate, lactate, laurate, lauryl sulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate and the like. A salt obtained by an appropriate alkali includes salts of alkali metals, alkaline earth metals, ammonium and $N^+(C_{1-4}\ alkyl)_4$. This application is also intended to include quaternary ammonium salts formed by any compounds containing an N group. Water-soluble products, oil-soluble products or dispersed products can be obtained by quaternization. The salts of alkali metals or alkaline earth metals include sodium, lithium, potassium, calcium, magnesium and the like. The pharmaceutically acceptable salt further includes appropriate and non-toxic ammonium, quaternary ammonium salts and amine cations formed by equilibrium-resistant ions, such as halides, hydroxides, carboxylates, sulfates, phosphates, nitrates, $C_{1-8}$ sulfonates and aromatic sulfonates.

The term "prodrug" used in this application represents that a compound is converted into the compound as shown in Formula (I) in the body. Such conversion is affected by hydrolysis of a precursor drug in the blood or by enzymatic conversion of the precursor drug into a parent structure in the blood or tissues. A precursor drug compound of this application may be an ester, and the ester capable of being used as a precursor drug in existing applications includes phenyl esters, aliphatic ($C_{1-24}$) esters, acyloxymethyl esters, carbonates, carbamates and amino acid esters. For example, a compound containing hydroxyl in this application can be acylated to obtain a compound in a precursor drug form. Other precursor drug forms include phosphates. For example, such phosphate compounds are obtained by phosphorylation of hydroxyl on the parent structure. A complete discussion of the precursor drug can refer to the following documents: T. Higuchi and V Stella, Pro-drugs as Novel Delivery Systems, Vol. 14 of the A. C. S Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

Any asymmetric atoms (such as carbon and the like) of the compound of this application may exist in a racemate or enantiomer enriched form, such as in the form of an (*R*)-, (*S*)-, or (*R*, *S*)-configuration. In some embodiments, in the aspect of the (*R*)- or (*S*)-configuration, the asymmetric atoms have at least 50% of excess enantiomers, at least 60% of excess enantiomers, at least 70% of excess enantiomers, at least 80% of excess enantiomers, at least 90% of excess enantiomers, at least 95% of excess enantiomers, or at least 99% of excess enantiomers. A substituent on an atom having an unsaturated double bond may exist in a cis-(*Z*)- or trans-(*E*)-form, if possible.

Therefore, as described in this application, the compound of this application may exist in the form of one or a mixture of possible isomers, rotamers, atropisomers and tautomers, such as in the form of basically pure geometric (cis- or trans-) isomers, diastereoisomers, optical isomers (enantiomers), racemates or mixtures thereof.

Any resulting mixtures of isomers can be separated into pure or basically pure geometric or optical isomers, diastereoisomers and racemates based on physicochemical differences of components, for example, separation is conducted by chromatography and/or fractional crystallization.

The racemates of any resulting final products or intermediates can be split into optical enantiomers by a method familiar to a person skilled in the art among known methods, for example, by separating salts of resulting diastereoisomers. Racemate products may also be separated by chiral chromatography, such as high pressure liquid chromatography (HPLC) using a chiral adsorbent. In particular, the enantiomers may be prepared by asymmetric synthesis (such as Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E. L. Stereochemistry of Carbon Compounds (McGraw-Hill, NY, 1962); and Wilen, S.H. Tables of Resolving Agents and Optical Resolutions p. 268 (E. L. Eliel, Ed., Univ. of Notre Dame Press, Notre Dame, IN 1972).

As described in this application, the compound of this application may be optionally substituted with one or more substituents, such as the compounds as shown in the general formulas above, or specific examples in the embodiments, subclasses, and a class of compounds included in this application. It is to be understood that the term "optionally substituted" and the term "substituted or unsubstituted" may be used interchangeably. The term "optionally", "optional" or "option" refers to that a subsequent event or condition described may or may not occur, and the description includes situations in which the event or condition occurs and situations in which the event or condition does not occur. Generally, the term "optionally", whether or not placed in front of the term "substituted", refers to that one or more hydrogen atoms in a given structure is/are substituted with a specific substituent. Unless otherwise indicated, an optional substituent group may undergo substitution at various substitutable positions of a group. When more than one position in a given structural formula can be substituted by one or more substituents selected from a specific group, the substituents, being same or different, may perform substitution at various positions. The substituent may be, but is not limited to, F, Cl, Br, CN, $N_3$, OH, $NH_2$, $NO_2$, oxo (=O), $OR^a$, $-NR^bR^c$, $C_{1-6}$ alkyl, $C_{1-6}$ aliphatic family, $C_{1-6}$ haloalkyl, $C_{1-6}$ alkoxyl, $C_{1-6}$ alkylamino, $C_{2-6}$ alkenyl, $C_{2-6}$ alkynyl, $C_{3-8}$ cycloalkyl, $C_{3-8}$ cycloalkyl-$C_{1-4}$ alkylene, heterocyclyl composed of 3-12 atoms, (heterocyclyl composed of 3-12 atoms)-$C_{1-4}$ alkylene, $C_{6-10}$ aryl, $C_{6-10}$ aryl-$C_{1-4}$ alkylene, heteroaryl composed of 5-14 atoms or (heteroaryl composed of 5-14 atoms)-$C_{1-4}$ alkylene, where $R^a$, $R^b$ and $R^c$ are defined as described in this application.

Moreover, unless otherwise clearly indicated, the descriptions "... is independently", "where ... is independently" and "...is independently" used in this application may be used interchangeably, and understood broadly, either referring to that in different groups, specific options expressed by same symbols do not affect each other, or referring to that in the same group, specific options expressed by the same symbol do not affect each other.

In various parts of the specification, substituents of the compound disclosed in this application are disclosed according to group types or ranges. In particular, this application includes each independent sub-combination of various members of these group types and ranges. For example, the term "$C_{1-6}$ alkyl" specifically refers to independently disclosed

methyl, ethyl, $C_3$ alkyl, $C_4$ alkyl, $C_5$ alkyl, and $C_6$ alkyl.

In various parts of this application, connection substituents are described. When a structure clearly requires a connection group, Markush variables listed for the group are understood as the connection groups. For example, when the structure requires a connection group and a Markush group for the variable is defined as including "alkyl" or "aryl", it is to be understood that the "alkyl" or "aryl" represents a connected alkylene group or arylene group, respectively.

The term "alkyl" or "alkyl group" used in this application represents a saturated straight or branched univalent hydrocarbon atom group containing 1-20 carbon atoms. Unless otherwise specified in detail, the alkyl group contains 1-20 carbon atoms; in some embodiments, the alkyl group contains 1-10 carbon atoms; in some other embodiments, the alkyl group contains 1-9 carbon atoms; in some other embodiments, the alkyl group contains 1-8 carbon atoms; in some other embodiments, the alkyl group contains 1-6 carbon atoms; in some other embodiments, the alkyl group contains 1-4 carbon atoms; and in some other embodiments, the alkyl group contains1-3 carbon atoms.

Examples of the alkyl group include, but are not limited to, methyl (Me, $-CH_3$), ethyl (Et, $-CH_2CH_3$), n-propyl (*n*-Pr, $-CH_2CH_2CH_3$), isopropyl (*i*-Pr, $-CH(CH_3)_2$), n-butyl (n-Bu, $-CH_2CH_2CH_2CH_3$), isobutyl (*i*-Bu, $-CH_2CH(CH_3)_2$), sec-butyl (*s*-Bu, $-CH(CH_3)CH_2CH_3$), tert-butyl (*t*-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), n-heptyl, n-octyl and the like, where the alkyl group may be independently unsubstituted or substituted with one or more substituents as described in this application.

The term "alkyl" and a prefix "alkane" used in this application include straight and branched saturated carbon chains.

The term "alkylene" represents a saturated divalent hydrocarbyl group obtained by removing two hydrogen atoms from a straight or branched saturated hydrocarbyl. Unless otherwise specified in detail, the alkylene group contains 1-10 carbon atoms; in some other embodiments, the alkylene group contains 1-6 carbon atoms; in some other embodiments, the alkylene group contains 1-4 carbon atoms; and in some other embodiments, the alkylene group contains 1-2 carbon atoms. Such examples include methylene ($-CH_2-$), ethylidene ($-CH_2CH_2-$), isopropylidene ($-CH(CH_3)CH_2-$) and the like, where the alkylene group may be independently unsubstituted or substituted with one or more substituents as described in this application.

The term "alkenyl" represents a straight or branched univalent hydrocarbyl containing 2-12 carbon atoms, 2-8 carbon atoms, 2-6 carbon atoms, or 2-4 carbon atoms, where in at least one position, C-C is in an $sp^2$ double-bond unsaturated state, the alkenyl group may be independently unsubstituted or substituted with one or more substituents as described in this application, and the group includes "cis" and "trans" or "*Z*" and "*E*" configurations. Specific examples include, but are not limited to, vinyl ($-CH=CH_2$), allyl ($-CH_2CH=CH_2$) and the like.

The term "alkynyl" represents a straight or branched univalent hydrocarbyl containing 2-12 carbon atoms, 2-8 carbon atoms, 2-6 carbon atoms or 2-4 carbon atoms, where in at least one position, C-C is in a sp three-bond unsaturated state, and the alkynyl group may be independently unsubstituted or substituted with one or more substituents as described in this application. Specific examples include, but are not limited to, acetylenyl ($-C=CH$), propargyl ($-CH_2C\equiv CH$), 1-propargyl ($-C\equiv C-CH_3$) and the like.

The term "alkoxyl" represents that an alkyl group is connected to rest parts of a molecule by oxygen atoms, where the alkyl group is defined as described in this application. Unless otherwise specified in detail, the alkoxyl group contains 1-20 carbon atoms; in some embodiments, the alkoxyl group contains 1-10 carbon atoms; in some other embodiments, the alkoxyl group contains 1-8 carbon atoms; in some other embodiments, the alkoxyl group contains 1-6 carbon atoms; in some other embodiments, the alkoxyl group contains 1-4 carbon atoms; and in some other embodiments, the alkoxyl group contains 1-3 carbon atoms.

Examples of the alkoxyl group include, but are not limited to, methoxyl (MeO, $-OCH_3$), ethoxyl (EtO, $-OCH_2CH_3$), 1-propoxyl (*n*-PrO, *n*-propoxyl, $-OCH_2CH_2CH_3$), 2-propoxyl (*i*-PrO, *i*-propoxyl, $-OCH(CH_3)_2$), 1-butoxyl (*n*-BuO, *n*-butoxyl, $-OCH_2CH_2CH_2CH_3$), 2-methyl-1-propoxyl (*i*-BuO, *i*-butoxyl, $-OCH_2CH(CH_3)_2$), 2-butoxyl (*s*-BuO, *s*-butoxyl, $-OCH(CH_3)CH_2CH_3$), 2-methyl-2-propoxyl (*t*-BuO, *t*-butoxyl, $-OC(CH_3)_3$), 1-pentyloxy (*n*-pentyloxy, $-OCH_2CH_2CH_2CH_2CH_3$), 2-pentyloxy ($-OCH(CH_3)CH_2CH_2CH_3$), 3-pentyloxy ($-OCH(CH_2CH_3)_2$), 2-methyl-2-butoxyl ($-OC(CH_3)_2CH_2CH_3$), 3-methyl-2-butoxyl ($-OCH(CH_3)CH(CH_3)_2$), 3-methyl-1-butoxyl ($-OCH_2CH_2CH(CH_3)_2$), 2-methyl-1-butoxyl ($-OCH_2CH(CH_3)CH_2CH_3$) and the like, where the alkoxyl group may be independently unsubstituted or substituted with one or more substituents as described in this application.

The term "haloalkyl", "haloalkenyl" or "haloalkoxyl" represents that an alkyl, alkenyl or alkoxyl group is substituted with one or more halogen atoms. Such examples include, but are not limited to, trifluoromethyl, trifluoromethoxyl and the like.

The terms "carbon ring", "carbocyclyl" or "carbocyclic" may be used interchangeably herein, and all refer to a non-aromatic carbon ring system that is saturated or contains one or more unsaturated units or contains 3-14 ring carbon atoms. In some embodiments, the number of carbon atoms is 3-12; in some other embodiments, the number of carbon atoms is 3-10; in some other embodiments, the number of carbon atoms is 3-8; in some other embodiments, the number of carbon atoms is 5-6; and in some other implementations, the number of carbon atoms is 6-8. The term "carbocyclyl" includes a monocyclic, bicyclic or polycyclic fused, spiro or bridged carbon ring system, and also includes a polycyclic ring system in which a carbon ring may be fused with one or more non-aromatic carbon rings or heterocyclic rings or one or more aromatic rings or combinations thereof, where an atomic group or point is connected to the carbon ring. A bicyclic carbocyclyl includes bridged bicyclic carbocyclyl, fused bicyclic carbocyclyl and spiro bicyclic carbocyclyl. A "fused" bicyclic ring system includes two rings sharing 2 adjacent ring atoms. A bridged bicyclic group includes two rings sharing 3 or 4 adjacent ring atoms. A spiro ring system shares 1 ring atom. A suitable carbocyclyl group includes, but is not limited to, cycloalkyl, cycloalkenyl, and cycloalkynyl. Examples of the carbocyclyl group further include, but are definitely not limited to, cyclopropyl, cyclobutyl, cyclopentyl, 1-cyclopentyl-1-alkenyl, 1-cyclopentyl-2-alkenyl, 1-cyclopentyl-3-alkenyl, cyclohexyl, 1-cyclohexyl-1-alkenyl, 1-cyclohexyl-2-alkenyl, 1-cyclohexyl-3-alkenyl, cyclohexadienyl, cycloheptyl, cyclooctyl, cyclononyl, cyclodecyl, cycloundecyl, cyclododecyl and the like. A bridged carbocyclyl group includes, but is not limited to, bicyclo[2.2.2]octyl, bicyclo[2.2.1]heptyl, bicyclo[3.3.1]nonyl, bicyclo[3.2.3]nonyl and the like..

The term "cycloalkyl" refers to a saturated monocyclic, bicyclic or tricyclic system containing 3-12 ring carbon atoms in which one or more connection points are connected to rest parts of a molecule. In some embodiments, the cycloalkyl is a ring system containing 3-10 ring carbon atoms; in some other embodiments, the cycloalkyl is a ring system containing 3-8 ring carbon atoms; in some other embodiments, the cycloalkyl is a ring system containing 3-6 ring carbon atoms; in some other embodiments, the cycloalkyl is a ring system containing 5-6 ring carbon atoms; and the cycloalkyl group may be independently unsubstituted or substituted with one or more substituents as described in this application.

The terms "heterocyclyl" and "heterocyclic ring" may be used interchangeably herein, and refer to a saturated or partially unsaturated non-aromatic monocyclic, bicyclic or tricyclic ring system containing 3-12 ring atoms, where at least one ring atom is selected from nitrogen, sulfur and oxygen atoms, and one or more connection points in the ring system are connected to rest parts of a molecule. The term "heterocyclyl" includes a monocyclic, bicyclic or polycyclic fused, spiro or bridged heterocyclic ring system, and also includes a polycyclic ring system in which a heterocyclic ring may be fused with one or more non-aromatic carbon rings or heterocyclic rings or one or more aromatic rings or combinations thereof, where an atomic group or point is connected to the heterocyclic ring. A bicyclic heterocyclyl includes bridged bicyclic heterocyclyl, fused bicyclic heterocyclyl and spiro bicyclic heterocyclyl. Unless otherwise specified, the heterocyclyl may be a carbon group or a nitrogen group, and the $-CH_2-$ group may be optionally substituted with -C(=O)-. A sulfur atom of a ring may be optionally oxidized into an S-oxide. A nitrogen atom of a ring may be optionally oxidized into an A-oxide. In some embodiments, the heterocyclyl is a monocyclic or bicyclic heterocyclyl composed of 3-8 atoms; in some other embodiments, the heterocyclyl is a monocyclic or bicyclic heterocyclyl composed of 3-6 atoms; in some other embodiments, the heterocyclyl is a monocyclic or bicyclic heterocyclyl composed of 6-8 atoms; in some other embodiments, the heterocyclyl is a heterocyclyl composed of 5-6 atoms; in some other embodiments, the heterocyclyl is a heterocyclyl composed of 4 atoms; in some other embodiments, the heterocyclyl is a heterocyclyl composed of 5 atoms; in some other embodiments, the heterocyclyl is a heterocyclyl composed of 6 atoms; in some other embodiments, the heterocyclyl is a heterocyclyl composed of 7 atoms; and in some other embodiments, the heterocyclyl is a heterocyclyl composed of 8 atoms.

Examples of the heterocyclyl include, but are not limited to: oxirane group, azacyclobutyl, oxacyclobutyl, thiocyclobutyl, pyrrolidyl, 2-pyrrolinyl, 3-pyrrolinyl, pyrazolinyl, pyrazolidyl, imidazolinyl, imidazolidinyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2*H*-pyranyl, 4*H*-pyranyl, tetrahydrothianyl, piperidyl, morpholinyl, thiomorpholinyl, piperazinyl, dioxalkyl, dithionyl, thioxanyl, perpiperazinyl, perpiperidyl, oxacycloheptyl, thiocycloheptyl, oxazapinyl, diazepinyl, thiazepinyl, indolinyl, 1,2,3,4-tetrahydroisoquinolinyl, 1,3-benzodioxol, and 2-oxa-5-azobicyclo[2.2.1]hept-5-yl. Examples of the heterocyclyl in which the $-CH_2-$ group is substituted with -C(=O)- include, but are not limited to, 2-oxopyrrolyl, oxo-1,3-thiazolyl, 2-piperidinonyl, 3,5-dioxypiperidyl, and pyrimidinedionyl. Examples of the heterocyclyl in which a sulfur atom is oxidized include, but are not limited to, sulfolanyl and 1,1-dioxothiomorphinyl. A bridged heterocyclyl group includes, but is not limited to, 2-oxabicyclo[2.2.2]octyl, 1-azabicyclo[2.2.2]octyl, 3-azabicyclo[3.2.1]octyl and the like. The heterocyclyl group may optionally substituted with one or more substituents as described in this application.

The term "bridge" refers to a bond, atom, or unbranched atom chain that connects two different parts of a molecule. Two atoms (usually but not always two tertiary carbon atoms) connected by a bridge are represented as "bridge heads".

The term "spiro" refers to a ring system having an atom (usually a quaternary carbon atom) as the only common atom between two rings.

The term "composed of n atoms", where n is an integer, typically describes the number of ring-forming atoms in a molecule, and the number of ring-forming atoms in the molecule is n. For example, piperidinyl is a heterocyclyl

composed of 6 atoms, while 1,2,3,4-tetrahydronaphthyl is a carbocyclyl group composed of 10 atoms. In a bridge ring or a spiro ring, the number of ring atoms includes atoms in a bridge or spiro.

The term "heteroatom" refers to O, S, N, P, and Si, including any oxidized form of N, S, and P; the forms of primary amines, secondary amines, tertiary amines and quaternary ammonium salts; or the form that hydrogen on a nitrogen atom in a heterocyclic ring is substituted, such as N (like N in 3,4-dihydro-2*H*-pyrrolyl), NH (like NH in pyrrolidinyl), or NR (like NR in *N*-substituted pyrrolidinyl).

The term "halogen" refers to F, Cl, Br, or I.

The term "$N_3$" represents an azide structure. This group may be connected to other groups. For example, this group may be connected to a methyl to form triazomethane ($MeN_3$), or connected to a phenyl to form azidobenzene ($PhN_3$).

The term "aryl" may be used alone or used as a large part of "aralkyl", "arylalkoxyl" or "aryoxyalkyl", and represents a monocylic, bicylic or tricylic carbon ring system containing 6-14 ring atoms, 6-12 ring atoms or 6-10 ring atoms, where at least one ring system is aromatic, each ring system contains a ring composed of 3-7 atoms, and one or more attachment points are connected to rest parts of a molecule. The term "aryl" and the term "aromatic ring" or "aromaticring" may be used interchangeably. For example, the aromatic ring may include phenyl, naphthyl, and anthracyl. The aryl group may be independently unsubstituted or substituted with one or more substituents as described in this application.

The term "heteroaryl" may be used alone or used as a large part of "heteroaryl alkyl" or "heteroaryl alkoxyl", and represents a monocylic, bicylic or tricylic ring system containing 5-14 ring atoms, 5-12 ring atoms, 5-10 ring atoms or 5-6 ring atoms, where at least one ring system is aromatic, at least one ring system contains one or more heteroatoms, each ring system contains a ring composed of 5-7 atoms, and one or more attachment points are connected to rest parts of a molecule. Unless otherwise specified, the heteroaryl may be a carbon group or a nitrogen group, and the $-CH_2-$ group may be optionally substituted with -C(=O)-. A sulfur atom of a ring may be optionally oxidized into an *S*-oxide. A nitrogen atom of a ring may be optionally oxidized into an A-oxide. The term "heteroaryl" and the term "heteroaromatic ring" or "heteroaromatic compound" may be used interchangeably. In some other embodiments, the heteroaryl is a heteroaryl composed of 5-12 atoms, including 1, 2, 3, or 4 heteroatoms independently selected from O, S, and N. In some other embodiments, the heteroaryl is a heteroaryl composed of 5-10 atoms, including 1, 2, 3, or 4 heteroatoms independently selected from O, S, and N. In some other embodiments, the heteroaryl is a heteroaryl composed of 5-6 atoms, including 1, 2, 3, or 4 heteroatoms independently selected from O, S, and N. In some other embodiments, the heteroaryl is a heteroaryl composed of 5 atoms, including 1, 2, 3, or 4 heteroatoms independently selected from O, S, and N. In some other embodiments, the heteroaryl is a heteroaryl composed of 6 atoms, including 1, 2, 3, or 4 heteroatoms independently selected from O, S, and N.

In some other embodiments, the heteroaryl includes, but is not limited to, the following monocyclic groups: 2-furyl, 3-furyl, *N*-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, *N*-pyrrolyl, 2-pyrrolyl, 3-pyrrolyl, 2-pyridyl, 3-pyridyl, 4-pyridyl, 2-pyrimidinyl, 4-pyrimidinyl, 5-pyrimidinyl, pyridazinyl (such as 3-pyridazinyl), 2-thiazolyl, 4-thiazolyl, 5-thiazolyl, tetrazolyl (such as 5*H*-tetrazolyl, 2*H*-tetrazolyl), triazolyl (such as 2-triazolyl, 5-triazolyl, 4*H* 1,2,4-triazolyl, 1*H*-1,2,4-triazolyl, 1,2,3- triazolyl), 2-thiophenyl, 3-thiophenyl, pyrazolyl (such as 2-pyrazolyl and 3-pyrazolyl), isothiazolyl, 1,2,3-oxadiazolyl, 1,2,5-oxadiazolyl, 1,2,4-oxadiazolyl, 1,3,4-oxadiazolyl, 1,2,3-thiodiazolyl, 1,3,4-thiodiazolyl, 1,2,5-thiodiazolyl, pyrazinyl, 1,3,5-triazinyl; and the heteroaryl also includes, but is definitely not limited to, the following bicyclic groups: benzimidazolyl, benzofuryl, benzothiophenyl, indolyl (such as 2-indolyl), purinyl, quinolinyl (such as 2-quinolinyl, 3-quinolinyl, 4-quinolinyl), isoquinolinyl (such as 1-isoquinolinyl, 3-isoquinolinyl, or 4-isoquinolinyl), oxthiazolyl,

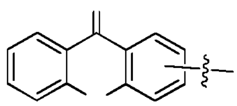

and

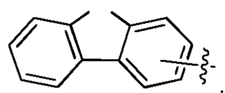

The heteroaryl group is optionally substituted with one or more substituents as described in this application.

The term "carboxyl", whether used alone or in combination with other terms, such as "carboxyalkyl", represents $-CO_2H$. The term "carbonyl", whether used alone or in combination with other terms, such as "aminocarbonyl" or "acyloxyl", represents -(C=O)-.

The term "alkylamino" includes "*N*-alkylamino" and "*N*,*N*-dialkylamino", where an amino group is independently

substituted with one or two alkyl groups. In some embodiments, the alkylamino is a lower alkylamino group in which one or two $C_{1-6}$ alkyls are connected to a nitrogen atom. In some other embodiments, the alkylamino is a lower $C_{1-3}$ alkylamino group. Suitable alkylamino groups may be monoalkyl amino or dialkyl amino. Such examples include, but are not limited to, *N*-methylamino, *N*-ethylamino, *N*,*N*-dimethylamino, *N*,*N*-diethylamino and the like.

The term "arylamino" represents that an amino group is substituted with one or two aryl groups. Such examples include, but are not limited to, *N*-phenylamino. In some embodiments, an aromatic ring on the arylamino may be further substituted.

The term "aminoalkyl" includes a $C_{1-10}$ straight or branched alkyl group substituted with one or more aminos. In some embodiments, the aminoalkyl is a $C_{1-6}$ "lower aminoalkyl" substituted with one or more amino groups. Such examples include, but are not limited to, aminomethyl, aminoethyl, aminopropyl, aminobutyl, and aminohexyl.

As described in this application, a ring system formed by connecting a bond on a substituent to a central ring represents that the substituent may perform substitution at any substitutable position on the ring. The ring system includes a monocylic, bicylic or polycylic ring system.

The term "unsaturated" used in this application represents that a group has one or more unsaturations.

The term "comprise" or "include" is an open expression, namely, including contents specified in this application, but not excluding contents in other aspects.

As described in this application, the term "pharmaceutically acceptable carrier" includes any solvent, dispersing medium, coating material, surfactant, antioxidant, preservative (such as antibacterial agent, antifungal agent), isotonic agent, salt, drug stabilizer, binder, excipient, dispersant, lubricant, sweetener, flavoring agent, colorant, or a combination thereof, and all these carriers are known to a person skilled in the art (as described in Remington's Pharmaceutical Sciences, 18th Ed. Mack Printing Company, 1990, pp. 1289-1329). Except when any conventional carrier is incompatible with an active ingredient, use of these carriers in a therapeutic or pharmaceutical composition is involved.

The term "inhibit LSD 1" used in this application includes reduction of the expression quantity or activity of LSD1 (such as reduction by at least 10%) and total inhibition of the expression or activity of LSD1 (such as inhibition of the expression or activity of LSD1 by 100%). In some embodiments, the expression or activity of LSD1 is inhibited by at least 50%, at least 65%, at least 75%, at least 85%, at least 90%, or at least 95%.

The term "effective amount" of the compound of this application refers to an amount leading to an expected biological response. In this application, the expected biological response is to inhibit LSD1, prevent recurrence, evolution, onset or process of symptoms associated with overexpression of LSD1, or enhance or improve a preventive or therapeutic effect of another anti-tumor therapy method used. The exact amount of a compound administered to a curee depends on the application mode and the severity and characteristics of the curee, such as health conditions, age, sex, body weight and drug tolerance. An appropriate dose will be determined by a skilled person based on these and other factors. When the compound is administered in combination with other anti-tumor agents, such as administered in combination with an anti-mitotic drug, the "effective amount" of a second reagent depends on the type of the drug used. An appropriate dose of an approved reagent is known and can be adjusted by a skilled person based on a disease of a curee, the type of the disease being treated and the amount of the compound of this application. In a case that the dose is not specified, the effective amount is taken. For example, the compound of this application may be administered to a curee for therapeutic or preventive treatment in a dose range of about 0.01-100 mg/body weight/day.

The term "treatment" used in this application refers to therapeutic and preventive treatment. For example, the therapeutic treatment includes alleviating or relieving the process, severity and/or duration of a disease mediated by overexpression or hyperactivity of LSD1, or relieving one or more symptoms (specifically, one or more identifiable symptoms) of a disease mediated by overexpression or hyperactivity of LSD1 by administering one or more therapy methods (such as one or more therapeutic agents (such as the compound and composition of this application)). In a specific embodiment, the therapeutic treatment includes improving at least one measurable physical parameter of a disease mediated by overexpression or hyperactivity of LSD1. In other embodiments, the therapeutic treatment includes inhibiting the process of a disease mediated by overexpression or hyperactivity of LSD1 by (for example) stabilizing identifiable symptoms physically, by (for example) stabilizing physical parameters physiologically or by using the two methods. In other embodiments, the therapeutic treatment includes alleviating or stabilizing a disease mediated by overexpression or hyperactivity of LSD1, such as papillary thyroid carcinoma, breast cancer, gastric cancer, bronchogenic carcinoma, lung cancer, acute myeloid leukemia, small cell lung cancer, prostate cancer, or non-hodgkin lymphoma.

The term "protective group" or "PG" refers to that a substituent reacts with other functional groups usually for blocking or protecting a particular function. For example, the term "protective group of amino" refers to that a substituent is connected to an amino group for blocking or protecting a function of the amino in a compound. Suitable protective groups of amino include acetyl, trifluoroacetyl, tert-butoxycarbonyl (BOC, Boc), benzyloxycarbonyl (CBZ, Cbz), and 9-fluorenylmethyloxycarbonyl (Fmoc). Similarly, the term "protective group of hydroxyl" refers to a substituent of hydroxyl for blocking or protecting a function of the hydroxyl. Suitable protective groups include acetyl and silyl. The term "protective group of carboxyl" refers to a substituent of carboxyl for blocking or protecting a function of the carboxyl. General protective groups of carboxyl include $-CH_2CH_2SO_2Ph$, cyanoethyl, 2-(trimethylsilyl)ethyl, 2-(trimethylsilyl)ethoxymethyl,

2-(p-toluenesulfonyl)ethyl, 2-(p-nitrobenzenesulfonyl)ethyl, 2-(diphenylphosphonyl)ethyl, nitroethyl and the like. General description of the protective group can refer to: T W. Greene, Protective Groups in Organic Synthesis, John Wiley & Sons, New York, 1991; and P. J. Kocienski, Protecting Groups, Thieme, Stuttgart, 2005.

**Description of compound of this application**

This application provides a reversible LSD1 inhibitor having a novel structure, which has higher LSD1 inhibitory activity and lower hERG inhibitory activity.

This application provides a compound. The compound is a compound as shown in Formula (I) or a stereoisomer, tautomer, deuterated compound, oxynitride, solvate, metabolite, pharmaceutically acceptable salt or prodrug of the compound having a structure as shown in Formula (I):

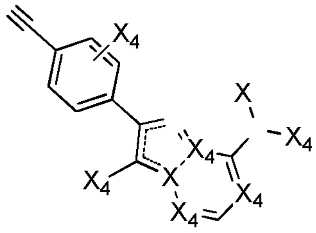

(I)

where X is C, N, or O;

$X_1$, $X_2$, $X_3$, and $X_4$ are respectively selected from C or N, provided that $X_1$ is different from $X_2$, $X_1$ is different from $X_4$, and $X_2$ is different from $X_3$;

$R_1$ and $R_2$ are independently H, deuterium, $C_{1-6}$ alkyl, $C_{3-12}$ carbocyclyl, $C_{3-12}$ carbocyclyl-$C_{1-4}$ alkylene, heterocyclyl composed of 3-12 atoms, (heterocyclyl composed of 3-12 atoms)-$C_{1-4}$ alkylene, $C_{6-10}$ aryl, $C_{6-10}$ aryl-$C_{1-4}$ alkylene, heteroaryl composed of 5-14 atoms, (heteroaryl composed of 5-14 atoms)-$C_{1-4}$ alkylene, or $R_1$, $R_2$ and X connected thereto form a $C_{3-12}$ carbon ring or a heterocyclic ring composed of 3-12 atoms, and where the $C_{1-6}$ alkyl, the $C_{3-12}$ carbocyclyl, the $C_{3-12}$ carbocyclyl-$C_{1-4}$ alkylene, the heterocyclyl composed of 3-12 atoms, the (heterocyclyl composed of 3-12 atoms)-$C_{1-4}$ alkylene, the $C_{6-10}$ aryl, the $C_{6-10}$ aryl-$C_{1-4}$ alkylene, the heteroaryl composed of 5-14 atoms, the (heteroaryl composed of 5-14 atoms)-$C_{1-4}$ alkylene, or the $C_{3-12}$ carbon ring or the heterocyclic ring composed of 3-12 atoms formed by $R_1$, $R_2$ and X connected thereto are independently unsubstituted or substituted with 1, 2, 3, 4, or 5 R';

$R_3$ is $C_{6-10}$ aryl or heteroaryl composed of 5-10 atoms, and where the $C_{6-10}$ aryl and the heteroaryl composed of 5-10 atoms are independently unsubstituted or substituted with 1, 2, 3, or 4 R";

$R_4$ is H, deuterium, F, Cl, Br, CN, $NO_2$, $-OR^a$, $-NR^bR^c$, or $C_{1-6}$ alkyl;

R' and R" are independently H, deuterium, F, Cl, Br, CN, $NO_2$, =O, $-OR^a$, $-NR^bR^c$, $-S(=O)_2R^a$, $C_{1-6}$ alkyl, $C_{3-8}$ cycloalkyl, $C_{3-8}$ cycloalkenyl, heterocyclyl composed of 3-12 atoms, where the $C_{1-6}$ alkyl, the $C_{3-8}$ cycloalkyl, the $C_{3-8}$ cycloalkenyl and the heterocyclyl composed of 3-12 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$; and

$R^a$, $R^b$, and $R^c$ are independently H, deuterium, $C_{1-6}$ alkyl, $C_{1-6}$ haloalkyl, heterocyclyl composed of 3-6 atoms, or $R^b$, $R^c$ and nitrogen atoms connected thereto form a heterocyclic ring composed of 3-6 atoms, where the $C_{1-6}$ alkyl and the heterocyclic ring composed of 3-6 atoms are independently unsubstituted or substituted with 1, 2, 3, or 4 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

In other embodiments, this application relates to a stereoisomer, tautomer, deuterated compound, oxynitride, solvate, metabolite, pharmaceutically acceptable salt or prodrug of a compound having a structure as shown in Formula (II) or (III):

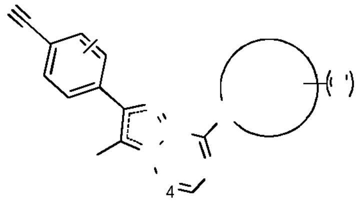

(II),

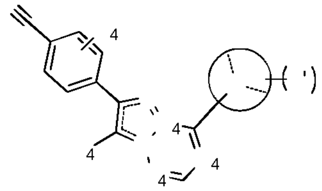

(III),

where ring A is a $C_{3-6}$ carbon ring or a heterocyclic ring composed of 3-6 atoms;
ring B is a bridge ring composed of 6-8 atoms;
m is 0, 1, 2, 3, 4, or 5; and
X, $X_1$, $X_2$, $X_3$, $X_4$, $R_3$, $R_4$, and R' are defined as described in this application.

According to the embodiment of this application, the compound having the structure as shown in Formula (II) (III) has better LSD1 inhibitory activity.

In some other embodiments, this application relates to a stereoisomer, tautomer, deuterated compound, oxynitride, solvate, metabolite, pharmaceutically acceptable salt or prodrug of a compound having a structure as shown in Formula (IV), Formula (V), Formula (VI), or Formula (VII):

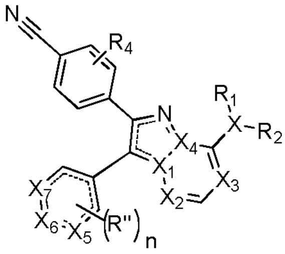
(IV),
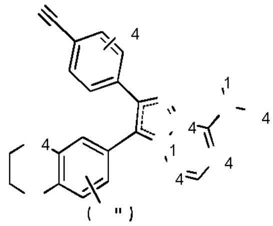
(V),

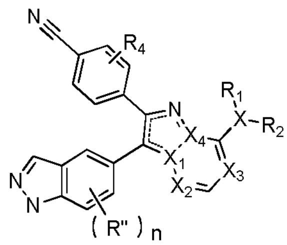
(VI),
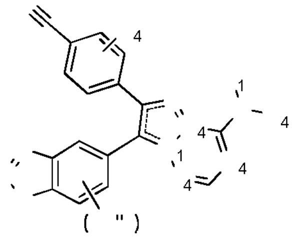
(VII),

where $X_5$ is empty or C; $X_6$ and $X_7$ are independently selected from C or N respectively; $X_8$ is O, S, C, or N;
n is 0, 1, 2, 3, or 4; and
X, $X_1$, $X_2$, $X_3$, $X_4$, $R_1$, $R_2$, $R_4$, and R" are defined as described in this application.

According to the embodiment of this application, the compound having the structure as shown in Formula (VI) or (VII) has lower hERG inhibitory activity.

In some other embodiments, this application relates to a stereoisomer, tautomer, deuterated compound, oxynitride, solvate, metabolite, pharmaceutically acceptable salt or prodrug of a compound having a structure as shown in Formula (VIII) or Formula (IX):

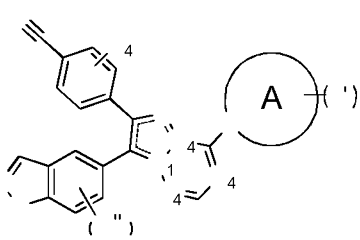
(VIII),
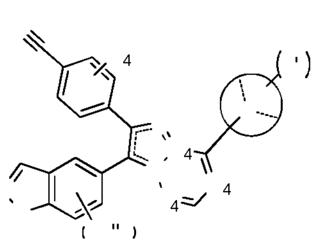
(IX),

where ring A is a $C_{3-6}$ carbon ring or a heterocyclic ring composed of 3-6 atoms;
ring B is a bridge ring composed of 6-8 atoms;
m is 0, 1, 2, 3, 4, or 5;
n is 1, 2, 3, or 4;
R" is $C_{1-6}$ alkyl or $C_{3-8}$ cycloalkyl, where the $C_{1-6}$ alkyl and the $C_{3-8}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$; and
X, $X_1$, $X_2$, $X_3$, $X_4$, $R_4$, and R' are defined as described in this application.

According to the embodiment of this application, the compound having the structure as shown in Formula (VIII) or (IX) has better LSD1 inhibitory activity and lower hERG inhibitory activity.

In some other embodiments, this application relates to a stereoisomer, tautomer, deuterated compound, oxynitride, solvate, metabolite, pharmaceutically acceptable salt or prodrug of a compound having a structure as shown in Formula (X) or Formula (XI):

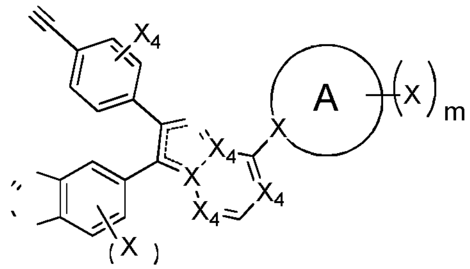

(X),

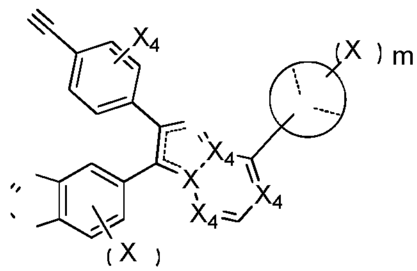

(XI),

where ring A is a $C_{3-6}$ carbon ring or a heterocyclic ring composed of 3-6 atoms;
ring B is a bridge ring composed of 6-8 atoms;
m is 0, 1, 2, 3, 4, or 5; and
n is 0, 1, 2, 3, or 4.
X, $X_1$, $X_2$, $X_3$, $X_4$, $R_4$, R', and R" are defined as described in this application.

According to the embodiment of this application, the compound having the structure as shown in Formula (X) or (XI) has better LSD1 inhibitory activity and lower hERG inhibitory activity.

In some other embodiments, X in the compound having the structure as shown in Formula (I) to Formula (XI) is N.

In some other embodiments, $R_1$ and $R_2$ are independently H, deuterium, $C_{1-6}$ alkyl, $C_{3-6}$ carbocyclyl, $C_{3-6}$ carbocyclyl-$C_{1-4}$ alkylene, heterocyclyl composed of 3-6 atoms, (heterocyclyl composed of 3-6 atoms)-$C_{1-4}$ alkylene, $C_{6-8}$ aryl, $C_{6-8}$ aryl-$C_{1-4}$ alkylene, heteroaryl composed of 5-8 atoms, (heteroaryl composed of 5-8 atoms)-$C_{1-4}$ alkylene, or $R_1$, $R_2$ and X connected thereto form a $C_{3-8}$ carbon ring or a heterocyclic ring composed of 3-8 atoms, and where the $C_{1-6}$ alkyl, the $C_{3-6}$ carbocyclyl, the $C_{3-6}$ carbocyclyl-$C_{1-4}$ alkylene, the heterocyclyl composed of 3-6 atoms, the (heterocyclyl composed of 3-6 atoms)-$C_{1-4}$ alkylene, the $C_{6-8}$ aryl, the $C_{6-8}$ aryl-$C_{1-4}$ alkylene, the heteroaryl composed of 5-8 atoms, the (heteroaryl composed of 5-8 atoms)-$C_{1-4}$ alkylene, or the $C_{3-8}$ carbon ring or the heterocyclic ring composed of 3-8 atoms formed by $R_1$, $R_2$ and X connected thereto are independently unsubstituted or substituted with 1, 2, or 3 R'.

In some other embodiments, $R_3$ is $C_{6-9}$ aryl or heteroaryl composed of 5-9 atoms, and where the $C_{6-9}$ aryl and the heteroaryl composed of 5-9 atoms are independently unsubstituted or substituted with 1 or 2 R".

In some other embodiments, $R_4$ is H, deuterium, F, Cl, Br, CN, $NO_2$, -OH, $-NH_2$, or $C_{1-3}$ alkyl.

In some other embodiments, R' is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, heterocyclyl composed of 3-6 atoms, where the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

In some other embodiments, R" is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, heterocyclyl composed of 3-6 atoms, where the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, as in the compound having the structure as shown in Formula (VI), Formula (VII), Formula (VIII), or Formula (IX), R" is $C_{1-3}$ alkyl, $C_{3-6}$ cycloalkyl, where the $C_{1-3}$ alkyl and the $C_{3-6}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, $R_1$ and $R_2$ are independently H, deuterium, $C_{1-6}$ alkyl, $C_{3-6}$ carbocyclyl, heterocyclyl composed of 3-6 atoms, or $R_1$, $R_2$ and X connected thereto form a $C_{3-8}$ carbon ring or a heterocyclic ring composed of 3-8 atoms, where the $C_{1-6}$ alkyl, the $C_{3-6}$ carbocyclyl, the heterocyclyl composed of 3-6 atoms, or the $C_{3-8}$ carbon ring or the heterocyclic ring composed of 3-8 atoms formed by $R_1$, $R_2$ and X connected thereto are independently unsubstituted or substituted with 1, 2, or 3 R'.

In some other embodiments, $R_1$ and $R_2$ are independently H, deuterium, methyl, ethyl, isopropyl, n-propyl, n-butyl, tert-butyl, cyclopropyl, cyclobutyl, cyclopentyl, or

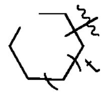
,

and the methyl, the ethyl, the isopropyl, the n-propyl, the n-butyl and the tert-butyl are unsubstituted or substituted with cyclopropyl, cyclobutyl, cyclopentyl, or

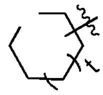
;

the cyclopropyl, the cyclobutyl, the cyclopentyl and the

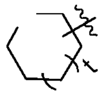

are unsubstituted or substituted with $-NH_2$; and t is 0 or 1.

In some other embodiments, $R_1$, $R_2$ and X connected thereto form

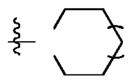
,

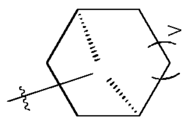
,

S is 0, 1, or 2, V is 1 or 2, and the

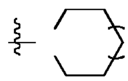

and the

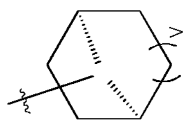

are unsubstituted or substituted with deuterium, F, Cl, Br, or $-NR^bR^c$.

In some other embodiments, the ring A is

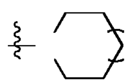
,

S is 0, 1 or 2, and the

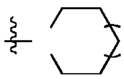

is unsubstituted or substituted with deuterium, F, Cl, Br, or $-NR^bR^c$.

In some other embodiments, the ring B is

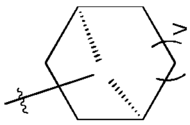

,

V is 1 or 2, and the

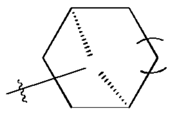

is unsubstituted or substituted with deuterium, F, Cl, Br, or $-NR^bR^c$.

In some other embodiments, $R_3$ is phenyl, naphthyl, pyrrolyl, pyridyl, pyrazolyl, imidazolyl, triazolyl, tetrazolyl, oxazolyl, oxadiazolyl, 1,3,5-triazinyl, thiazolyl, thienyl, pyrazinyl, pyridazinyl, pyrimidinyl, indolyl, purinyl, quinolinyl, isoquinolinyl, phenothianyl, where the phenyl, the naphthyl, the pyrrolyl, the pyridyl, the pyrazolyl, the imidazolyl, the triazolyl, the tetrazolyl, the oxazolyl, the oxadiazolyl, the 1,3,5-triazinyl, the thiazolyl, the thienyl, the pyrazinyl, the pyridazinyl, the pyrimidinyl, the indolyl, the purinyl, the quinolinyl, the isoquinolinyl and the phenothianyl are independently unsubstituted or substituted with 1 or 2 R".

In some other embodiments, $R_3$ is phenyl, and the phenyl is unsubstituted or substituted with F, Cl, Br, or $-OCH_3$.

In some other embodiments, $R_3$ is naphthyl, and the naphthyl is unsubstituted or substituted with R".

In some other embodiments, $R_3$ is

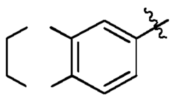

,

and the

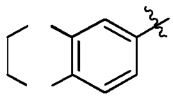

is unsubstituted or substituted with methyl, ethyl, isopropyl, n-propyl, n-butyl, or tert-butyl.

In some other embodiments, $R_3$ is

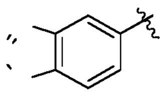

,

and the

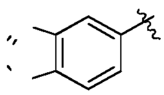

is unsubstituted or substituted with R".

In some other embodiments, $R_3$ is

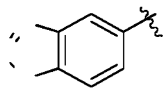

,

the

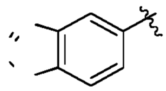

is unsubstituted or substituted with R", R" is $C_{1-3}$ alkyl, $C_{3-6}$ cycloalkyl, where the $C_{1-3}$ alkyl and the $C_{3-6}$ cycloalkyl are independently unsubstituted or substituted with 1 or 2 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, $R_3$ is

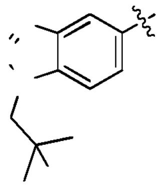

.

In some other embodiments, $R_3$ is

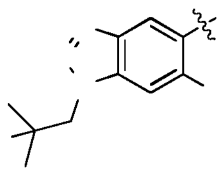

.

In some other embodiments, $R_3$ is

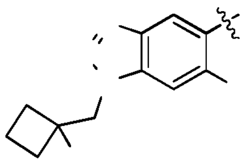

.

In some other embodiments, $R_3$ is

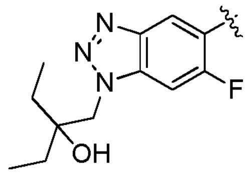

.

In some other embodiments, $R_3$ is

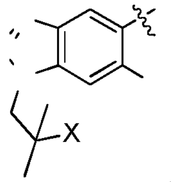

.

In some other embodiments, $R_3$ is

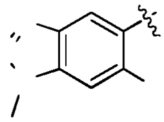

.

In some other embodiments, $R_3$ is

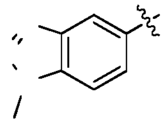

.

In some other embodiments, $R_3$ is

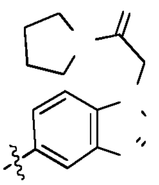

.

In some other embodiments, $R_3$ is

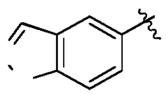

,

the

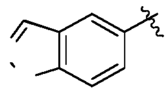

is substituted with R", R" is $C_{1-3}$ alkyl, $C_{3-6}$ cycloalkyl, where the $C_{1-3}$ alkyl and the $C_{3-6}$ cycloalkyl are independently unsubstituted or substituted with 1 or 2 substituents, and the substituents are independently selected from deuterium, F, Cl, Br, CN, =O, -$OR^a$, -$NR^bR^c$, $C_{1-6}$ alkyl, $C_{1-6}$ haloalkyl, or -CO-$NR^bR^c$.

In some other embodiments, $R_4$ is F, Cl, or Br.

In some other embodiments, R' is independently H, deuterium, F, Cl, Br, or -$NR^bR^c$.

In some other embodiments, R" is independently H, deuterium, F, Cl, Br, CN, $NO_2$, =O, -$OR^a$, -$NR^bR^c$, $C_{1-3}$ alkyl, $C_{3-6}$ cycloalkyl, where the $C_{1-3}$ alkyl and the $C_{3-6}$ cycloalkyl are independently unsubstituted or substituted with 1, 2, or 3 substituents, and the substituents are independently selected from deuterium, F, Cl, Br, CN, =O, -$OR^a$, -$NR^bR^c$, $C_{1-6}$ alkyl, $C_{1-6}$ haloalkyl, or -CO-$NR^bR^c$.

In some other embodiments, as in the compound having the structure as shown in Formula (VI), Formula (VII), Formula (VIII), or Formula (IX), R" is $C_{1-3}$ alkyl, $C_{3-6}$ cycloalkyl, where the $C_{1-3}$ alkyl and the $C_{3-6}$ cycloalkyl are independently unsubstituted or substituted with 1 or 2 substituents, and the substituents are independently selected from

deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

In some other embodiments, $R^a$, $R^b$, and $R^c$ are independently H, deuterium, methyl, ethyl, isopropyl, n-propyl, n-butyl, tert-butyl, $C_{1-3}$ haloalkyl, heterocyclyl composed of 3-6 atoms, or $R^b$, $R^c$ and nitrogen atoms connected thereto form a heterocyclic ring composed of 3-6 atoms, where the methyl, the ethyl, the isopropyl, the n-propyl, the n-butyl, the tert-butyl and the heterocyclic ring composed of 3-6 atoms are independently unsubstituted or substituted with 1, 2, or 3 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

In another aspect of this application, this application provides a compound, which has one of the following structures or is a stereoisomer, tautomer, oxynitride, solvate, metabolite, pharmaceutically acceptable salt or prodrug having the following structures:

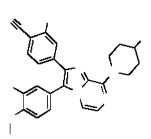
1
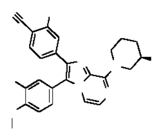
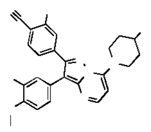
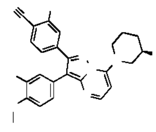
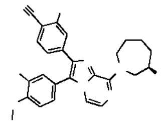

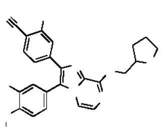
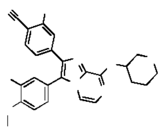
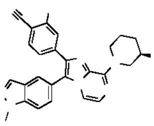
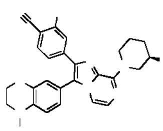
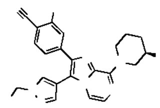
1

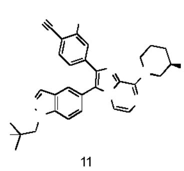
11
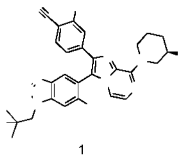
1
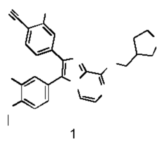
1
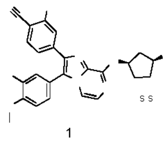
S S
1
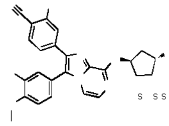
S S S
1

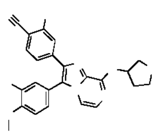
1
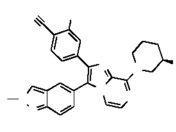
1
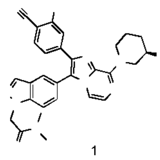
1
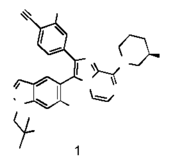
1
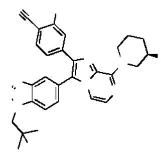

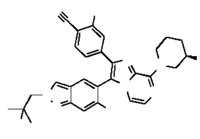
1
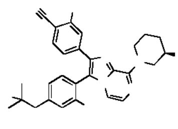
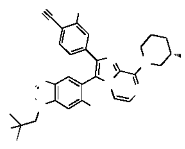
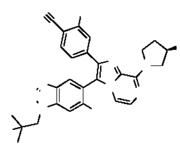
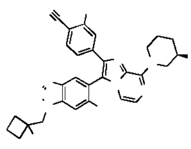

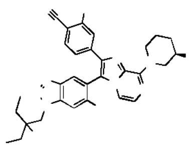
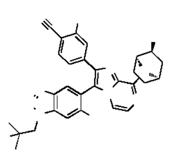
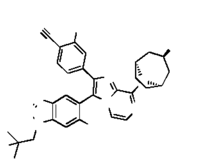

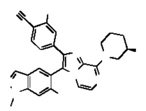
S
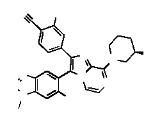
S
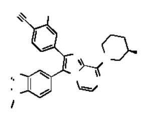
S
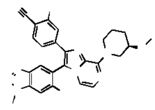
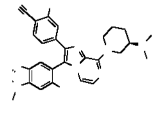

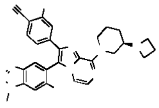
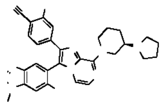
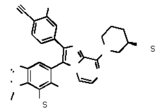
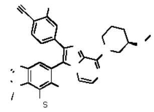
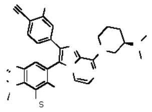

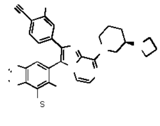
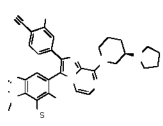
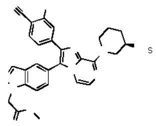
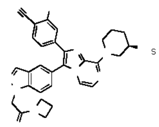
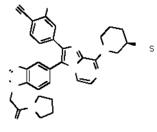

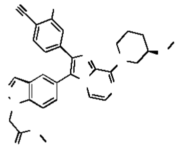
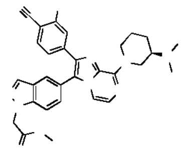
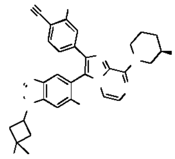
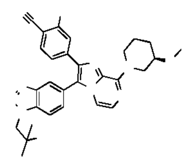
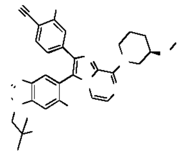

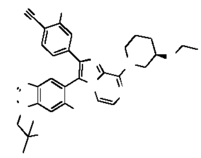
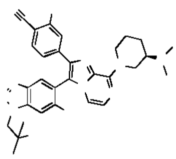
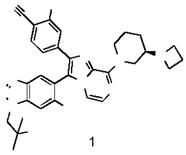

1

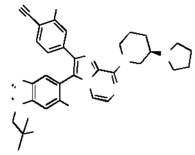
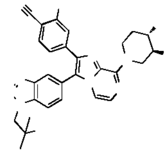

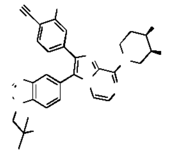
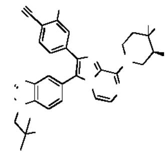
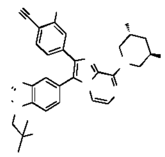
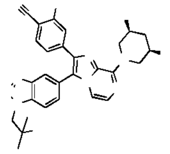
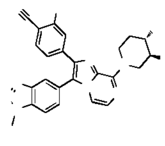

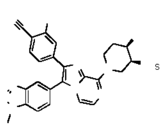
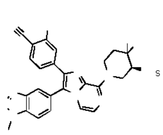
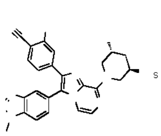
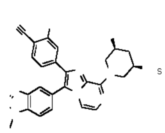
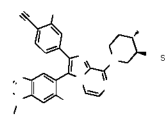

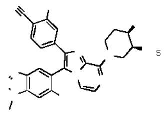
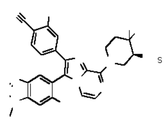
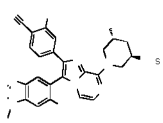
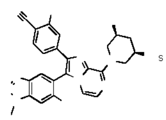
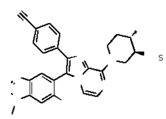

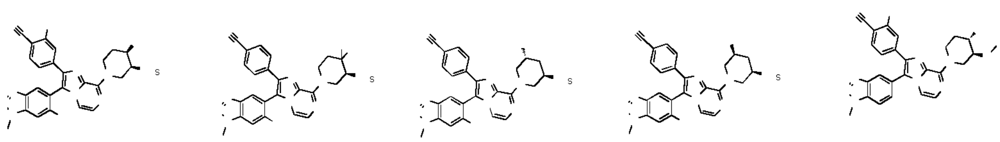

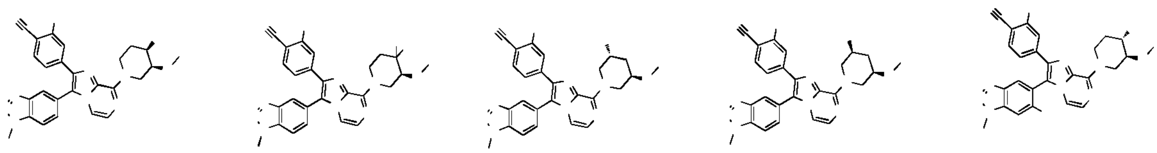

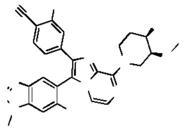 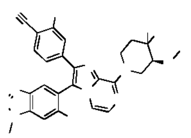 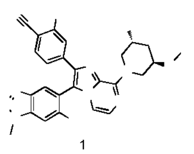
1
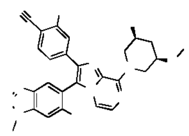 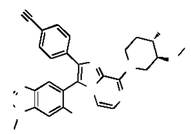

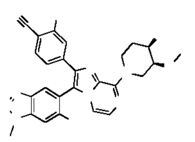 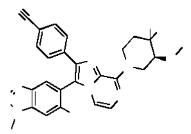 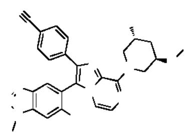 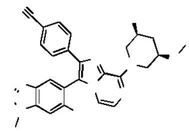 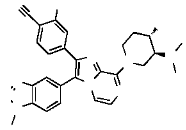

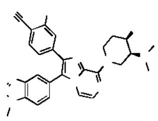 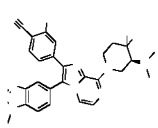 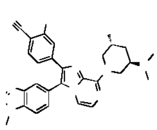 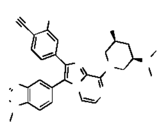 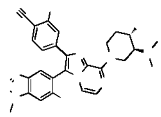

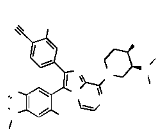 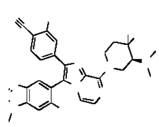 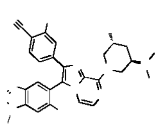 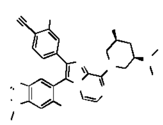 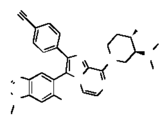

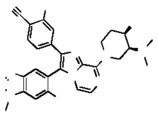 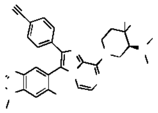 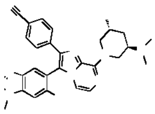 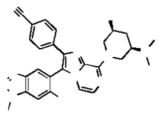 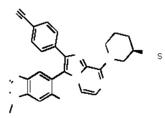

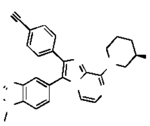 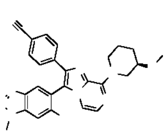 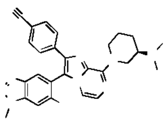 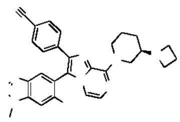 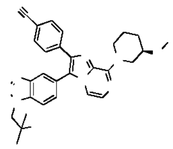

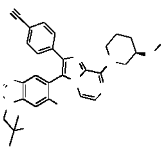 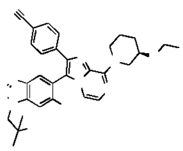 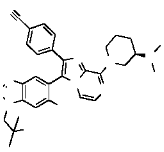 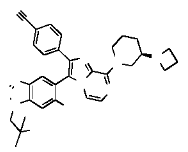 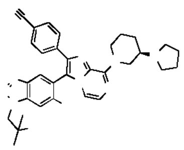.

In some other embodiments, this application relates to a pharmaceutical composition, which includes an effective amount of the compound as described above.

In some other embodiments, the pharmaceutical composition further includes: a pharmaceutically acceptable carrier, an adjuvant, a medium or a combination thereof.

In some other embodiments, the pharmaceutical composition further includes one or more therapeutic agents, and the therapeutic agent is selected from other anti-tumor drugs.

In some other embodiments, the therapeutic agent is an anti-mitotic drug, an alkylating agent, an anti-metabolic drug, a topoisomerase inhibitor, an estrogen receptor regulator, an androgen receptor regulator, a small-molecule inhibitor targeting protein kinase, and an antibody drug targeting protein kinase.

In some other embodiments, the anti-mitotic drug is paclitaxel, vincristine.

In some other embodiments, the alkylating agent is cisplatin, oxaliplatin, carboplatin, or cyclophosphamide.

In some other embodiments, the anti-metabolic drug is gemcitabine, 5-fluorouracil, or methotrexate.

In some other embodiments, the topoisomerase inhibitor is epipodophyllotoxin, etoposide, topotecan, or camptothecin.

In some other embodiments, the estrogen receptor regulator is tamoxifen or fulvestrant.

In other embodiments, the androgen receptor regulator is bicalutamide.

In some other embodiments, the small-molecule inhibitor targeting protein kinase is dasatinib, bosutinib, gefitinib, erlotinib, lapatinib, imatinib, nilotinib, sorafenib, tipifarnib, sunitinib, axitinib.

In some other embodiments, the antibody drug targeting protein kinase is trastuzumab, panitumumab, cetuximab.

In some other embodiments, this application relates to use of the compound as described above or the pharmaceutical composition as described above in preparation of a medicine, and the medicine is used for preventing, disposing, treating, or alleviating a disease associated with overexpression or hyperactivity of LSD1 in a patient.

In some other embodiments, the disease associated with overexpression of LSD1 is a tumor.

In some other embodiments, the tumor is papillary thyroid carcinoma, breast cancer, gastric cancer, bronchogenic carcinoma, lung cancer, acute myeloid leukemia, small cell lung cancer, prostate cancer, or non-hodgkin lymphoma.

In some other embodiments, this application relates to use of the compound as described above or the pharmaceutical composition as described above in preparation of a medicine, and the medicine is used for inhibiting LSD1.

In some embodiments, the salt refers to a pharmaceutically acceptable salt. The term "pharmaceutically acceptable" refers to that a substance or composition is chemically and/or toxicologically compatible with other ingredients of a preparation and/or mammals receiving treatment with the same.

The compound of this application further includes other salts of the compound. The other salts may not be pharmaceutically acceptable salts, and may be used for preparing and/or purifying the compound of this application and/or separating an intermediate of an enantiomer of the compound of this application.

Medicinal acid addition salts can be formed with inorganic acids and organic acids, for example, acetate, aspartate, benzoate, benzenesulfonate, bromide/hydrobromide, bicarbonate/carbonate, bisulfate/sulfate, camphorsulfonate, chloride/hydrochloride, chlorophylline salt, citrate, edisylate, fumarate, glucoheptonate, gluconate, glucuronate, hippurate, hydroiodate/iodide, hydroxyethyl sulfonate, lactate, lacturonate, lauryl sulfate, malate, maleate, malonate, mandelate, methanesulfonate, methylsulfate, naphthoate, naphthalenesulfonate, nicotinate, nitrate, octadecanoate, oleate, oxalate, palmitate, perpate, phosphate/hydrophosphate/dihydric phosphate, polygalactodate, propionate, stearate, succinate, sulfosalicylate, tartrate, toluenesulfonate and trifluoroacetate.

The inorganic acids from which the salts can be derived include, for example, hydrochloric acid, hydrobromic acid, sulfuric acid, nitric acid, phosphoric acid and the like.

The organic acids from which the salts can be derived include, for example, acetic acid, propionic acid, hydroxyacetic acid, oxalic acid, maleic acid, malonic acid, succinic acid, fumaric acid, tartaric acid, citric acid, benzoic acid, mandelic acid, methanesulfonic acid, ethanesulfonic acid, p-toluenesulfonic acid, sulfosalicylic acid and the like.

Medicinal alkali addition salts can be formed with inorganic alkalies and organic alkalies.

The inorganic alkalies from which the salts can be derived include, for example, ammonium salts and metals from the I family to the XII family in the periodic table of elements. In some embodiments, the salts are derived from sodium, potassium, ammonium, calcium, magnesium, iron, silver, zinc, and copper; and particularly suitable salts include ammonium salts, potassium salts, sodium salts, calcium salts, and magnesium salts.

The organic alkalies from which the salts can be derived include primary amines, secondary amines and tertiary amines, substituted amines including natural substituted amines, cyclic amines, basic ion exchange resins and the like. Some organic amines include, for example, isopropylamine, benzathine, cholinate, diethanolamine, diethylamine, lysine, meglumine, piperazine, and tromethamine.

The medicinal salts of this application may be synthesized by parent compounds and alkaline or acidic components according to conventional chemical methods. Generally, such salts may be prepared by making free acid forms of these compounds react with a stoichiometric amount of suitable alkalies (such as hydroxides, carbonates and bicarbonates of Na, Ca, Mg, or K), or making free alkali forms of these compounds react with a stoichiometric amount of suitable acids. Such reactions are usually carried out in water, an organic solvent or a mixture thereof. Generally, non-aqueous media, such as ether, ethyl acetate, ethanol, isopropanol or acetonitrile, are used in appropriate cases. For example, in "Remington's Pharmaceutical Sciences", 20th edition, Mack Publishing Company, Easton, Pa., (1985); and "Handbook of Medicinal Salts: Properties, Selection, and Use", Stahl and Wermuth (Wiley-VCH, Weinheim, Germany, 2002), lists of other suitable salts may be found.

Moreover, the compound of this application, including a salt thereof, may also be obtained in a hydrate form thereof, or may include other solvents for crystallization. The compound of this application may be inherently or designed to form a solvate having a medicinal solvent (including water). Therefore, this application is intended to include a solvated form and an unsolvated form.

Any structural formula provided in this application is also intended to represent an unlabeled form and an isotope labeled form of these compounds. Isotope labeled compounds have the structures as described in the general formulas provided in this application, except that one or more atoms are substituted with a selected atomic weight or mass number of atoms. Exemplary isotopes that may be introduced into the compound of this application include isotopes of hydrogen, carbon, nitrogen, oxygen, phosphorus, sulfur, fluorine, and chlorine, such as $^{2}H$ , $^{3}H$, $^{11}C$, $^{13}C$, $^{14}C$, $^{15}N$, $^{18}F$, $^{31}P$, $^{32}P$, $^{36}S$, $^{37}Cl$, or $^{125}I$.

In another aspect, the compound of this application includes compounds defined in this application labeled

with various isotopes, for example, compounds having radioactive isotopes such as $^3H$, $^{14}C$ and $^{18}F$, and compounds having non-radioactive isotopes such as $^2H$ and $^{13}C$. The isotope labeled compounds can be used for metabolic study (using $^{14}C$), reaction kinetics study (using, for example, $^2H$ or $^3H$), or detection or imaging technologies such as positron emission tomography (PET), single photon emission computed tomography (SPECT) for determining distribution of a drug or a substrate tissue, or radiation therapy for patients. $^{18}F$ labeled compounds are particularly ideal for PET or SPECT study. An isotope labeled compound as shown in Formula (I) may be prepared by using a conventional technology familiar to a person skilled in the art or replacing previously used unlabeled reagents with suitable isotope labeled reagents as described in the embodiments and preparation processes in this application.

In addition, substitution of heavier isotopes, particularly deuterium (namely $^2H$ or D), can provide certain therapeutic benefits which are brought by higher metabolic stability. For example, the benefits are brought by increase of the half-life in the body, decrease of dose requirements, or improvement of therapeutic indexes. It is to be understood that deuterium in this context is regarded as a substituent of the compound as shown in Formula (I). The concentration of the heavier isotopes, particularly deuterium, can be defined by an isotope enrichment factor. The term "isotope enrichment factor" used in this application refers to the ratio of the isotopic abundance to the natural abundance of a specified isotope. When deuterium is specified as a substituent of the compound of this application, the isotope enrichment factor of the compound for various specified deuterium atoms is at least 3500 (52.5% deuterium doped at various specified deuterium atoms), at least 4000 (60% deuterium doped), at least 4500 (67.5% deuterium doped), at least 5000 (75% deuterium doped), at least 5500 (82.5% deuterium doped), at least 6000 (90% deuterium doped), at least 6333.3 (95% deuterium doped), at least 6466.7 (97% deuterium doped), at least 6600 (99% deuterium doped), or at least 6633.3 (99.5% deuterium doped). A medicinal solvate of this application includes solvates in which crystalline solvents may be substituted with an isotope, such as $D_2O$, acetone-$d_6$, or DMSO-$d_6$.

**Composition, preparation and administration of the compound of this application**

This application provides a pharmaceutical composition. The pharmaceutical composition includes an effective amount of the compound described in this application or a stereoisomer thereof. According to a specific embodiment of this application, the pharmaceutical composition further includes at least one pharmaceutically acceptable carrier, diluent, adjuvant or medium, and optionally includes other therapeutic and/or preventive ingredients. In some embodiments, the pharmaceutical composition includes an effective amount of at least one pharmaceutically acceptable carrier, diluent, adjuvant or medium.

The pharmaceutically acceptable carrier may include an inert ingredient not excessively inhibiting the biological activity of the compound. The pharmaceutically acceptable carrier is biocompatible, such as non-toxic, non-inflammatory, non-immunogenic, or free of other adverse reactions or side effects after administration to a patient. A standard pharmaceutical technology can be used.

As described in this application, the pharmaceutical composition or a pharmaceutically acceptable composition of this application further includes a pharmaceutically acceptable carrier, adjuvant or excipient. As applied in this application, any solvent, diluent, liquid excipient, dispersant, suspension agent, surfactant, isotonic agent, thickener, emulsifier, preservative, solid binder or lubricant and the like suitable for a particular target dosage form are involved. According to Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York, various carriers used in preparation of the pharmaceutically acceptable composition and publicly known preparation methods thereof are disclosed. Except for conventional carrier media which are incompatible with the compound of this application, such as leading to adverse biological effects or harmful interactions with any other components in the pharmaceutically acceptable composition, any other conventional carrier media and use thereof are also considered as falling within the scope of this application.

Some examples of substances that may be used as the pharmaceutically acceptable carrier include, but are not limited to, ion exchangers, alumina, aluminum stearate, lecithin, serum proteins (such as human serum albumin), buffer substances (such as Tween 80, phosphate, glycine, sorbic acid or potassium sorbate), metaglyceride mixtures of saturated vegetable fatty acids, water, salts or electrolytes (such as protamine sulfate, disodium hydrogen phosphate, potassium hydrogen phosphate, sodium chloride or zinc salts), silica gel, magnesium trisilate, polyvinylpyrrolidone, polyacrylates, wax, polyethylene-polypropylene oxide-block copolymers, methyl cellulose, hydroxypropyl methyl cellulose, lanolin, saccharides (such as lactose, glucose and sucrose), starch (such as corn starch and potato starch), cellulose and derivatives thereof (such as sodium carboxymethyl cellulose, ethyl cellulose and cellulose acetate), powdered tragacanth gum, malt, gel, talc, excipients (such as cocoa butter and suppository wax), oils (such as peanut oil, cottonseed oil, safflower oil, sesame oil, olive oil, corn oil and soybean oil), glycols (such as propylene glycol or polyethylene glycol), esters (such as ethyl oleate and ethyl dodecanoate), agar, buffers (such as magnesium hydroxide and aluminum hydroxide), alginic acid, apyrogenic raw water, isotonic brine, Ringer's solutions, ethanol, phosphate buffers and other non-toxic compatible lubricants (such as sodium lauryl sulfate and magnesium stearate), and the composition may also

include colorants, anti-bonding agents, coating agents, sweeteners, fragrances, preservatives and antioxidants based on the judgement of a formulator.

The compound or composition of this application may be administered in any suitable manner, and the compound and a pharmaceutically acceptable composition may be administered to humans or other animals orally, rectally, parenterally, intracisternally, intravaginally, intraperitoneally, topically (as by a powder, ointment or drop), or orally as an oral or nasal spray based on the severity of a disease treated.

A liquid dosage form for oral use includes, but is not limited to, pharmaceutically acceptable emulsions, microemulsions, solutions, suspensions, syrups, and elixirs. In addition to active compounds, the liquid dosage form may include an inert diluent commonly used in the art, such as water or other solvents, solubilizers and emulsifiers, such as ethanol, isopropanol, ethyl carbonate, ethyl acetate, benzyl alcohol, benzyl benzoate, propylene glycol, 1,3-butanediol, dimethylformamide, oils (especially cottonseed oil, peanut oil, corn oil, germ oil, olive oil, castor oil and sesame oil), glycerol, tetrahydrofurfuryl alcohol, polyethylene glycol, fatty acid esters of sorbitan and mixtures thereof. In addition to the inert diluent, an oral composition may also include an adjuvant, such as wetting agents, emulsifiers, suspension agents, sweeteners, flavoring agents and fragrances.

An injectable preparation, such as sterile injectable water or oil suspension, may be prepared by using a suitable dispersant or wetting agent and a suspension agent according to known technologies. A sterile injectable preparation may also be a sterile injectable solution, suspension or emulsion in a non-toxic parenterally acceptable diluent or solvent, such as a solution in 1,3-butanediol. In acceptable media and solvents, water, Ringer's solutions, U.S.P. and isotonic sodium chloride solutions may be used. In addition, a sterile non-volatile oil is used as a solvent or suspension medium according to conventions. Thus, any odorless non-volatile oil, including synthetic monoglyceride or diglyceride, may be used. In addition, fatty acids, such as octadecenoic acid, are used for preparing injections.

For example, injectable preparations may be sterilized by filtration with a bacterial retention filter or by adding a bactericide in the form of a sterile solid composition that can be dissolved or dispersed in sterile water or other sterile injectable media before use.

In order to prolong the effect of the compound or composition of this application, absorption of the compound by subcutaneous or intramuscular injection is usually expected to be slowed down. This purpose can be achieved by using a liquid suspension of a crystal or an amorphous substance having poor water solubility. Then, the absorption rate of the compound depends on the dissolution rate, and the dissolution rate depends on the size and crystalline form of the crystal. Alternatively, absorption of the compound applied parenterally is slowed down by dissolving or suspending the compound in an oil medium. An injectable storage form of the compound is prepared by forming a microcapsule matrix of the compound in a biodegradable polymer such as polylactide-polyglycolic acid. The release rate of the compound can be controlled based on the ratio of the compound to the polymer and properties of the particular polymer used. Other examples of the biodegradable polymer include polyortho esters and polyanhydrides. An injectable storage preparation may also be prepared by trapping the compound in liposomes or microemulsions compatible with body tissues.

A composition administered rectally or intravaginally can be obtained by particularly mixing the compound of this application with a suitable non-irritating excipient or carrier, such as cocoa butter, polyethylene glycol or a suppository prepared from suppository wax. The excipient or carrier is a solid at ambient temperature or a liquid at body temperature, and thus is melt in the rectum or vaginal cavity to release the active compound.

An oral solid dosage form includes capsules, tablets, pills, powders and granules. In this solid dosage form, the active compound is mixed with at least one inert pharmaceutically acceptable excipient or carrier such as sodium citrate or dicalcium phosphate and/or a) fillers or expanders, such as starch, lactose, sucrose, glucose, mannitol and silicic acid, b) binders, such as carboxymethyl cellulose, alginate, gel, polyvinylpyrrolidone, sucrose and arabic gum, c) humectants, such as glycerol, d) disintegrators, such as agar, calcium carbonate, potato or cassava starch, alginic acid, certain silicates and sodium carbonate, e) solution blockers, such as paraffin, f) absorption accelerators, such as quaternary ammonium compounds, g) wetting agents, such as cetyl alcohol and glyceryl monostearate, h) absorbents, such as kaolin and bentonite, and i) lubricants, such as talc, calcium stearate, magnesium stearate, solid polyethylene glycol, sodium lauryl sulfate and mixtures thereof. In cases of capsules, tablets and pills, the dosage form may also include buffers.

Similar types of solid compositions may also be used as fillers in soft and hard gel capsules by using lactose, milk saccharides, polymer polyethylene glycol and other excipients. The solid dosages forms, such as tablets, troches, capsules, pills and granules, can be prepared by coatings and shells, such as enteric coatings and other coatings well-known in the pharmaceutical art. These solid dosages forms may optionally include an emulsion and may also have properties of a composition so that only an active ingredient is optionally released in a delayed manner, or preferably released in a part of the intestines. Examples of an embedding composition that can be used include polymers and wax. Similar types of solid compositions may also be used as fillers in soft and hard gel capsules by using lactose, milk saccharides, polymer polyethylene glycol and other excipients.

An active compound may also be in a microsealed form including one or more of the above excipients. The

solid dosages forms, such as tablets, troches, capsules, pills and granules, can be prepared by coatings and shells, such as enteric coatings, controlled release coatings and other coatings well-known in the pharmaceutical art. In this solid dosage form, the active compound may be mixed with at least one inert diluent, such as sucrose, lactose, or starch. Generally, in addition to the inert diluent, this dosage form may also include other substances, such as tablet lubricants and other tablet adjuvants, such as magnesium stearate and microcrystalline cellulose. In cases of capsules, tablets and pills, the dosage form may also include buffers. These solid dosages forms may optionally include an emulsion and may also have properties of a composition so that only an active ingredient is optionally released in a delayed manner, or preferably released in a part of the intestines. Examples of an embedding composition that can be used include polymers and wax.

Topical or transdermal application forms of the compound of this application include ointments, soft ointments, emulsion ointments, lotions, gels, powders, solutions, sprays, inhalants or patches. Under sterile conditions, the active compound is mixed with a pharmaceutically acceptable carrier and any required preservatives or possible required buffers. Ophthalmic preparations, ear drops and eye drops are also considered as falling within the scope of this application. In addition, use of skin patches having the added advantage of controlling the delivery of the compound to the body is considered in this application. This dosage form may be prepared by dissolving or dispersing the compound in an appropriate medium. An absorption promoter can also be used for increasing the flow of the compound in the skin. The rate may be controlled by providing a rate control membrane or dispersing the compound in a polymer matrix or gel.

The composition of this application may also be applied orally, parenterally, or topically, rectally, nasally, orally or vaginally by inhalation of a spray, or applied by implanting a medicine box. The term "parenterally" used in this application includes, but is not limited to, subcutaneous, intravenous, muscular, intraarticular, intrasynovial, intrasternal, intrathecal, intrahepatic, intrafocal and intracranial injection or infusion technologies. In particular, a composition is applied orally, intraperitoneally, or intravenously.

A sterile injectable form of the composition of this application may be water or an oil suspension. The suspension may be prepared by using a suitable dispersant or wetting agent and a suspension agent according to known technologies in the art. A sterile injectable preparation may also be a sterile injectable solution or suspension in a non-toxic parenterally acceptable diluent or solvent, such as a solution in 1,3-butanediol. In acceptable media and solvents, water, Ringer's solutions and isotonic sodium chloride solutions may be used. In addition, a sterile non-volatile oil is used as a solvent or suspension medium according to conventions. Thus, any odorless non-volatile oil, including synthetic monoglyceride or diglyceride, may be used. In addition, natural pharmaceutically acceptable oils, such as olive oil or castor oil, particularly in a polyoxyethylene form, fatty acids such as octadecenoic acid and glyceride derivatives thereof are used for preparation of injections. These oil solutions or suspensions may also include long-chain alcohol diluents or dispersants, such as carboxymethyl cellulose or similar dispersants commonly used in preparation of pharmaceutically acceptable dosage forms, including emulsions and suspensions. Other commonly used surfactants, such as Tweens, Spans and other emulsifiers or bioavailability enhancers commonly used for production of pharmaceutically acceptable solids, liquids or other dosage forms may also be used for preparation purposes.

The pharmaceutical composition of this application may be taken orally in any orally acceptable dosage form, including but not limited to capsules, tablets, aqueous suspensions or solutions. In a case of oral tablets, a commonly used carrier includes, but is not limited to, lactose and starch. A lubricant, such as magnesium stearate, is usually added. In a case of oral capsules, a useful diluent includes lactose and dried corn starch. When a water suspension is required orally, an active ingredient is combined with an emulsifier and a suspension agent. Some sweeteners, flavoring agents or colorants may also be added as required.

Alternatively, the pharmaceutical composition of this application may be administered in a suppository form for rectal use. The pharmaceutical composition may be prepared by mixing a reagent with a non-irritating excipient, and the excipient is a solid at room temperature or a liquid at rectal temperature, and thus is melt in the rectum to release a drug. Such substance includes, but is not limited to, cocoa butter, beeswax and polyethylene glycol.

In particular, the pharmaceutical composition of this application may also be administered topically when a therapy target includes areas or organs easily accessible to topical drops, including eyes, skin or lower intestines with diseases. A topical preparation suitable for each of these areas or organs is easy to prepare.

A rectal suppository preparation (see as above) or a suitable enema preparation can be topically dropped on lower intestines. Topical skin patches can also be used.

For topical dropping, the pharmaceutical composition may be formulated as a suitable ointment containing an active component capable of being suspended or dissolved in one or more carriers. A carrier suitable for topical dropping of the compound of this application includes, but is not limited to, mineral oil, vaseline oil, albolene, propylene glycol, polyoxyethylene, polypropylene oxide compounds, emulsified wax and water. Alternatively, the pharmaceutical composition may be formulated as a suitable lotion or emulsion ointment containing an active component capable of being suspended or dissolved in one or more pharmaceutically acceptable carriers. Suitable carriers include, but are not limited to, mineral oil, sorbitan monostearate, polysorbate 60, cetyl ester wax, cetearyl alcohol, 2-octyldodecyl alcohol, benzyl alcohol and water.

For ophthalmic use, the pharmaceutical composition may be formulated as a micro-powdered suspension in isotonic pH-regulated sterile saline, or particularly a solution in isotonic pH-regulated sterile saline, with or without a preservative such as benzalkonium chloride. Alternatively, for ophthalmic use, the pharmaceutical composition may be formulated as an ointment, such as vaseline.

The pharmaceutical composition may also be administered by a nasal vaporization spray or inhalant. This composition is prepared by technologies well-known in the pharmaceutical art, and is prepared into a solution in saline by using benzyl alcohol and other suitable preservatives, absorption promoters for increasing the bioavailability, fluorocarbons and/or other conventional solubilizers or dispersants.

The compound used in a method of this application may be formulated in a unit dosage form. The term "unit dosage form" refers to a physical discrete unit suitable for use as a unit dose for a curee, and each unit includes a predetermined amount of an active substance calculated to achieve a desired effect, which is optionally combined with a suitable pharmaceutical carrier. The unit dosage form may be used as one of a single dose per day or multiple doses per say (such as about 1-4 times or more times per day). When the unit dosage form is used in multiple doses per day, the various doses may be in the same or different unit dosage forms.

**Use of the compound and composition of this application**

The compound and the pharmaceutical composition provided in this application can be used for preparing a medicine for preventing, treating or alleviating a disease associated with overexpression or hyperactivity of LSD1 in a patient. Preferably, the disease associated with overexpression or hyperactivity of LSD1 is a tumor. For example, the tumor includes papillary thyroid carcinoma, breast cancer, gastric cancer, bronchogenic carcinoma, lung cancer, acute myeloid leukemia, small cell lung cancer, prostate cancer, or non-hodgkin lymphoma.

This application further provides use of the compound or the pharmaceutical composition thereof in preparing a medicine for inhibiting LSD1.

This application provides a method for treating, preventing or alleviating a disease caused by overexpression or hyperactivity of LSD1. The method includes administering a therapeutic effective amount of the compound or the pharmaceutical composition thereof to a patient in need of treatment. The disease caused by overexpression or hyperactivity of LSD1 is a tumor, such as papillary thyroid carcinoma, breast cancer, gastric cancer, bronchogenic carcinoma, lung cancer, acute myeloid leukemia, small cell lung cancer, prostate cancer, or non-hodgkin lymphoma. In addition, the compound or the pharmaceutical composition thereof provided in this application may be administered in combination with other therapy methods or therapeutic agents. These substances may be administered simultaneously, sequentially or at regular intervals.

The dose of the compound or the pharmaceutical composition required for treatment, prevention or alleviation and other effects usually depends on the specific compound administered, the patient, a specific disease or conditions and severity thereof, an administration route, the administration frequency and the like, and is required to be determined by an attending physician based on specific situations. For example, the compound or the pharmaceutical composition provided in this application may be applied intravenously once a week or even at longer intervals.

In summary, this application provides a novel compound, and the compound can be used as an LSD1 inhibitor. The compound of this application is suitable for being prepared into medicines in a variety of dosage forms, and can be widely used for treating tumors, such as papillary thyroid carcinoma, breast cancer, gastric cancer, bronchogenic carcinoma, lung cancer, acute myeloid leukemia, small cell lung cancer, prostate cancer, or non-hodgkin lymphoma.

The compound and the pharmaceutical composition of this application have therapeutic benefits to humans, and can also be used for treating mammals in pets, introduced varieties of animals and farm animals by veterinarians. Examples of other animals include horses, dogs and cats. Herein, the compound of this application includes a pharmaceutically acceptable derivative thereof.

**General synthesis process**

In order to describe this application, embodiments are listed below. However, it is to be understood that this application is not limited to these embodiments, and only methods for implementing this application are provided.

Generally, the compound of this application can be prepared by the method described in this application. Unless further specified, the substituents are defined as shown in Formula (I). The following reaction solutions and embodiments are used for further illustrating the contents of this application.

A person skilled in the art knows that chemical reactions described in this application may be used for properly preparing many other compounds of this application, and all other methods for preparing the compound of this application are considered as falling within the scope of this application. For example, non-exemplary compounds of this application may be successfully synthesized by a person skilled in the art according to a modification method, such as appropriately protecting an interfering group, using other known reagents in addition to those described in this application, or making

some routine modifications to reaction conditions. In addition, reactions disclosed in this application or known reaction conditions are also considered as being applicable to preparation of other compounds of this application.

Unless otherwise indicated, all temperatures in the embodiments described below are set at Celsius degree. Solvents used in this application are commercially available. Unless otherwise indicated, reagents purchased from commercial suppliers such as Aldrich Chemical Company, Arco Chemical Company and Alfa Chemical Company, J&K Scientific Ltd. are used without further purification.

The LCMS model used for detection in the following embodiments is Agilent 1200 Series, Agilent 1260 Infinity 2, or Agilent 1260-6125B, and the nuclear magnetic resonance model is as follows: Bruker AVANCE 3 400MHZ Ultra shield TM Digital NMR.

Compounds are named according to conventional naming rules in the art or named by using ChemDraw software.

Experimental steps for preparing the compounds disclosed in this application are listed in the following synthesis solutions.X, $X_1$, $X_2$, $X_3$, $X_4$, $X_5$, $X_6$, $X_7$, $X_8$, $R_1$, $R_2$, $R_3$, $R_4$, R', R", $R^a$, $R^b$, $R^c$, ring A, ring B, m, n, s, t, and v are defined as described in this application.

**Synthesis solution 1**

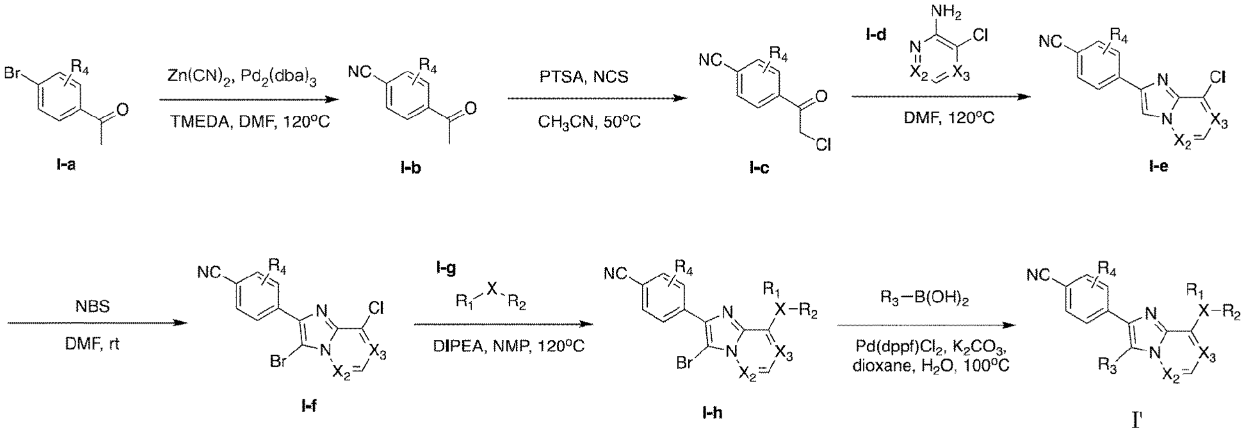


A 4-bromoacetylbenzene compound I-a is subjected to a reaction with $Zn(CN)_2$ under catalysis of palladium to obtain a corresponding 4-cyanoacetylbenzene compound I-b, and I-b is subjected to a reaction with PTSA and NCS to obtain an intermediate I-c. Then, I-c is subjected to a ring-closing reaction with an amino heterocyclic compound at high temperature to obtain an intermediate I-e, I-e is brominated to obtain I-f, and I-f is subjected to a nucleophilic substitution reaction with I-g to obtain I-h. Finally, I-h is coupled with a corresponding boric acid compound under catalysis of palladium to obtain the compound I'.

**Synthesis solution 2**

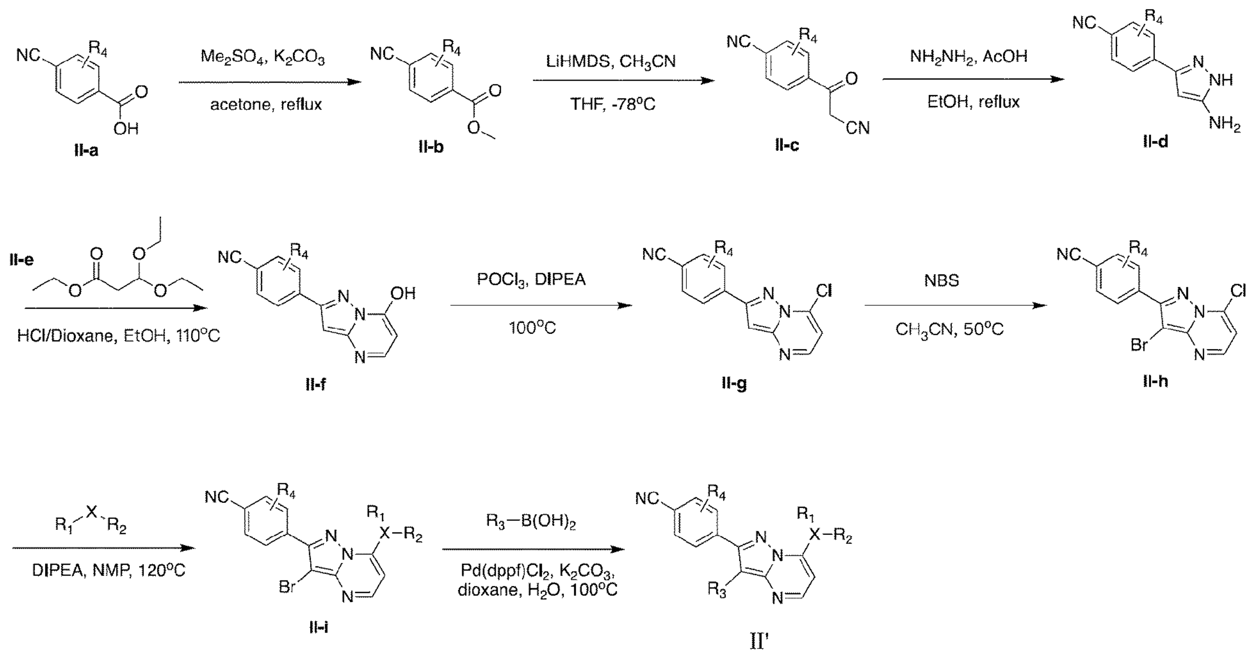


A 4-cyanobenzoic acid compound II-a is methylated under dimethyl sulfate to obtain an intermediate II-b, II-b is subjected to a reaction with acetonitrile to obtain II-c, II-c is subjected to a ring-closing reaction with hydrazine to obtain II-d, and II-d is further subjected to a ring-closing reaction with II-e to obtain an intermediate II-f. Then, II-f is subjected to a reaction with phosphorus oxychloride to obtain a corresponding chloride II-g, II-g is brominated with NBS to obtain II-h, and II-h is subjected to a nucleophilic substitution reaction to obtain II-i. II-i is further subjected to a suzuki coupling reaction with a corresponding boric acid compound to obtain II'.

DESCRIPTION OF EMBODIMENTS

The following embodiments are intended to describe this application, rather than to limit the scope of this application.

## Preparation embodiments

In the following preparation embodiments, preparation processes of compounds of this application are described in detail by the applicant with some of the compounds of this application as examples.

## Embodiment 1

Synthesis of 4-(8-(4-aminopiperidin-l-yl)-3-(3-fluoro-4-methoxyphenyl)imidazo [1,2-a]pyrazin-2-yl)-2-fluorobenzonitrile (compound 1)

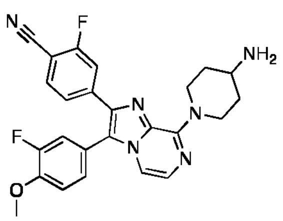

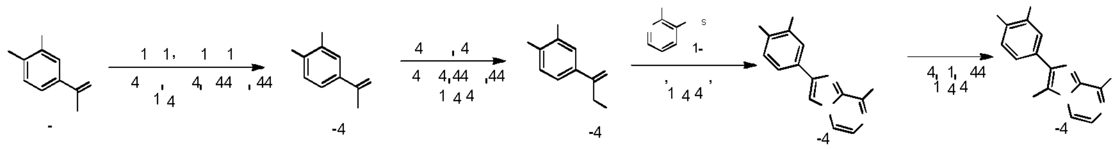

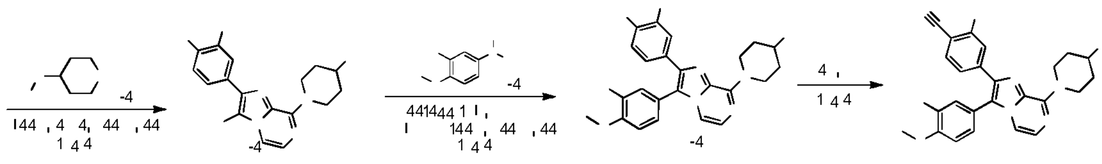

**Step 1**

A compound 1-1 (6 g, 27.65 mmol), $Zn(CN)_2$ (2.03 g, 17.28 mmol), N,N,N',N'-tetramethylethylenediamine (0.7 g, 6.05 mmol), $Pd_2(dba)_3$ (0.79 g, 1.73 mmol) and Xant-Phos (2 g, 6.92 mmol) were dissolved in DMF (50 mL). The reaction system was heated to 120°C and reacted under the protection of nitrogen for 4 h. The reaction system was quenched with water. Extraction was conducted with ethyl acetate (3*100 mL), drying was conducted with anhydrous sodium sulfate, and spin-drying was conducted. Then, column chromatography was conducted (PE:EA=6:1) to obtain a yellow solid compound 1-2 (4.1 g, yield: 90%). $^1$HNMR (400 MHz, DMSO) δ 8.13 (d, J = 6.6 Hz, 1H), 7.97 (dd, J = 26.0, 9.0 Hz, 2H), 2.65 (s, 3H).

**Step 2**

The compound 1-2 (4.1 g, 25.15 mmol) was dissolved in acetonitrile (50 mL), NCS (2.0 g, 15.1 mmol) and PTSA (2.4 g, 12.6 mmol) were added to the reaction system, and a reaction mixture was heated to 80°C and reacted under the protection of nitrogen for 1 h. The reaction system was quenched with water. Extraction was conducted with ethyl acetate (3 * 80 mL), drying was conducted with anhydrous sodium sulfate, and spin-drying was conducted. Then, column chromatography was conducted (PE:EA=5:1) to obtain a yellow oily compound 1-3 (3.5 g, yield: 70%). $^1$H NMR (400 MHz, $CDCl_3$) δ 7.85 (d, J = 9.4 Hz, 1H), 7.81 (dd, J = 8.6, 4.9 Hz, 2H), 4.66 (s, 2H).

**Step 3**

The compound 1-3 (3.5 g, 17.8 mmol) and a compound 1-3A (3.43 g, 17.8 mmol) were dissolved in DMF (50 mL), and a reaction mixture was heated to 120°C and reacted under the protection of nitrogen for 24 h. The reaction system was quenched with water. Extraction was conducted with ethyl acetate (3*80 mL), drying was conducted with anhydrous sodium sulfate, and spin-drying was conducted. Then, column chromatography was conducted (PE:EA=1:1) to obtain a white solid compound 1-4 (1.1 g, yield: 22%). LCMS $(M+H)^+$ = 273.2.

**Step 4**

The compound 1-4 (800 mg, 2.94 mmol) was dissolved in acetonitrile (20 mL), NBS (575.9 mg, 3.23mmol) was added to the reaction system, and a reaction mixture was heated to 40°C and reacted under the protection of nitrogen for 12 h. The reaction system was quenched with water. Extraction was conducted with ethyl acetate (3*40 mL), drying was conducted with anhydrous sodium sulfate, and spin-drying was conducted. Then, column chromatography was conducted (PE:EA=1:1) to obtain a white solid compound 1-5 (450 mg, yield: 43%). LCMS $(M+H)^+$ = 351.1.

**Step 5**

The compound 1-5 (300 mg, 0.86 mmol) was dissolved in NMP (8.0 mL), DIPEA (221.9 mg, 1.72 mmol) and a compound 1-5A (172 mg, 0.86 mmol) were added to the reaction system, and a reaction mixture was heated to 120°C and reacted for 4 h. The reaction system was quenched with water. Extraction was conducted with ethyl acetate (3*30 mL), drying was conducted with anhydrous sodium sulfate, and spin-drying was conducted. Then, column chromatography was conducted (PE:EA=3:1) to obtain a white solid compound 1-6 (150 mg, yield: 33%). LCMS: $(M+H)^+$ = 515.2.

**Step 6**

The compound 1-6 (140 mg, 0.27 mmol), a compound 1-6A (69.46 mg, 0.41 mmol), Pd(dppf)$Cl_2$ (13.91 mg, 0.03 mmol) and $K_2CO_3$ (75.18 mg, 0.54 mmol) were dissolved in 1,4-dioxane/$H_2O$ (4/0.5 mL), the reaction system was heated to 100°C, and a reaction solution was subjected to a reaction under the protection of nitrogen for 5 h. The reaction system was quenched with water. Extraction was conducted with ethyl acetate (3*20 mL), drying was conducted with anhydrous sodium sulfate, and spin-drying was conducted. Then, column chromatography was conducted (PE:EA=1:1) to obtain a white solid compound 1-7 (50 mg, yield: 33%). LCMS: $(M+H)^+$ = 561.3.

**Step 7**

The compound 1-7 (50 mg, 0.09 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 1 (10.5 mg, yield: 22%). LCMS: $(M+H)^+$ = 461.0. $^1$H NMR (400 MHz, DMSO) δ 7.94 (m, 3H), 7.66 (m, 1H), 7.49 (m, 2H), 7.37 (m, 2H), 7.29 (m, 1H), 5.47 (m, 2H), 3.96 (s, 3H), 3.42 (m, 1H), 3.23 (m, 2H), 2.05 (m, 2H), 1.57 (m, 2H). $^{19}$F NMR (376 MHz, DMSO) δ -74.23 (s, 4.73F), -107.94 (s, 1F), -133.25 (s, 1 F).

**Embodiment 2**

Synthesis of (R)-4-(8-(3 -aminopiperidin-1 -yl)-3 -(3 -fluoro-4-methoxyphenyl)imidazo [1,2-a]pyrazin-2-yl)-2-fluorobenzonitrile (compound 2)

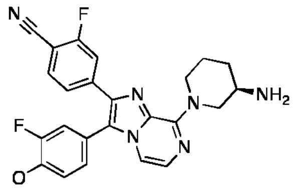

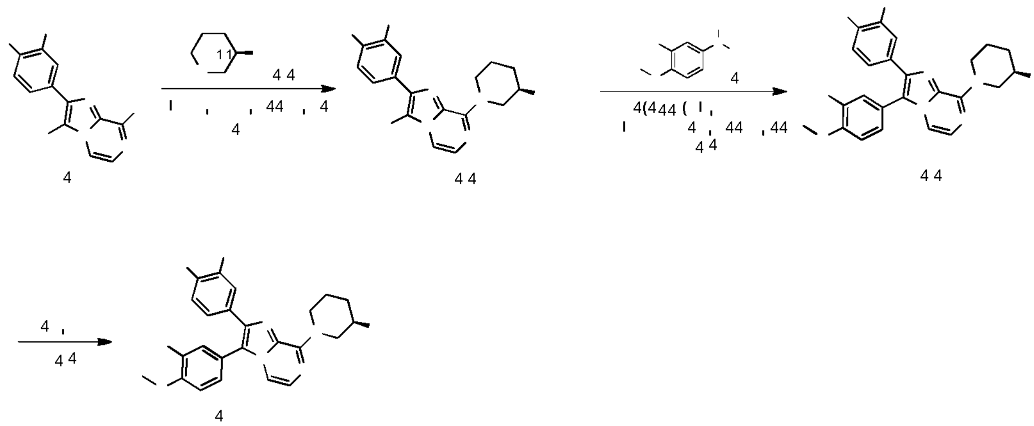

**Step 1**

The compound 2-1 (220 mg, 0.63 mmol) was dissolved in NMP (8.0 mL), DIPEA (162.6 mg, 1.26 mmol) and a compound 2-2 (125.7 mg, 0.63 mmol) were added to the reaction system, and a reaction mixture was heated to 120°C and stirred for a reaction for 4 h. The reaction system was quenched with water. Extraction was conducted with ethyl acetate (3*30 mL), drying was conducted with anhydrous sodium sulfate, and an organic phase was concentrated under reduced pressure to obtain a crude product. Then, the crude product was treated with column chromatography (PE:EA=3:1) to obtain a white solid compound 2-3 (100 mg, yield: 30.1%). LCMS: $(M+H)^+$ = 515.2.

**Step 2**

The compound 2-3 (100 mg, 0.20 mmol), a compound 2-4 (49.61 mg, 0.29 mmol), Pd(dppf)$Cl_2$ (14.22 mg, 0.02 mmol) and $K_2CO_3$ (53.7 mg, 0.39 mmol) were dissolved in 1,4-dioxane/$H_2O$ (4/0.5 mL), the reaction system was heated to 100°C, and a reaction solution was subjected to a reaction under the protection of nitrogen for 5 h. The reaction system was quenched with water. Extraction was conducted with ethyl acetate (3*20 mL), drying was conducted with anhydrous sodium sulfate, and an organic phase was concentrated under reduced pressure to obtain a crude product. Then, the crude product was treated with column chromatography (PE:EA=1:1) to obtain a white solid compound 2-5 (50 mg, yield: 41.0%). LCMS: $(M+H)^+$ = 561.3.

**Step 3**

The compound 2-5 (50 mg, 0.09 mmol) was dissolved in DCM (2 mL), and TFA (1 mL) was dropped into the reaction system. A reaction solution was stirred at room temperature for 2 h. The reaction solution was concentrated under reduced pressure to obtain a crude product. Then, the crude product was separated by a high performance liquid chromatography column to obtain a white solid compound 2 (13.1 mg,yield: 33%). LCMS: $(M+H)^+$ = 461.2. $^1H$ NMR (400 MHz, DMSO) $\delta$ 8.08 (m, 3H), 7.89 (m, 1H), 7.76 (m, 1H), 7.46 (m, 2H), 7.39 (m, 2H), 7.28 (m, 1H), 5.08 (m, 2H), 3.96 (s, 3H), 3.56 (m, 2H), 3.35 (m, 1H), 2.09 (m, 1H), 1.89 (m, 1H), 1.68 (m, 2H). $^{19}F$ NMR (376 MHz, DMSO) $\delta$ -74.34 (s, 4.2F), -107.92 (s, 1F), -133.21 (s, 1 F).

**Embodiment 3**

Synthesis of 4-(7-(4-aminopiperidin-1-yl)-3-(3-fluoro-4-methoxyphenyl)imidazo [1,5-a]pyrazin-2-yl)-2-fluorobenzonitrile (compound 3)

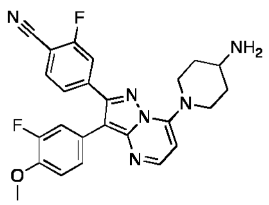

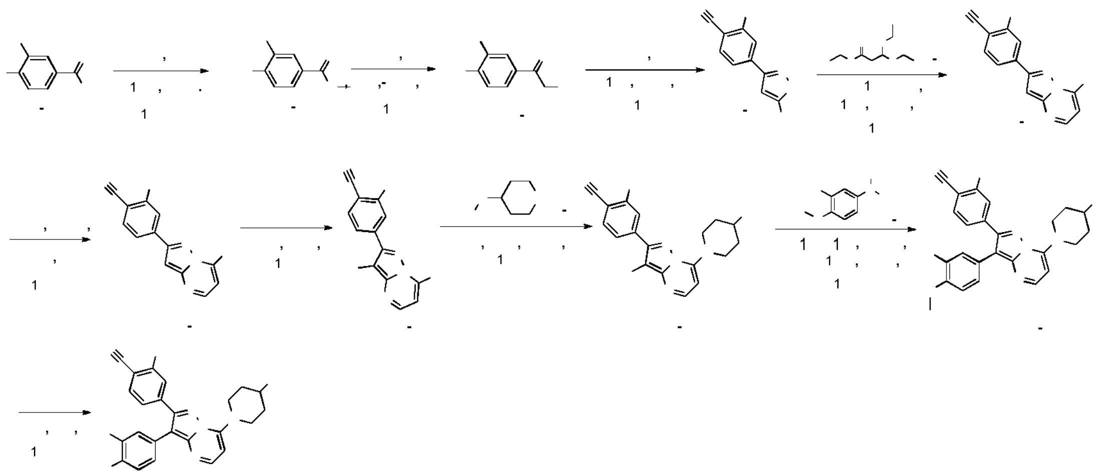

**Step 1**

A compound 3-1 (11 g, 85 mmol), dimethyl sulfate (16.12 g, 128 mmol) and potassium carbonate (21.18 g, 153 mmol) were dissolved in acetone (100 mL). The reaction system was heated to 60°C and reacted under the protection of nitrogen for 4 h. Water was added to the reaction system, extraction was conducted with ethyl acetate (3*100 mL), drying was conducted with anhydrous sodium sulfate, and an organic phase was concentrated under reduced pressure to obtain a crude product. Then, the crude product was treated with column chromatography (PE:EA=5:1) to obtain a

white solid compound 3-2 (9.3 g, yield: 61%). $^1H$ NMR (400 MHz, DMSO) δ 8.17 - 8.05 (m, 1H), 7.96 (dd, J = 16.1, 9.0 Hz, 2H), 3.91 (s, 3H).

**Step 2**

The compound 3-2 (5.4 g, 30.2 mmol) was dissolved in tetrahydrofuran (60 mL), and LiHMDS (45.3 mL, 45.3 mmol) was dropped into the reaction system at -78°C and reacted under the protection of nitrogen for 0.5 h. Acetonitrile (1.48 g, 36.2 mmol) was dropped into the reaction system at -78°C, and the reaction system was heated to 0°C and reacted under the protection of nitrogen for 1 h. A reaction solution was concentrated under reduced pressure until a large amount of solids were precipitated. Then, suction filtration was conducted, and a filter cake was washed with dichloromethane (50 mL*3) and dried to obtain a yellow solid compound 3-3 (3.7 g, yield: 65%). LCMS $(M+H)^+$ = 189.0.

**Step 3**

The compound 3-3 (3.5 g, 18.6 mmol) and hydrazine hydrate (2.8 g, 55.8 mmol) were dissolved in ethanol (50 mL), acetic acid (3 mL) was added, and a reaction solution was heated to 75°C and reacted under the protection of nitrogen for 8 h. The reaction system was quenched with water. Extraction was conducted with ethyl acetate (3*80 mL), drying was conducted with anhydrous sodium sulfate, and an organic phase was concentrated under reduced pressure to obtain a crude product. Then, the crude product was treated with column chromatography (PE:EA=1:1) to obtain a white solid compound 3-4 (1.5 g, yield: 31%). LCMS $(M+H)^+$ = 260.1.

**Step 4**

The compound 3-4 (1.5 g, 5.79 mmol) was dissolved in ethanol (20 mL), a compound 3-4A (1.65 g, 8.68 mmol) was added to the reaction system, and 4M HCl/1,4-dioxane (3 mL) was dropped at room temperature. A reaction solution was heated to 110°C and reacted under the protection of nitrogen for 1 h. The reaction system was quenched with water. Extraction was conducted with ethyl acetate (3*50 mL), drying was conducted with anhydrous sodium sulfate, and an organic phase was concentrated under reduced pressure to obtain a crude product. Then, the crude product was treated with column chromatography (PE:EA=2:1) to obtain a colorless oily compound 3-5 (400 mg, yield: 27%). LCMS $(M+H)^+$ = 255.2.

**Step 5**

The compound 3-5 (400 mg, 1.57 mmol) was dissolved in $POCl_3$ (10 mL), DIPEA (608 mg, 4.71 mmol) was added to the reaction system, and a reaction solution was heated to 100°C and reacted under the protection of nitrogen for 2 h. The reaction solution was concentrated under reduced pressure to obtain a crude product. Then, the crude product was treated with column chromatography (PE:EA=3:1) to obtain a white solid compound 3-6 (350 mg, yield: 82%). LCMS: $(M+H)^+$ = 272.0.

**Step 6**

The compound 3-6 (350 mg, 1.29 mmol) was dissolved in acetonitrile (10 mL), NBS (344 mg, 1.94 mmol) was added to the reaction system, and a reaction solution was heated to 50°C and reacted under the protection of nitrogen for 2 h. The reaction system was quenched with water. Extraction was conducted with ethyl acetate (3*30 mL), drying was conducted with anhydrous sodium sulfate, and an organic phase was concentrated under reduced pressure to obtain a crude product. Then, the crude product was treated with column chromatography (PE:EA=3:1) to obtain a white solid compound 3-7 (200 mg, yield: 44.0%). LCMS: $(M+H)^+$ = 351.2.

**Step 7**

The compound 3-7 (100 mg, 0.28 mmol) was dissolved in NMP (8.0 mL), DIPEA (162.6 mg, 0.56 mmol) and a compound 3-7A (84 mg, 0.42 mmol) were added to the reaction system, and a reaction solution was heated to 120°C and reacted under the protection of nitrogen for 4 h. The reaction system was quenched with water. Extraction was conducted with ethyl acetate (3*30 mL), drying was conducted with anhydrous sodium sulfate, and an organic phase was concentrated under reduced pressure to obtain a crude product. Then, the crude product was treated with column chromatography (PE:EA=3:1) to obtain a white solid compound 3-8 (68 mg, yield: 47%). LCMS: $(M+H)^+$ = 515.3.

**Step 8**

The compound 3-8 (68 mg, 0.13 mmol), a compound 3-8A (33.74 mg, 0.20 mmol), Pd(dppf)$Cl_2$ (7.3 mg, 0.01 mmol) and $K_2CO_3$ (35.9 mg, 0.26 mmol) were dissolved in 1,4-dioxane/$H_2O$ (3/0.5 mL), the reaction system was heated to 100°C, and a reaction solution was subjected to a reaction under the protection of nitrogen for 8 h. The reaction system was quenched with water. Extraction was conducted with ethyl acetate (3*30 mL), drying was conducted with anhydrous sodium sulfate, and an organic phase was concentrated under reduced pressure to obtain a crude product. Then, the crude product was treated with column chromatography (PE:EA=1:1) to obtain a white solid compound 3-9 (52 mg, yield: 41.0%). LCMS: $(M+H)^+$ = 561.3.

**Step 9**

The compound 3-9 (52 mg, 0.09 mmol) was dissolved in DCM (2 mL), and TFA (1 mL) was dropped into the reaction system. A reaction was carried out at room temperature under the protection of nitrogen for 2 h. A reaction solution was concentrated under reduced pressure to obtain a crude product. Then, the crude product was separated by a high performance liquid chromatography column to obtain a white solid compound 3 (21 mg, yield: 51%). LCMS: $(M+H)^+$ = 461.0. $^1H$ NMR (400 MHz, DMSO) $\delta$ 8.37 (d, J = 5.1 Hz, 1H), 8.00 (d, J = 7.2 Hz, 3H), 7.73 (d, J = 10.5 Hz, 1H), 7.55 (d, J = 9.5 Hz, 1H), 7.34 (d, J = 12.7 Hz, 1H), 7.21 (t, J = 8.9 Hz, 1H), 7.13 (d, J = 9.8 Hz, 1H), 6.61 (m, 1H), 4.56 (d, J = 12.7 Hz, 2H), 3.88 (s, 3H), 3.40 (m, 1H), 3.22 (t, J = 12.1 Hz, 2H), 2.08 (d, J = 12.7 Hz, 2H), 1.74 (d, J = 15.3 Hz, 2H). $^{19}F$ NMR (376 MHz, DMSO) $\delta$ -74.55 (s, 3.35F), -109.92 (s, 1F), -135.21 (s, 1 F).

**Embodiment 4**

Synthesis of (R)4-(7-(3-aminopiperidin-1-yl)-3-(3-fluoro-4-methoxyphenyl)imidazo [1,5-a]pyrazin-2-yl)-2-fluorobenzonitrile (compound 4)

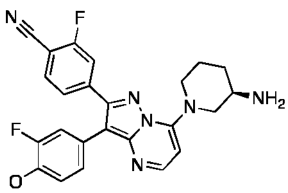

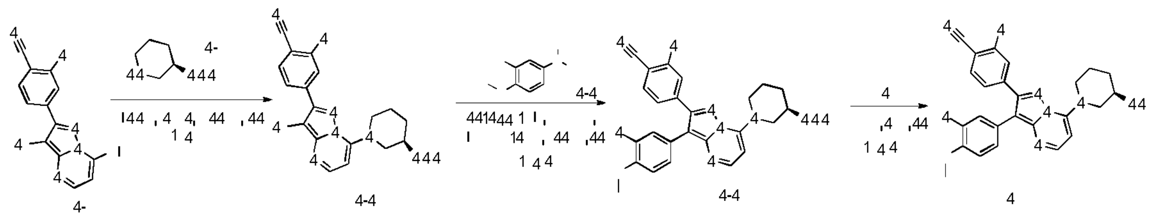

**Step 1**

The compound 4-1 (100 mg, 0.28 mmol) was dissolved in NMP (8.0 mL), DIPEA (162.6 mg, 0.56 mmol) and a compound 4-1A (84 mg, 0.42 mmol) were added to the reaction system, and a reaction solution was heated to 120°C and reacted under the protection of nitrogen for 4 h. The reaction system was quenched with water. Extraction was conducted with ethyl acetate (3*30 mL), drying was conducted with anhydrous sodium sulfate, and an organic phase was concentrated under reduced pressure to obtain a crude product. Then, the crude product was treated with column chromatography (PE:EA=3:1) to obtain a white solid compound 4-2 (90 mg, yield: 63%). LCMS: $(M+H)^+$ = 515.2.

**Step 2**

The compound 4-2 (90 mg, 0.17 mmol), a compound 4-2A (43.35 mg, 0.25 mmol), Pd(dppf)$Cl_2$ (14.6 mg, 0.02 mmol) and $K_2CO_3$ (46.9 mg, 0.34 mmol) were dissolved in 1,4-dioxane/$H_2O$ (3/0.5 mL), the reaction system was heated to 100°C, and a reaction solution was subjected to a reaction under the protection of nitrogen for 8 h. The reaction system

was quenched with water. Extraction was conducted with ethyl acetate (3*30 mL), drying was conducted with anhydrous sodium sulfate, and an organic phase was concentrated under reduced pressure to obtain a crude product. Then, the crude product was treated with column chromatography (PE:EA=1:1) to obtain a white solid compound 4-3 (65 mg, yield: 68.3%). LCMS: $(M+H)^+$ = 561.0.

**Step 3**

The compound 4-3 (65 mg, 0.12 mmol) was dissolved in DCM (2 mL), and TFA (1 mL) was dropped into the reaction system. A reaction was carried out at room temperature under the protection of nitrogen for 2 h. The reaction solution was concentrated under reduced pressure to obtain a crude product. Then, the crude product was separated by a high performance liquid chromatography column to obtain a white solid compound 4 (41 mg,yield: 74%). LCMS: $(M+H)^+$ = 461.0. $^1H$ NMR (400 MHz, DMSO) $\delta$ 8.40 (d, J = 5.1 Hz, 1H), 8.11 (M, 3H), 8.02 (m, 1H), 7.81 (dd, J = 10.5, 1.2 Hz, 1H), 7.55 (dd, J = 8.1, 1.4 Hz, 1H), 7.30 (dd, J = 20.4, 18.4 Hz, 1H), 7.20 (d, J = 8.9 Hz, 1H), 7.12 (m, 1H), 6.64 (d, J = 5.2 Hz, 1H), 4.47 (d, J = 9.2 Hz, 1H), 4.02 (d, J = 13.2 Hz, 1H), 3.88 (s, 3H), 3.46 (m, 2H), 3.36 (t, J = 9.9 Hz, 1H), 2.00 (m, 2H), 1.73 (m, 2H). $^{19}F$ NMR (376 MHz, DMSO) $\delta$ -74.45 (s, 3.29F), -106.42 (s, 1F), -135.21 (s, 1 F).

**Embodiment 5**

Synthesis of (R)-4-(8-(3-aminopiperidin-1-yl)-3-(1-methyll-1H-indazol-5-yl)imidazo [1,2-a]pyrazin-2-yl)-2-fluorobenzonitrile (compound 5)

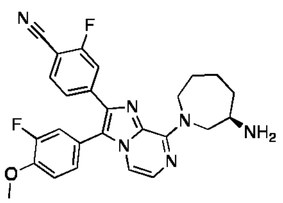

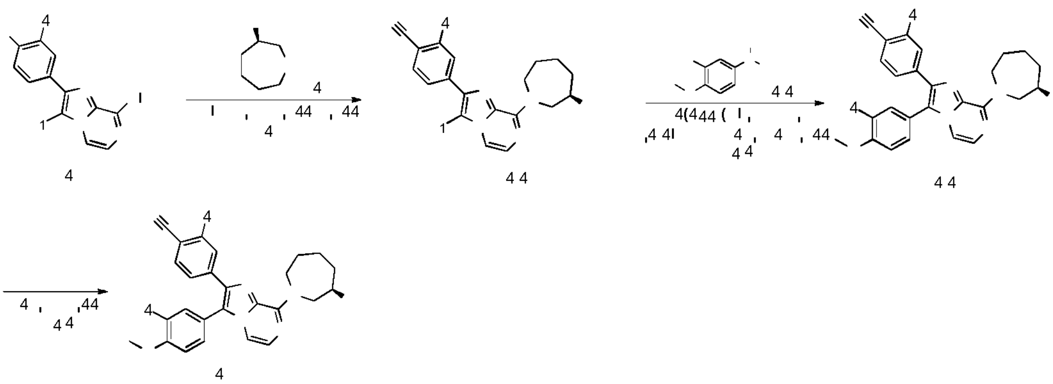

**Step 1**

A compound 5-1 (70 mg, 0.2 mmol), a compound 5-1A (42 mg, 0.2 mmol) and DIPEA (53 mg, 0.4 mmol) were dissolved in DMF (3 mL), the reaction system was heated to 120°C, and a reaction solution was subjected to a reaction under the protection of nitrogen for 12 h. The reaction solution was concentrated to obtain a crude product. Then, the crude product was separated by a high performance liquid chromatography column to obtain a white solid compound 5-2 (60 mg, yield: 55%). LCMS: $(M+H)^+$ = 529.1.

**Step 2**

The compound 5-2 (60 mg, 0.11 mmol), a compound 5-2A (30 mg, 0.16 mmol), $Pd(dppf)Cl_2$ (9 mg, 0.01 mmol) and $K_2CO_3$ (30 mg, 0.22 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was

concentrated to obtain a crude product. Then, the crude product was separated by a high performance liquid chromatography column to obtain a white solid compound 5-3 (15 mg, yield: 24%). LCMS: $(M+H)^+$ = 575.1.

**Step 3**

The compound 5-3 (15 mg, 0.03 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 5 (7.4 mg, yield: 60%). LCMS: $(M+H)^+$ = 465.3. $^1$H NMR (400 MHz, DMSO) δ 8.03 (s, 2H), 7.92 - 7.85 (m, 1H), 7.70 (d, J = 10.6 Hz, 1H), 7.49 (dd, J = 8.8, 1.8 Hz, 1H), 7.47 - 7.45 (m, 1H), 7.43 (t, J = 8.8 Hz, 1H), 7.38 (d, J = 4.6 Hz, 1H), 7.33 (d, J = 4.6 Hz, 1H), 7.30 (d, J = 8.4 Hz, 1H), 4.41 (s, 2H), 3.96 (s, 3H), 3.89 (dd, J = 14.4, 6.6 Hz, 2H), 3.69 (s, 1H), 1.90 (d, J = 8.8 Hz, 2H), 1.78 (d, J = 35.2 Hz, 2H), 1.51 (dd, J = 50.4, 11.6 Hz, 2H). $^{19}$F NMR (376 MHz, DMSO) δ -74.02 (s, 3.78F), -107.95 (s, 1F), -108.00 (s, 0.92F).

**Embodiment 6**

Synthesis of 2-fluoro-4-(3-(3-fluoro-4-methoxyphenyl)-8-((pyrrolidin-2-ylmethyl) amino)imidazo[1,2-a]pyrazin-2-yl)benzonitrile (compound 6)

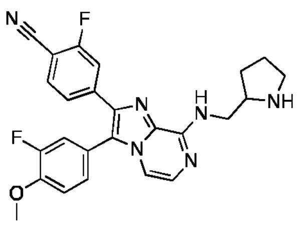

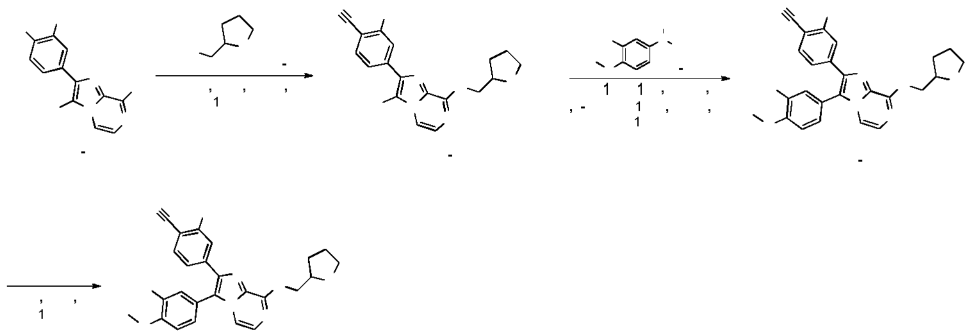

**Step 1**

A compound 6-1 (150 mg, 0.43 mmol), a compound 6-1A (85 mg, 0.43 mmol) and DIPEA (111 mg, 0.86 mmol) were dissolved in DMF (3 mL), the reaction system was heated to 100°C, and a reaction solution was subjected to a reaction under the protection of nitrogen for 12 h. The reaction solution was concentrated to obtain a crude product. Then, the crude product was separated by a high performance liquid chromatography column to obtain a white solid compound 6-2 (80 mg, yield: 36%). LCMS: $(M+H)^+$ = 515.1.

**Step 2**

The compound 6-2 (80 mg, 0.16 mmol), a compound 6-2A (92 mg, 0.24 mmol), $Pd(dppf)Cl_2$ (12 mg, 0.02 mmol) and $K_2CO_3$ (44 mg, 0.32 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 6-3 (30 mg, yield: 33%). LCMS: $(M+H)^+$ = 561.1.

**Step 3**

The compound 6-3 (30 mg, 0.05 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 6 (7 mg, yield: 30%). LCMS: $(M+H)^+$ = 461.3. $^1H$ NMR (400 MHz, DMSO) $\delta$ 9.03 (s, 1H), 8.69 (s, 1H), 8.09 (t, J = 5.8 Hz, 1H), 7.95 - 7.87 (m, 1H), 7.69 (d, J = 11.0 Hz, 1H), 7.51 - 7.48 (m, 1H), 7.47 (dd, J = 5.8, 1.8 Hz, 1H), 7.42 (t, J = 8.8 Hz, 1H), 7.37 - 7.31 (m, 2H), 7.27 (d, J = 14.0 Hz, 1H), 7.06 (d, J = 50. 8 Hz, 1H), 3.96 (s, 3H), 3.86 (d, J = 6.2 Hz, 1H), 3.81 - 3.76 (m, 2H), 3.28 - 3.14 (m, 2H), 2.08 (dd, J = 12.2, 4.8 Hz, 1H), 2.00 - 1.84 (m, 2H), 1.77 (dd, J = 12.4, 8.0 Hz, 1H). $^{19}F$ NMR (376 MHz, DMSO) $\delta$ -74.11 (s, 6F), -108.23 (s, 1F), -133.32 (s, 0.92F).

**Embodiment 7**

Synthesis of 2-fluoro-4-(3-(3-fluoro-4-methoxyphenyl)-8-(piperidin-3-ylamino) imidazo[1,2-a]pyrazin-2-yl)benzonitrile (compound 7)

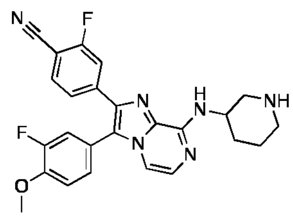

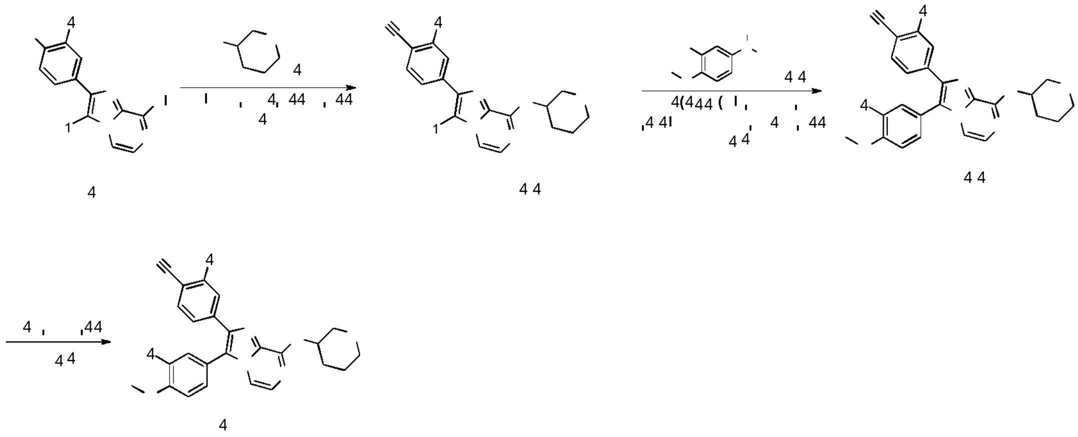

**Step 1**

A compound 7-1 (150 mg, 0.43 mmol), a compound 7-1A (85 mg, 0.43 mmol) and DIPEA (111 mg, 0.86 mmol) were dissolved in DMF (3 mL), the reaction system was heated to 120°C, and a reaction solution was subjected to a reaction under the protection of nitrogen for 12 h. The reaction solution was concentrated to obtain a crude product. Then, the crude product was separated by a high performance liquid chromatography column to obtain a white solid compound 7-2 (40 mg, yield: 36%). LCMS: $(M+H)^+$ = 515.1.

**Step 2**

The compound 7-2 (40 mg, 0.08 mmol), a compound 7-2A (20 mg, 0.12 mmol), $Pd(dppf)Cl_2$ (15 mg, 0.02 mmol) and $K_2CO_3$ (22 mg, 0.32 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 7-3 (20 mg, yield: 45%). LCMS: $(M+H)^+$ = 561.1.

**Step 3**

The compound 7-3 (20 mg, 0.04 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 7 (10 mg, yield: 55%). LCMS: $(M+H)^+$ = 461.3. $^1H$ NMR (400 MHz, DMSO) δ 8.75 (d, J = 28.6 Hz, 2H), 7.94 - 7.86 (m, 1H), 7.82 (d, J = 8.2 Hz, 1H), 7.72 (d, J = 11.0 Hz, 1H), 7.49 (d, J = 1.2 Hz, 1H), 7.47 (dd, J = 4.2, 1.8 Hz, 1H), 7.41 (t, J = 8.8 Hz, 1H), 7.32 (s, 1H), 7.30 (d, J = 10.4 Hz, 1H), 4.50 (s, 1H), 3.96 (s, 3H), 3.34 (dd, J = 66.2, 11.2 Hz, 2H), 2.93 (dd, J = 57.4, 10.2 Hz, 2H), 1.98 (d, J = 24.2 Hz, 2H), 1.77 (t, J = 10.0 Hz, 2H). $^{19}F$ NMR (376 MHz, DMSO) δ -74.25 (s, 4.88F), -108.30 (s, 1F), -133.35 (s, 0.94F).

**Embodiment 8**

Synthesis of (R)-4-(8-(3-aminopiperidin-1-yl)-3-(1-methyll-1H-indazol-5-yl)imidazo [1,2-a]pyrazin-2-yl)-2-fluorobenzonitrile (compound 8)

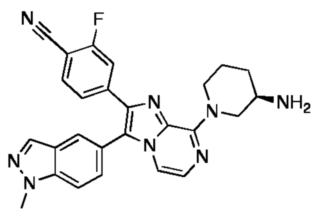

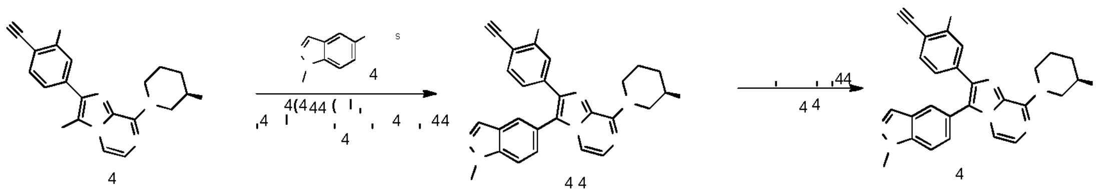

Step 1

A compound 8-1 (60 mg, 0.1 mmol), a compound 8-1A (26.4 mg, 0.15 mmol), $Pd(dppf)Cl_2$ (7 mg, 0.01 mmol) and $K_2CO_3$ (27 mg, 0.2 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 8-2 (20 mg, yield: 23%). LCMS: $(M+H)^+$ = 567.1.

**Step 3**

The compound 8-2 (20 mg, 0.02 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 8 (8 mg, yield: 80%). LCMS: $(M+H)^+$ = 467.3. $^1H$ NMR (400 MHz, DMSO) δ 8.20 (d, J = 0.8 Hz, 1H), 8.09 (d, J = 4.2 Hz, 2H), 7.98 (d, J = 1.4 Hz, 1H), 7.93 (d, J = 8.8 Hz, 1H), 7.86 - 7.79 (m, 1H), 7.74 (dd, J = 11.2, 1.2 Hz, 1H), 7.44 (ddd, J = 8.2, 5.4, 1.6 Hz, 2H), 7.36 (dd, J = 13.6, 4.6 Hz, 2H), 5.08 (dd, J = 38.8, 11.6 Hz, 2H), 4.16 (s, 3H), 3.64 - 3.55 (m, 2H), 3.37 (s, 1H), 2.09 (s, 1H), 1.91 (s, 1H), 1.69 (d, J = 8.6 Hz, 2H). $^{19}F$ NMR (376 MHz, DMSO) δ -74.13 (s, 3.85F), -108.00 (s, 1F).

**Embodiment 9**

Synthesis of (R)-4-(8-(3-aminopiperidin-1-yl)-3-(4-methyll-3,4-dihydro-2H-benzo [b][1,4]oxazin-7-yl)imidazo[1,2-a] pyrazin-2-yl)-2-fluorobenzonitrile (compound 9)

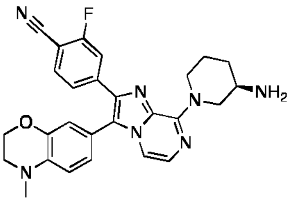

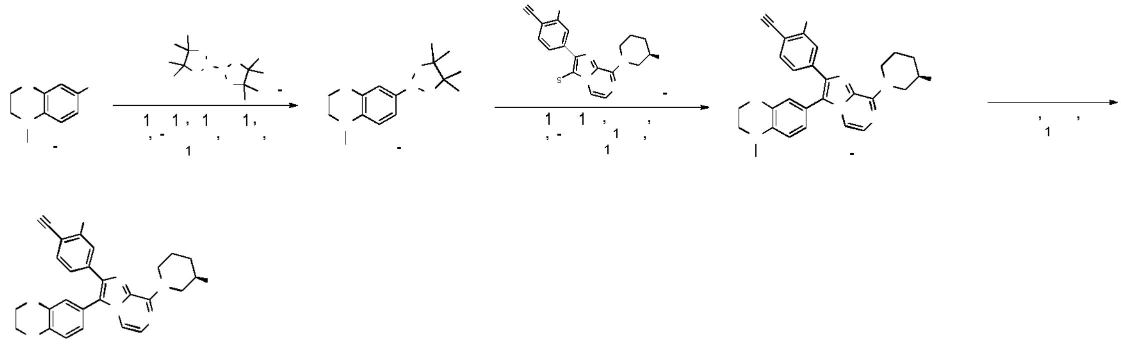

**Step 1**

A compound 9-1 (330 mg, 1.45 mmol), a compound 9-1A (188 mg, 2.2 mmol), $Pd_2(dba)_3$ (137 mg, 0.15 mmol), tricyclohexylphosphine (81 mg, 0.29 mmol) and potassium acetate (284 mg, 2.9 mmol) were dissolved in 1,4-dioxane (5 mL). The reaction system was heated to 110°C and reacted under the protection of nitrogen for 12 h. The reaction system was quenched with water. Extraction was conducted with ethyl acetate (3* 50mL), drying was conducted with anhydrous sodium sulfate, and spin-drying was conducted. Then, column chromatography was conducted (PE:EA=1:1) to obtain a colorless oily compound 9-2 (120 mg, yield: 30%). LCMS $(M+H)^+$ = 276.1.

**Step 2**

The compound 9-2 (120 mg, 0.43 mmol), a compound 9-2A (147 mg, 0.29 mmol), $Pd(dppf)Cl_2$ (20 mg, 0.03 mmol) and $K_2CO_3$ (80 mg, 0.58 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 9-3 (20 mg, yield: 8%). LCMS: $(M+H)^+$ = 584.1.

**Step 3**

The compound 9-3 (20 mg, 0.034 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 9 (6.7 mg, yield: 41%). LCMS: $(M+H)^+$ = 484.1. $^1H$ NMR (400 MHz, DMSO) δ 8.06 (s, 2H), 7.93 - 7.85 (m, 1H), 7.75 (d, J = 11.2 Hz, 1H), 7.58 (dd, J = 8.2, 1.4 Hz, 1H), 7.36 (q, J = 4.6 Hz, 2H), 6.91 - 6.85 (m, 2H), 6.78 (d, J = 1.8 Hz, 1H), 5.03 (dd, J = 37.2, 11.2 Hz, 2H), 4.32 - 4.25 (m, 2H), 3.58 (d, J = 12.0 Hz, 2H), 3.41 - 3.36 (m, 2H), 3.36 - 3.28 (m, 1H), 2.95 (s, 3H), 2.07 (s, 1H), 1.89 (s, 1H), 1.74 - 1.60 (m, 2H).

$^{19}F$ NMR (376 MHz, DMSO) δ -73.88 (s, 4F), -108.20 (s, 1F).

**Embodiment 10**

Synthesis of (R)-4-(8-(3-aminopiperidin-1-yl)-3-(1-(2,2,2-trifluoroethyl)-1H-pyrazol-4-yl)imidazo[1,2-a]pyrazin-2-yl)-2-fluorobenzonitrile (compound 10)

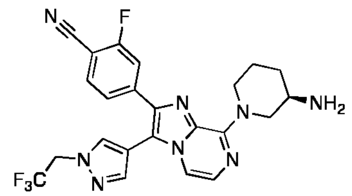

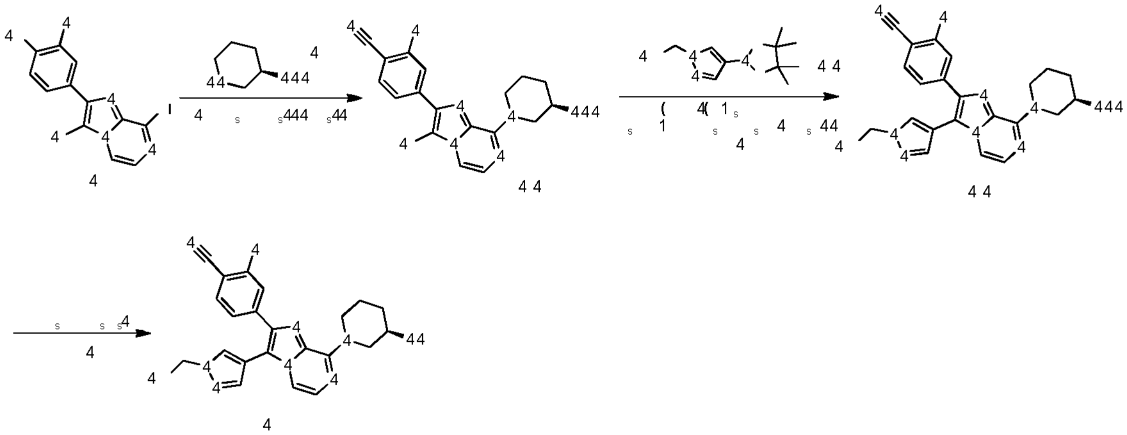

**Step 1**

A compound 10-1 (330 mg, 0.94 mmol), a compound 10-1A (188 mg, 0.94 mmol) and DIPEA (242.5 mg, 1.88 mmol) were dissolved in NMP (10 mL). The reaction system was heated to 120°C and reacted under the protection of nitrogen for 12 h. The reaction system was quenched with water. Extraction was conducted with ethyl acetate (3*50 mL), drying was conducted with anhydrous sodium sulfate, and spin-drying was conducted. Then, column chromatography was conducted (PE:EA=1:1) to obtain a brown solid compound 10-2 (180 mg, yield: 37%). LCMS $(M+H)^+$ = 514.1.

**Step 2**

The compound 10-2 (60 mg, 0.1 mmol), a compound 10-2A (26.4 mg, 0.15 mmol), $Pd(dppf)Cl_2$ (7 mg, 0.01 mmol) and $K_2CO_3$ (27 mg, 0.2 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 10-3 (13 mg, yield: 19%). LCMS: $(M+H)^+$ = 585.1.

**Step 3**

The compound 10-3 (13 mg, 0.02 mmol) was dissolved in DCM (2 mL), and TFA (1 mL) was dropped into the reaction system. A reaction solution was subjected to a reaction at room temperature for 2 h. The reaction solution was concentrated to obtain a crude product. Then, the crude product was separated by a high performance liquid chromatography column to obtain a white solid compound 10 (3.2 mg, yield: 30%). LCMS: $(M+H)^+$ = 485.1. $^1H$ NMR (400 MHz, DMSO) δ 8.31 (s, 1H), 8.04 (s, 2H), 7.95 (d, J = 8.2 Hz, 1H), 7.92 (s, 1H), 7.76 - 7.72 (m, 1H), 7.69 (dd, J = 8.2, 1.6 Hz, 1H), 7.44 (d, J = 4.6 Hz, 1H), 7.41 (d, J = 4.6 Hz, 1H), 5.31 (d, J = 9.0 Hz, 2H), 5.05 (d, J = 13.8 Hz, 2H), 3.33 (s, 2H), 2.06 (s, 2H), 1.88 (s, 1H), 1.66 (d, J = 8.2 Hz, 2H). $^{19}F$ NMR (376 MHz, DMSO) δ -70.22 (s, 2.85F), -73.88 (s, 4.25F), -108.20 (s, 1F).

**Embodiment 11**

Synthesis of (R)-4-(8-(3-aminopiperidin-1-yl)-3-(1-(2-hydroxy-2-methylpropyl)-1H-indazol-5-yl)imidazo[1,2-a]pyrazin-2-yl)-2-fluorobenzonitrile (compound 11)

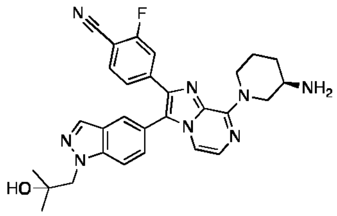

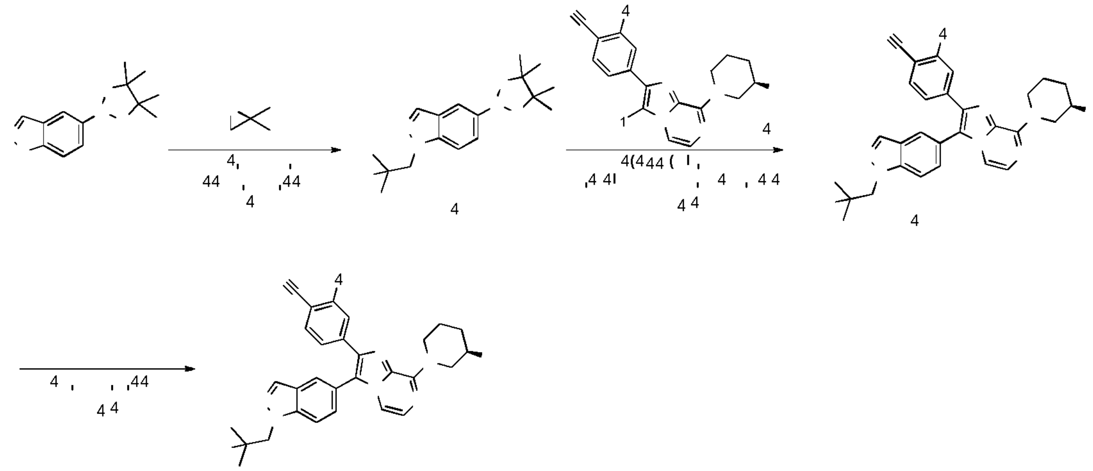

Step 1

A compound 11-1 (550 mg, 2.3 mmol), a compound 11-1A (811 mg, 11.2 mmol) and $K_2CO_3$ (580 mg, 4.5 mmol) were dissolved in DMF (8 mL), and the reaction system was heated to 100°C for a microwave reaction for 2 h. The reaction system was quenched with water. Extraction was conducted with ethyl acetate (3*20 mL), drying was conducted with anhydrous sodium sulfate, and spin-drying was conducted. Then, column chromatography was conducted (PE:EA=1:1) to obtain a white solid compound 11-2 (100 mg, yield: 14%). LCMS $(M+H)^+$ = 317.1.

**Step 2**

The compound 11-2 (100 mg, 0.32 mmol), a compound 11-2A (110 mg, 0.21 mmol), $Pd(dppf)Cl_2$ (10 mg, 0.02 mmol) and $K_2CO_3$ (58 mg, 0.42 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 11-3 (25 mg, yield: 19%). LCMS $(M+H)^+$ = 625.0.

**Step 3**

The compound 11-3 (25 mg, 0.05 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 11 (5.3 mg, yield: 20%). LCMS $(M+H)^+$ = 525.2. $^1$H NMR (400 MHz, DMSO) δ 8.21 (s, 1H), 8.07 (d, J = 4.2 Hz, 2H), 7.96 (d, J = 8.8 Hz, 2H), 7.87 - 7.79 (m, 1H), 7.73 (d, J = 11.2 Hz, 1H), 7.47 (dd, J = 8.2, 1.2 Hz, 1H), 7.40 (d, J = 10.2 Hz, 1H), 7.36 (dd, J = 9.2, 4.6 Hz, 2H), 5.17 - 4.97 (m, 2H), 4.41 (s, 2H), 3.62 (d, J = 8.4 Hz, 3H), 3.36 (s, 1H), 2.09 (s, 1H), 1.91 (s, 1H), 1.69 (d, J = 5.8 Hz, 2H), 1.20 (s, 6H). $^{19}$F NMR (376 MHz, DMSO) δ -74.15 (s, 4.27F), -108.11 (s, 1F).

**Embodiment 12**

Synthesis of (R)-4-(8-(3-aminopiperidin-1-yl)-3-(6-fluoro-1-(2-hydroxy-2-methylpropyl)-1H-benzo[d][1,2,3]triazol-5-yl)imidazo[1,2-a]pyrazin-2-yl)-2-fluorobenzonitrile (compound 12)

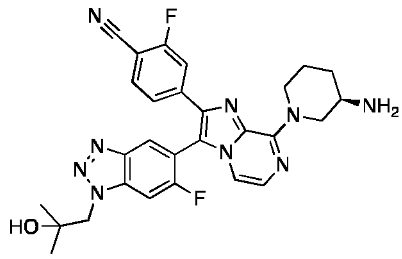

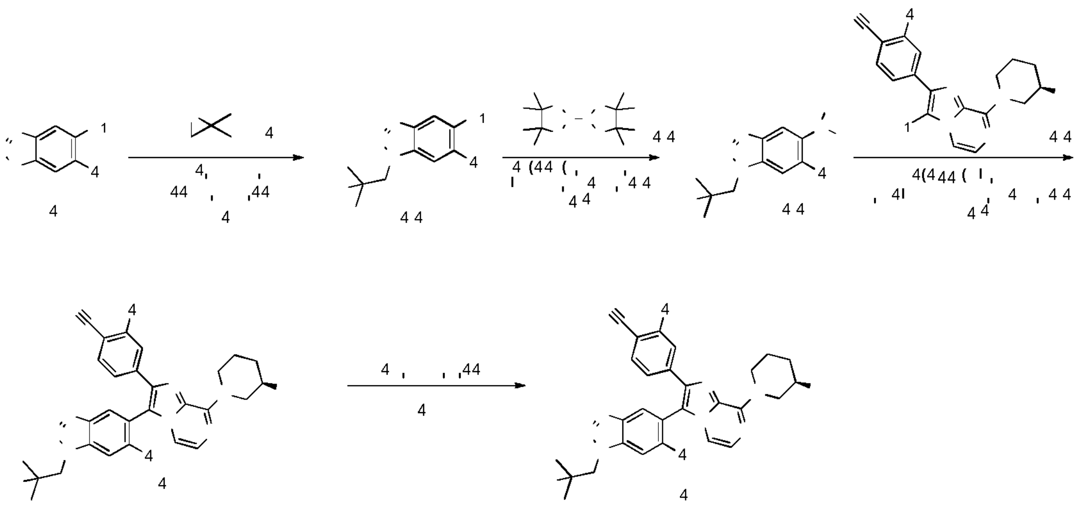

**Step 1**

A compound 12-1 (450 mg, 2.1 mmol), a compound 12-1A (756 mg, 10.5 mmol) and $K_2CO_3$ (580 mg, 4.2 mmol) were dissolved in DMF (8 mL), and the reaction system was heated to 100°C for a microwave reaction for 2 h. The reaction system was quenched with water. Extraction was conducted with ethyl acetate (3*20 mL), drying was conducted with anhydrous sodium sulfate, and spin-drying was conducted. Then, column chromatography was conducted (PE:EA=1:2) to obtain a white solid compound 12-2 (205 mg, yield: 33%). LCMS $(M+H)^+$ = 288.1.

**Step 2**

The compound 12-2 (120 mg, 0.42 mmol), a compound 12-2A (150 mg, 0.63 mmol), $Pd_2(dba)_3$ (38 mg, 0.04 mmol), tricyclohexylphosphine (24 mg, 0.08 mmol) and potassium acetate (82 mg, 0.84 mmol) were dissolved in 1,4-dioxane (3 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 12-3 (45 mg, yield: 42%). LCMS $(M+H)^+$ = 254.0.

**Step 3**

The compound 12-3 (45 mg, 0.18 mmol), a compound 12-3A (110 mg, 0.18 mmol), $Pd(dppf)Cl_2$ (10 mg, 0.02 mmol) and $K_2CO_3$ (50 mg, 0.36 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance

liquid chromatography column to obtain a white solid compound 12-4 (15 mg, yield: 13%). LCMS $(M+H)^+$ = 644.2.

**Step 4**

The compound 12-4 (15 mg, 0.02 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 12 (1.7 mg, yield: 16%. LCMS $(M+H)^+$ = 544.2. $^1$H NMR (400 MHz, DMSO) δ 8.44 (d, J = 5.2 Hz, 1H), 8.08 (d, J = 9.2 Hz, 4H), 7.86 (d, J = 8.0 Hz, 1H), 7.79 (d, J = 11.8 Hz, 1H), 7.50 - 7.35 (m, 3H), 5.08 (s, 1H), 4.94 (s, 1H), 4.69 (s, 2H), 3.37 (s, 3H), 2.10 (s, 1H), 1.88 (s, 1H), 1.71 (s, 2H), 1.26 (s, 3H), 1.20 (s, 3H). $^{19}$F NMR (376 MHz, DMSO) δ -73.79 (s, 5.93 F), -107.73 (s, 1F), -114.67 (s, 1F). (1.8 mg, yield: 16%). LCMS $(M+H)^+$ = 544.2. $^1$H NMR (400 MHz, DMSO) δ 8.44 (d, J = 5.2 Hz, 1H), 8.08 (d, J = 9.2 Hz, 4H), 7.86 (d, J = 8.0 Hz, 1H), 7.79 (d, J = 11.8 Hz, 1H), 7.50 - 7.35 (m, 3H), 5.08 (s, 1H), 4.94 (s, 1H), 4.69 (s, 2H), 3.37 (s, 3H), 2.10 (s, 1H), 1.88 (s, 1H), 1.71 (s, 2H), 1.26 (s, 3H), 1.20 (s, 3H). $^{19}$F NMR (376 MHz, DMSO) δ -73.79 (s, 6.58F), -107.68 (s, 0.95F), -107.73 (s, 0.2F), -114.35 (s, 0.22F), -119.26 (s, 1F).

**Embodiment 13**

Synthesis of 2-fluoro-4-(3-(3-fluoro-4-methoxyphenyl)-8-((pyrrolidin-3-ylmethyl) amino)imidazo[1,2-a]pyrazin-2-yl)benzonitrile (compound 13)

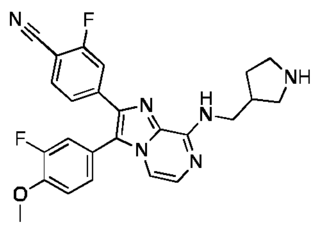

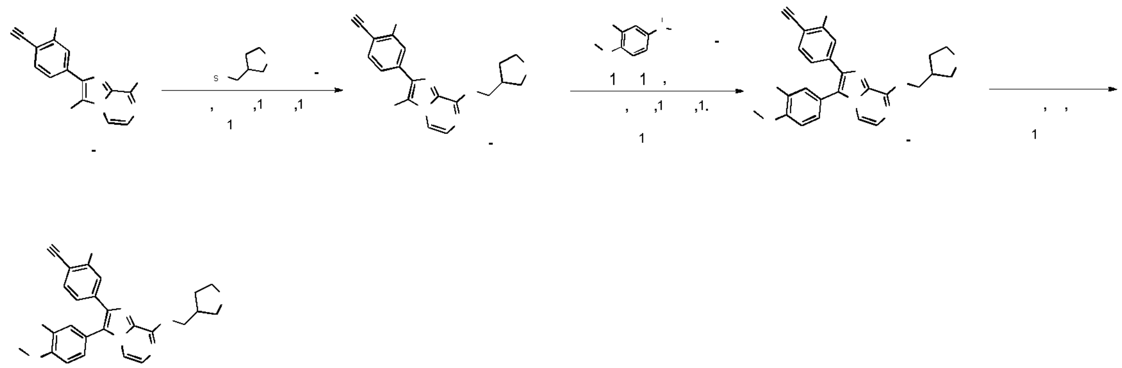

**Step 1**

A compound 13-1 (70 mg, 0.2 mmol) was dissolved in NMP (4 mL), a compound 13-2 (80 mg, 0.4 mmol) and DIPA (129 mg, 1 mmol) were added, and a reaction mixture was heated to 100°C and stirred for 1 h. Cooling was conducted to room temperature, dilution was conducted with ethyl acetate, and an organic phase was back-washed with a saturated salt solution for 3 to 4 times, dried with anhydrous sodium sulfate and concentrated. Then, a resulting crude residue was purified by column chromatography (PE:EA=2:1) to obtain a white solid compound 13-3 (70 mg, yield: 68%. LCMS $(M+H)^+$ = 515.0.

**Step 2**

The compound 13-3 (70 mg, 0.14 mmol), a compound 13-4 (47 mg, 0.3 mmol) and potassium carbonate (47 mg, 0.34 mmol) were dissolved in a mixed solution of dioxane (2 mL) and water (0.5 mL), and a catalyst Pd(dppf)$Cl_2$

(10 mg, 0.14 mmol) was added under the protection of nitrogen. A mixture was heated to 100°C and stirred for a reaction in an atmosphere of nitrogen for 1.5 h, and concentration was conducted to remove most of a solvent. Then, a resulting crude residue was purified by column chromatography (PE:EA=1:1) to obtain a white solid compound 13-5 (35 mg, yield: 46%. LCMS $(M+H)^+$ = 561.0.

**Step 3**

The compound 13-5 (35 mg, 0.06 mmol) was dissolved in dichloromethane (4 mL), and trifluoroacetic acid (1 mL) was dropped under an ice water bath. Stirring was conducted for a reaction at room temperature for 1 h, and concentration was conducted to remove most of a solvent. Then, a resulting crude product was treated with Prep-HPLC to obtain a white solid compound 13 (10 mg, yield: 35%). LCMS $(M+H)^+$ = 461.0. $^1$H NMR (400 MHz, DMSO) δ 8.73 - 8.66 (m, 1H), 8.42 - 8.10 (m, 1H), 7.94 -7.88 (m, 1H), 7.74 -7.67 (m, 1H), 7.51 - 7.45 (m, 2H), 7.44 - 7.38 (m, 1H), 7.34 - 7.26 (m, 3H), 3.95 (s, 3H), 3.60 - 3.57 (m, 2H), 3.33 - 3.25 (m, 2H), 3.20 - 3.11 (m, 1H), 3.05 - 2.96 (m, 1H), 2.81 - 2.71 (m, 1H), 2.10 - 1.99 (m, 1H), 1.80 - 1.69 (m, 1H). $^{19}$F NMR (376 MHz, DMSO) δ -74.11 (s, 3F), -108.28 (s, 1F), -133.35 (s, 1F).

**Embodiment 14**

Synthesis of cis-4-(8-(((1R,3S)-3-aminocyclopentyl)amino)-3-(3-fluoro-4-methoxyphenyl)imidazo[1,2-a]pyrazin-2-yl)-2-fluorobenzonitrile (compound 14)

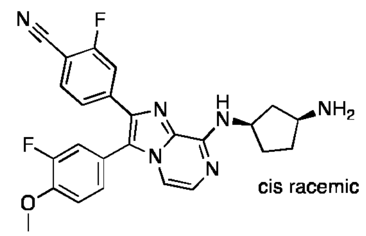


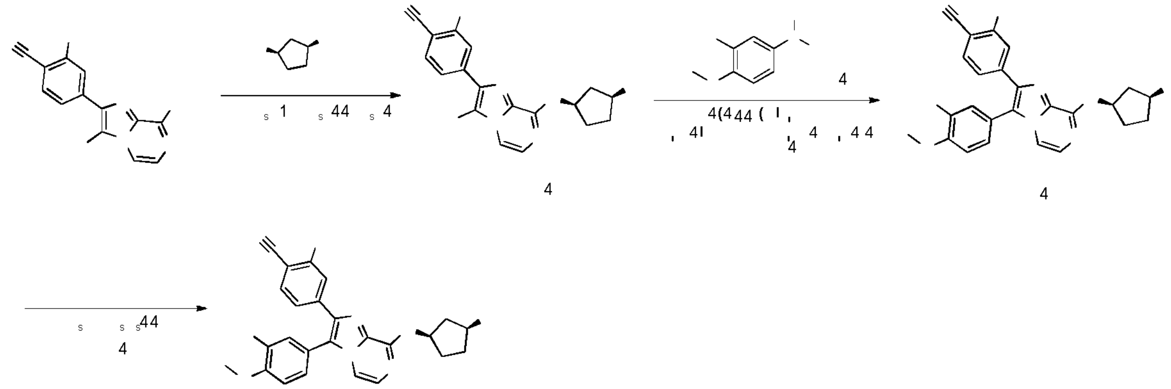

**Step 1**

A compound 14-1 (110 mg, 0.29 mmol), a compound 14-1A (57 mg, 0.29 mmol) and DIPEA (75 mg, 0.58 mmol) were dissolved in DMF (5 mL), and the reaction system was heated to 120°C and reacted under the protection of nitrogen for 4 h. The reaction system was quenched with water. Extraction was conducted with ethyl acetate (3*20 mL), drying was conducted with anhydrous sodium sulfate, and spin-drying was conducted. Then, column chromatography was conducted (PE:EA=1:1) to obtain a brownish yellow solid compound 14-2 (40 mg, yield: 35%). LCMS $(M+H)^+$ = 515.1.

**Step 2**

The compound 14-2 (40 mg, 0.08 mmol), a compound 14-2A (20 mg, 0.12 mmol), $Pd(dppf)Cl_2$ (10 mg, 0.02 mmol) and $K_2CO_3$ (22 mg, 0.16 mmol) were dissolved in 1,4-dioxane/$H_2O$ (3/0.5 mL), the reaction system was heated

to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 14-3 (23 mg, yield: 50%). LCMS $(M+H)^+$ = 561.2.

**Step 3**

The compound 14-3 (23 mg, 0.04 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 14 (7 mg, yield: 38%). LCMS $(M+H)^+$ = 461.3. $^1$H NMR (400 MHz, DMSO) $\delta$ 7.93 (d, J = 9.0 Hz, 3H), 7.89 (d, J = 7.2 Hz, 1H), 7.72 (dd, J = 11.2, 1.2 Hz, 1H), 7.48 (dt, J = 2.8, 2.0 Hz, 2H), 7.41 (t, J = 8.8 Hz, 1H), 7.32 (d, J = 4.8 Hz, 1H), 7.29 (d, J = 4.8 Hz, 2H), 4.54 (s, 1H), 3.95 (s, 3H), 3.59 (d, J = 5.8 Hz, 2H), 2.03 (s, 1H), 2.01 - 1.86 (m, 2H), 1.83 - 1.66 (m, 2H). $^{19}$F NMR (376 MHz, DMSO) $\delta$ -74.12 (s, 3.9F), -108.26 (s, 1F), -133.36 (s, 0. 92F).

**Embodiment 15**

Synthesis of trans-4-(8-(((1R,3R)-3-aminocyclopentyl)amino)-3-(3-fluoro-4-methoxyphenyl)imidazo[1,2-a]pyrazin-2-yl)-2-fluorobenzonitrile (compound 15)

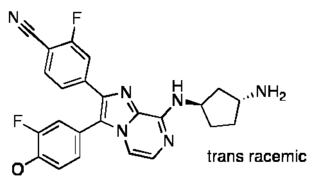


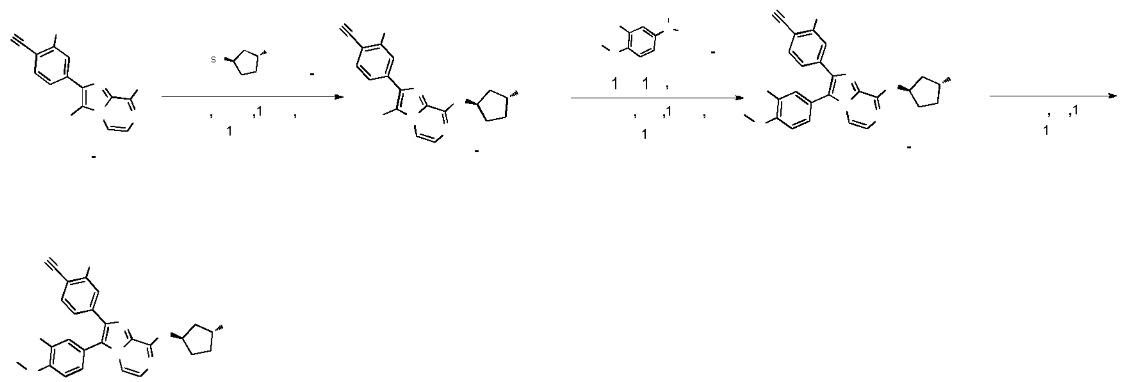

**Step 1**

A compound 15-1 (80 mg, 0.23 mmol) was dissolved in NMP (4 mL), a compound 15-2 (92 mg, 0.46 mmol) and DIPA (148 mg, 1.1 mmol) were added, and a reaction mixture was heated to 100°C and stirred for 1 h. Cooling was conducted to room temperature, dilution was conducted with ethyl acetate, and an organic phase was back-washed with a saturated salt solution for 3 to 4 times, dried with anhydrous sodium sulfate and concentrated. Then, a resulting crude residue was purified by column chromatography (PE:EA=2:1) to obtain a white solid compound 15-3 (95 mg, yield: 81%. LCMS $(M+H)^+$ = 515.0.

**Step 2**

The compound 15-3 (95 mg, 0.2 mmol), a compound 15-4 (47 mg, 0.3 mmol) and potassium carbonate (51 mg, 0.37 mmol) were dissolved in a mixed solution of dioxane (2 mL) and water (0.5 mL), and a catalyst Pd(dppf)$Cl_2$ (14 mg, 0.02 mmol) was added under the protection of nitrogen. A mixture was heated to 100°C and stirred for a reaction in an atmosphere of nitrogen for 2 h, and concentration was conducted to remove most of a solvent. Then, a resulting

crude residue was purified by column chromatography (PE:EA=1:1) to obtain a white solid compound 15-5 (60 mg, yield: 58%). LCMS $(M+H)^+$ = 561.0.

**Step 3**

The compound 15-5 (60 mg, 0.1 mmol) was dissolved in dichloromethane (4 mL), and trifluoroacetic acid (1 mL) was dropped under an ice water bath. Stirring was conducted for a reaction at room temperature for 1 h, and concentration was conducted to remove most of a solvent. Then, a resulting crude product was treated with prep-HPLC to obtain a white solid compound 15 (22 mg, yield: 45%). LCMS $(M+H)^+$ = 461.0. $^1H$ NMR (500 MHz, DMSO) δ 7.92 - 7.85 (m, 4H), 7.75 - 7.70 (m, 1H), 7.48 (d, J = 9.8 Hz, 2H), 7.44 - 7.39 (m, 1H), 7.30 (s, 3H), 4.75 - 4.70 (m, 1H), 3.96 (s, 3H), 3.76 - 3.72 (m, 1H), 2.24 - 2.13 (m, 3H), 2.09 - 2.03 (m, 1H), 1.82 - 1.74 (m, 1H), 1.63 - 1.57 (m, 1H). $^{19}F$ NMR (376 MHz, DMSO) δ -74.186 (s, 4F), -108.317 (s, 1F), -133.382 (s, 1F).

**Embodiment 16**

Synthesis of 2-fluoro-4-(3-(3-fluoro-4-methoxyphenyl)-8-(pyrrolidin-3-ylamino) imidazo[1,2-a]pyrazin-2-yl)benzonitrile (compound 16)

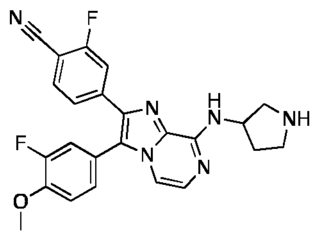

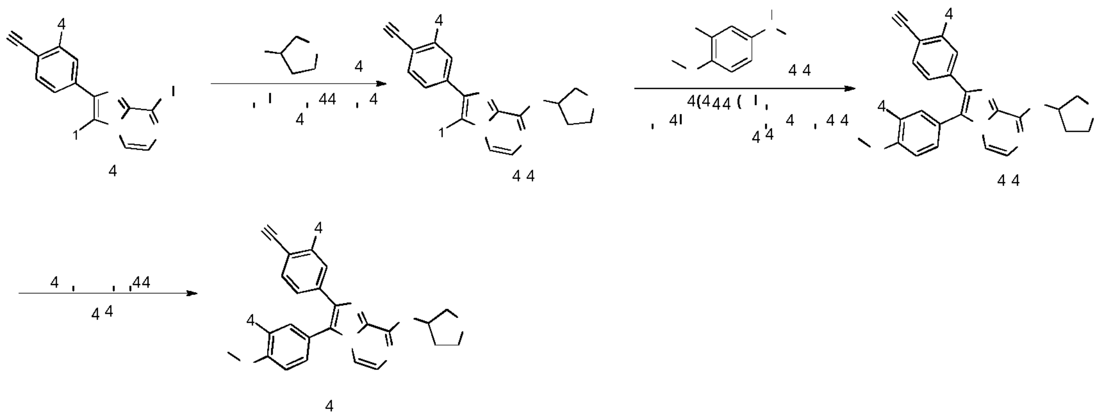

**Step 1**

A compound 16-1 (200 mg, 0.57 mmol), a compound 16-1A (106 mg, 0.57 mmol) and DIPEA (147 mg, 1.14 mmol) were dissolved in DMF (5 mL), and the reaction system was heated to 120°C and reacted under the protection of nitrogen for 4 h. The reaction system was quenched with water. Extraction was conducted with ethyl acetate (3*20 mL), drying was conducted with anhydrous sodium sulfate, and spin-drying was conducted. Then, column chromatography was conducted (PE:EA=1:1) to obtain a brownish yellow solid compound 16-2 (100 mg, yield: 35%). LCMS $(M+H)^+$ = 501.1.

**Step 2**

The compound 16-2 (100 mg, 0.2 mmol), a compound 16-2A (51 mg, 0.3 mmol), $Pd(dppf)Cl_2$ (10 mg, 0.02 mmol) and $K_2CO_3$ (55 mg, 0.4 mmol) were dissolved in 1,4-dioxane/$H_2O$ (3/0.5 mL), the reaction system was heated to 110°C, and a reaction solution was subjected to a reaction under the protection of nitrogen for 12 h. The reaction

solution was concentrated to obtain a crude product. Then, the crude product was separated by a high performance liquid chromatography column to obtain a white solid compound 16-3 (38 mg, yield: 35%). LCMS $(M+H)^+$ = 547.2.

**Step 3**

The compound 16-3 (38 mg, 0.07 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 16 (7 mg, yield: 22%). LCMS $(M+H)^+$ = 447.1. $^1H$ NMR (400 MHz, DMSO) $\delta$ 8.84 (s, 1H), 8.00 (d, J = 6.8 Hz, 1H), 7.96 - 7.86 (m, 1H), 7.76 - 7.67 (m, 1H), 7.48 (dd, J = 6.6, 5.2 Hz, 2H), 7.41 (t, J = 8.8 Hz, 1H), 7.35 (s, 2H), 7.30 (d, J = 8.4 Hz, 1H), 4.80 (s, 1H), 3.95 (s, 3H), 3.48 (dd, J = 13.6, 6.8 Hz, 2H), 3.35 - 3.25 (m, 2H), 2.29 (s, 1H), 2.16 (d, J = 20.2 Hz, 1H). $^{19}F$ NMR (376 MHz, DMSO) $\delta$ -74.11 (s, 4.03F), -108.28 (s, 1F), -133.36 (s, 0.97F).

**Embodiment 17**

Synthesis of (R)-4-(8-(3 -aminopiperidin-1-yl)-3 -(2-methyll-2H-indazol-5 -yl)imidazo [1,2-a]pyrazin-2-yl)-2-fluorobenzonitrile (compound 17)

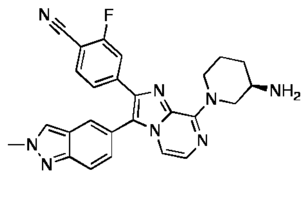

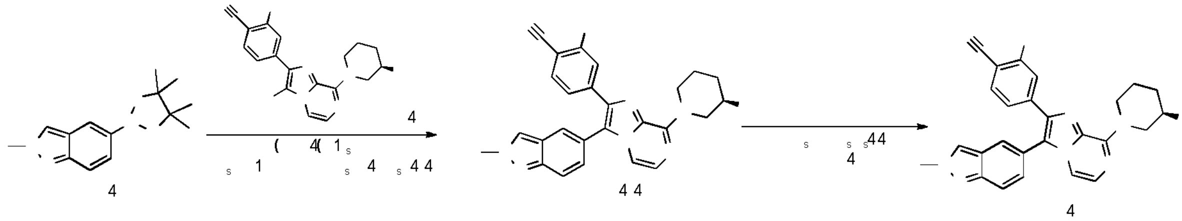

**Step 1**

A compound 17-1 (15 mg, 0.32 mmol), a compound 17-1A (20 mg, 0.04 mmol), $Pd(dppf)Cl_2$ (5 mg, 0.01 mmol) and $K_2CO_3$ (5.5 mg, 0.08 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 17-2 (6 mg, yield: 19%). LCMS $(M+H)^+$ = 567.0.

**Step 2**

The compound 17-2 (6 mg, 0.01 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 17 (3 mg, yield: 46%). LCMS $(M+H)^+$ = 467.3. $^1H$ NMR (400 MHz, DMSO) $\delta$ 8.51 (s, 1H), 8.09 (s, 2H), 7.95 (s, 1H), 7.84 (dd, J = 12.4, 5.6 Hz, 2H), 7.75 (d, J = 10.8 Hz, 1H), 7.49 (dd, J = 8.2, 1.2 Hz, 1H), 7.38 (s, 2H), 7.22 (dd, J = 8.8, 1.6 Hz, 1H), 5.06 (d, J = 34.0 Hz, 2H), 4.25 (s, 3H), 3.59 (d, J = 12.8 Hz, 2H), 2.09 (s, 2H), 1.91 (s, 1H), 1.69 (d, J = 8.4 Hz, 2H). $^{19}F$ NMR (376 MHz, DMSO) $\delta$ -73.77 (s, 4.77F), -108.07 (s, 1F).

**Embodiment 18**

Synthesis of (R)-4-(8-(3-aminopiperidin-1-yl)-3-(6-fluoro-1-(2-hydroxy-2-methylpropyl)-1H-benzo[d][1,2,3]triazol-5-yl)imidazo[1,2-a]pyrazin-2-yl)-2-fluorobenzonitrile (compound 18)

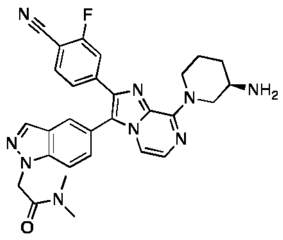

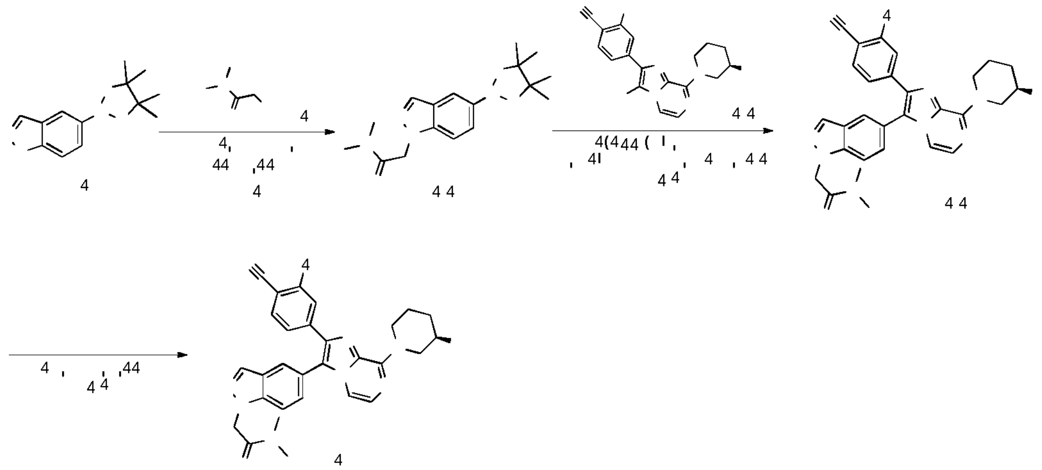

**Step 1**

A compound 18-1 (300 mg, 1.23 mmol), a compound 18-1A (405 mg, 2.46 mmol) and $Cs_2CO_3$ (1.2 g, 3.69 mmol) were dissolved in DMF (10 mL), and the reaction system was heated to 60°C and reacted under the protection of nitrogen for 2 h. The reaction system was quenched with water. Extraction was conducted with ethyl acetate (3*20 mL), drying was conducted with anhydrous sodium sulfate, and spin-drying was conducted. Then, column chromatography was conducted (PE:EA=2:1) to obtain a white solid compound 18-2 (120 mg, yield: 30%). LCMS $(M+H)^+$ = 330.1.

**Step 2**

The compound 18-2 (60 mg, 0.18 mmol), a compound 18-2A (60 mg, 0.12 mmol), $Pd(dppf)Cl_2$ (10 mg, 0.02 mmol) and $K_2CO_3$ (50 mg, 0.36 mmol) were dissolved in 1,4-dioxane/$H_2O$ (3/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 18-3 (20 mg, yield: 20%). LCMS $(M+H)^+$ = 638.1.

**Step 3**

The compound 18-3 (20 mg, 0.03 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 18 (3 mg, yield: 19%. LCMS $(M+H)^+$ = 538.3. $^1H$ NMR (400 MHz, DMSO) δ 8.21 (s, 1H), 8.06 (s, 2H), 7.99 (s, 1H), 7.84 (dd, J = 12.8, 8.0 Hz, 2H), 7.75 (d, J = 10.8 Hz, 1H), 7.47 (d, J = 8.2 Hz, 1H), 7.42 (d, J = 8.6 Hz, 1H), 7.37 (s, 2H), 5.53 (s, 2H), 5.07 (d, J = 21.0 Hz, 2H), 3.16 (s, 3H), 2.88 (s, 3H), 2.67 (s, 2H), 2.33 (s, 1H), 2.09 (s, 1H), 1.92 (s, 1H), 1.69 (s, 2H). $^{19}F$ NMR (376 MHz, DMSO) δ -73.79 (s, 4.77F), -107.97 (s, 1F).

**Embodiment 19**

Synthesis of (R)-4-(8-(3-aminopiperidin-1-yl)-3-(6-fluoro-1-(2-hydroxy-2-methylpropyl)-1H-indazol-5-yl)imidazo[1,2-a] pyrazin-2-yl)-2-fluorobenzonitrile (compound 19)

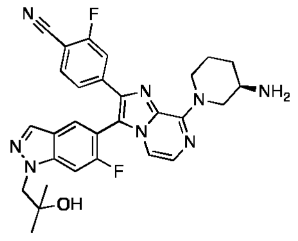

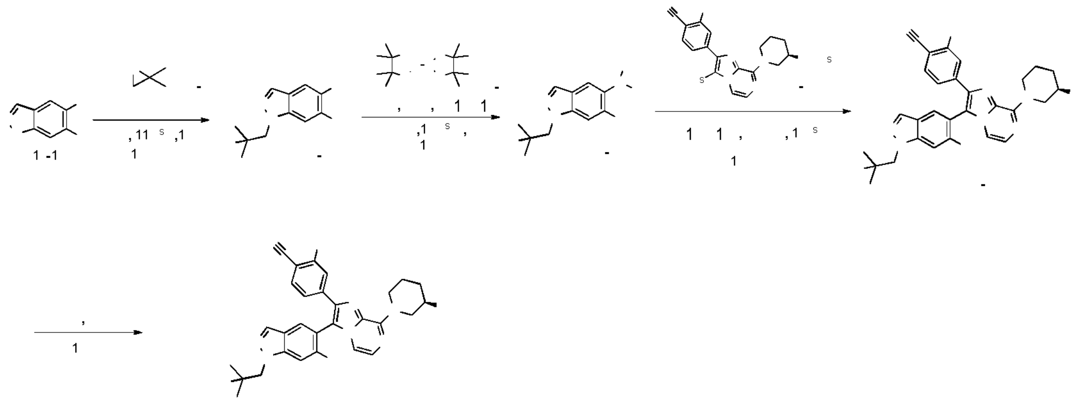

**Step 1**

A compound 19-1 (1 g, 4.7 mmol) was dissolved in DMF (15 mL), a compound 19-2 (673 mg, 9.35 mmol) and potassium carbonate (1.3 g, 9.35 mmol) were added, and the reaction system was heated to 110°C and stirred for a reaction overnight. Cooling was conducted to room temperature, ethyl acetate was added for dilution, washing was conducted with water, and an organic phase was concentrated to obtain a crude product. Then, the crude product was treated with column chromatography (PE:EA=1:2) to obtain a white solid compound 19-3 (600 mg, yield: 45%). LCMS $(M+H)^+$ = 287.0.

**Step 2**

A mixture of the compound 19-3 (300 mg,1 mmol), tricyclohexylphosphine (59 mg, 0.2 mmol), a compound 19-4 (400 mg, 1.57 mmol) and potassium acetate (290 mg, 2.1 mmol) was dissolved in dioxane (10 mL), and a catalyst $Pd_2(dba)_3$ (96 mg, 0.1 mmol) was added. The system was replaced with nitrogen for 3 times and heated to 100°C and reacted for 2 h. Cooling was conducted to room temperature, concentration was conducted to remove a solvent, extraction was conducted with water and ethyl acetate, and an organic phase was back-washed with a saturated salt solution and dried with anhydrous sodium sulfate to obtain a crude product. Then, the crude product was treated with column chromatography (PE:EA=1:1) to obtain a white solid compound 19-5 (200 mg, yield: 76%). LCMS $(M+H)^+$ = 253.0.

**Step 3**

The compound 19-5 (70 mg, 0.28 mmol) was dissolved in a mixed solvent of dioxane (5 mL) and water (1 mL), a compound 19-6 (143 mg, 0.28 mmol), potassium carbonate (77 mg, 0.56 mmol) and a catalyst $Pd(dppf)Cl_2$ (21 mg, 0.03 mmol) were added, and the system was replaced with nitrogen for 3 times and heated to 100°C and reacted for 2 h. Cooling was conducted to room temperature, concentration was conducted to remove the solvent, extraction was conducted with water and ethyl acetate, and an organic phase was back-washed with a saturated salt solution and dried with anhydrous sodium sulfate to obtain a crude product. Then, the crude product was treated with column chromatography (PE:EA=1:1) to obtain a white solid compound 19-7 (100 mg, yield: 56%). LCMS $(M+H)^+$ = 643.0.

**Step 4**

The compound 19-7 (50 mg, 0.08 mmol) was dissolved in dichloromethane (2 mL), and a dichloromethane solution (1 mL) of trifluoroacetic acid (1 mL) was dropped under an ice water bath. Stirring was conducted for a reaction at room temperature for 1 h. Concentration was conducted to obtain a crude product. Then, the crude product was treated with prep-HPLC to obtain a white solid compound 19 (27 mg, yield: 64%). LCMS $(M+H)^+$ = 543.0. $^1H$ NMR (400 MHz, DMSO) δ 8.23 (s, 1H), 8.12 - 8.01 (m, 4H), 7.91 - 7.83 (m, 2H), 7.81 -7.75 (m, 1H), 7.49 - 7.34 (m, 3H), 5.18 - 4.90 (m, 2H), 4.46 - 4.31 (m, 2H), 3.42 - 3.30 (m, 2H), 2.43 - 2.21 (m, 1H), 2.17 - 2.02 (m, 1H), 1.98 - 1.82 (m, 1H), 1.77 - 1.62 (m, 2H), 1.20 (d, J = 19.3 Hz, 6H). $^{19}F$NMR (376 MHz, DMSO) δ -73.94 (s, 3F), -107.80 (s, 1F), -116.61 (s, 1F).

**Embodiment 20**

Synthesis of (R)-4-(8-(3-aminopiperidin-1-yl)-3-(1-(2-hydroxy-2-methylpropyl)-1H-benzo[d][1,2,3]triazol-5-yl)imidazo[1,2-a]pyrazin-2-yl)-2-fluorobenzonitrile (compound 20)

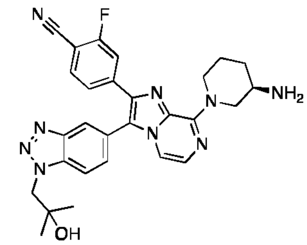

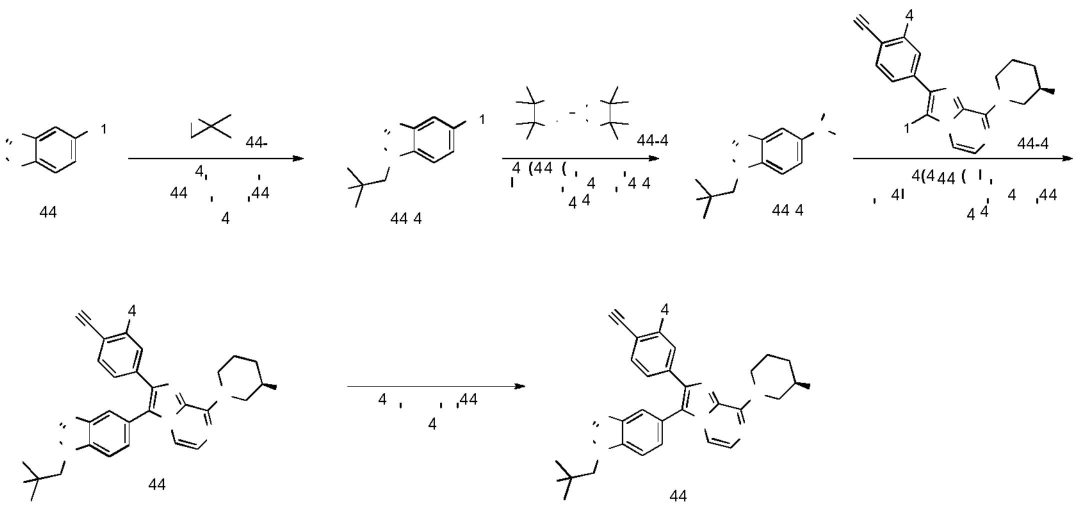

**Step 1**

A compound 20-1 (1 g, 5.1 mmol), a compound 20-1A (1.8 g, 25.5 mmol) and $K_2CO_3$ (1.4 g, 10.2 mmol) were dissolved in DMF (15 mL), and the reaction system was heated to 100°C for a microwave reaction for 2 h. The reaction system was quenched with water. Extraction was conducted with ethyl acetate (3*100 mL), drying was conducted with anhydrous sodium sulfate, and spin-drying was conducted. Then, column chromatography was conducted (PE:EA=1:2) to obtain a white solid compound 20-2 (250 mg, yield: 18%). LCMS $(M+H)^+$ = 270.1.

**Step 2**

The compound 20-2 (250 mg, 0.93 mmol), a compound 20-2A (354 mg, 1.4 mmol), $Pd_2(dba)_3$ (92 mg, 0.1 mmol), tricyclohexylphosphine (56 mg, 0.2 mmol) and potassium acetate (176 mg, 1.8 mmol) were dissolved in 1,4-dioxane (15 mL), the reaction system was heated to 110°C, and a reaction solution was subjected to a reaction under the protection of nitrogen for 12 h. The reaction solution was concentrated to obtain a crude product. Then, the crude

product was separated by a high performance liquid chromatography column to obtain a white solid compound 20-3 (100 mg, yield: 46%). LCMS $(M+H)^+$ = 236.0.

**Step 3**

The compound 20-3 (100 mg, 0.45 mmol), a compound 20-3A (154 mg, 0.3 mmol), Pd(dppf)$Cl_2$ (22 mg, 0.03 mmol) and $K_2CO_3$ (83 mg, 0.6 mmol) were dissolved in 1,4-dioxane/$H_2O$ (4/0.5 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 20-4 (50 mg, yield: 27%). LCMS $(M+H)^+$ = 626.2.

**Step 4**

The compound 20-4 (50 mg, 0.08 mmol) was dissolved in DCM (2 mL), and TFA (0.5mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 20 (22 mg, yield: 52%. LCMS $(M+H)^+$ = 526.3.

$^1$H NMR (400 MHz, DMSO) δ 8.30 (s, 1H), 8.15 (d, J = 8.7 Hz, 4H), 7.86 - 7.79 (m, 1H), 7.77 - 7.70 (m, 1H), 7.58 (dd, J = 8.6, 1.4 Hz, 1H), 7.44 (dd, J = 8.2, 1.4 Hz, 1H), 7.42 - 7.35 (m, 2H), 5.15 (d, J = 11.3 Hz, 1H), 5.05 (d, J = 12.5 Hz, 1H), 4.72 (s, 2H), 3.65 - 3.52 (m, 2H), 3.37 (s, 1H), 2.09 (s, 1H), 1.91 (d, J = 4.5 Hz, 1H), 1.69 (dd, J = 17.6, 9.4 Hz, 2H), 1.23 (s, 6H). $^{19}$F NMR (376 MHz, DMSO) δ -74.17 (s, 3F), -107.97 (s, 1F).

**Embodiment 21**

Synthesis of (R)-4-(8-(3-aminopiperidin-1-yl)-3-(6-fluoro-2-(2-hydroxy-2-methylpropyl)-2H-indazol-5-yl)imidazo[1,2-a]pyrazin-2-yl)-2-fluorobenzonitrile (compound 21)

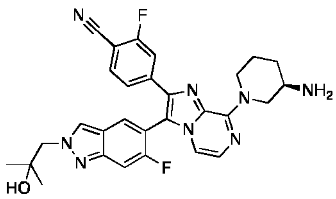

Step 1

A compound 21-1 (1 g, 4.7 mmol) was dissolved in DMF (15 mL), a compound 21-2 (673 mg, 9.35 mmol) and potassium carbonate (1.3 g, 9.35 mmol) were added, and the reaction system was heated to 110°C and stirred for a reaction overnight. Cooling was conducted to room temperature, ethyl acetate was added for dilution, washing was conducted with water, and an organic phase was concentrated to obtain a crude product. Then, the crude product was treated with column chromatography (PE:EA=1:2) to obtain a white solid compound 21-3 (450 mg, yield: 34%). LCMS $(M+H)^+$ = 287.0.

**Step 2**

A mixture of the compound 21-3 (390 mg, 1.4 mmol), tricyclohexylphosphine (77 mg, 0.3 mmol), a compound 21-4 (520 mg, 2 mmol) and potassium acetate (377 mg, 2.7 mmol) was dissolved in dioxane (10 mL), and a catalyst $Pd_2(dba)_3$ (125 mg, 0.14 mmol) was added. The system was replaced with nitrogen for 3 times and heated to 100°C and reacted for 2 h. Cooling was conducted to room temperature, concentration was conducted to remove a solvent, extraction was conducted with water and ethyl acetate, and an organic phase was back-washed with a saturated salt solution and dried with anhydrous sodium sulfate to obtain a crude product. Then, the crude product was treated with column chromatography (PE:EA=1:1) to obtain a white solid compound 21-5 (180 mg, yield: 52%). LCMS $(M+H)^+$ = 253.0.

**Step 3**

The compound 21-5 (100 mg, 0.4 mmol) was dissolved in a mixed solvent of dioxane (5 mL) and water (1 mL), a compound 21-6 (204 mg, 0.4 mmol), potassium carbonate (110 mg, 0.8 mmol) and a catalyst Pd(dppf)$Cl_2$ (29 mg, 0.04 mmol) were added, and the system was replaced with nitrogen for 3 times and heated to 100°C and reacted for 2 h. Cooling was conducted to room temperature, concentration was conducted to remove the solvent, extraction was conducted with water and ethyl acetate, and an organic phase was back-washed with a saturated salt solution and dried with anhydrous sodium sulfate to obtain a crude product. Then, the crude product was treated with column chromatography (PE:EA=1:1) to obtain a white solid compound 21-7 (200 mg, yield: 79%). LCMS $(M+H)^+$ = 643.0.

**Step 4**

The compound 21-7 (30 mg, 0.05 mmol) was dissolved in dichloromethane (2 mL), and a dichloromethane solution (1 mL) of trifluoroacetic acid (0.5 mL) was dropped under an ice water bath. Stirring was conducted for a reaction at room temperature for 1 h. Concentration was conducted to obtain a crude product. Then, the crude product was treated with prep-HPLC to obtain a white solid compound 21 (8 mg, yield: 32%). LCMS $(M+H)^+$ = 543.0. $^1H$ NMR (400 MHz, DMSO) $\delta$ 8.54 - 8.49 (m, 1H), 8.18 - 8.05 (m, 4H), 7.91 - 7.85 (m, 1H), 7.84 - 7.77 (m, 1H), 7.77 - 7.69 (m, 1H), 7.52 - 7.45 (m, 1H), 7.41 (s, 2H), 5.22 - 4.81 (m, 3H), 4.40 (s, 2H), 3.44 - 3.31 (m, 2H), 2.40 - 2.26 (m, 1H), 2.14 - 2.04 (m, 1H), 1.98 - 1.86 (m, 1H), 1.78 - 1.63 (m, 2H), 1.17 (d, J = 18.3 Hz, 6H). $^{19}F$ NMR (376 MHz, DMSO) $\delta$ -73.69 (s, 3F), -108.02 (s, 1F), -118.02 (s, 1F).

**Embodiment 22**

Synthesis of (R)-4-(8-(3-aminopiperidin-1-yl)-3-(2-fluoro-4-(2-hydroxy-2-methylpropyl)phenyl)imidazo[1,2-a]pyrazin-2-yl)-2-fluorobenzonitrile (compound 22)

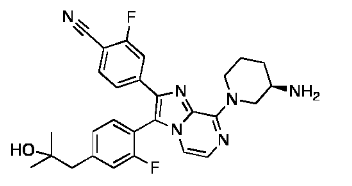

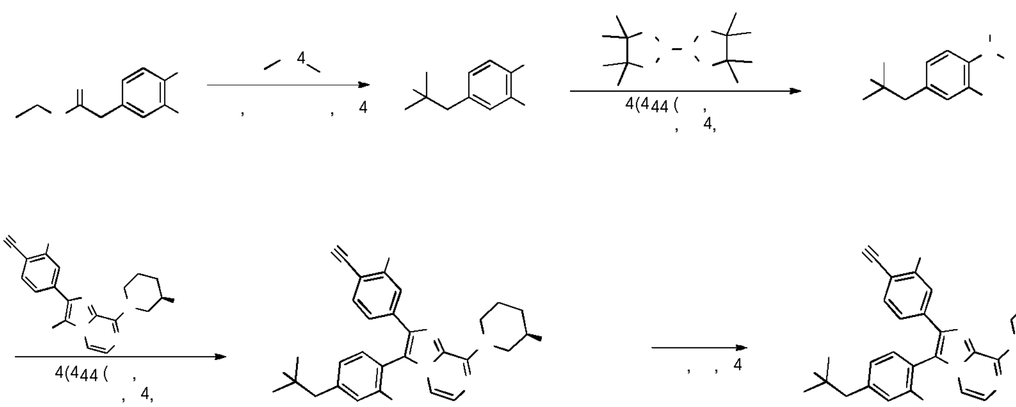

**Step 1**

A compound 22-1 (800 mg, 3 mmol) was dissolved in THF (6 mL), and 6 mL of a tetrahydrofuran solution of 1 M methyl magnesium bromide (22-2) (6 mmol) was added dropwise at -40 °C. After the dropping was completed, the temperature was restored to room temperature, and stirring was conducted for a reaction for 2 h. A saturated ammonium chloride solution was added dropwise for quenching, extraction was conducted with ethyl acetate, and an organic phase was back-washed with a saturated salt solution, dried with anhydrous sodium sulfate and concentrated to obtain a crude product. Then, the crude product was purified by SGC (PE:EA=3:1) to obtain a white solid compound 22-3 (600 mg, yield: 79%. $^1$HNMR (400 MHz, $CDCl_3$) $\delta$ 7.49 - 7.44 (m, 1H), 7.02 (dd, J = 9.6, 1.9 Hz, 1H), 6.90 (dd, J = 8.2, 1.9 Hz,

1H), 2.73 (s, 2H), 1.23 (s, 6H).

**Step 2**

The compound 22-3 (20 mg, 0.08 mmol) and a compound 22-4 (25 mg, 0.1 mmol) were dissolved in dioxane (5 mL), potassium acetate (16 mg, 0.163 mmol) and Pd(dppf)$Cl_2$ (6 mg, 0.008 mmol) were added, and the system was replaced with nitrogen for 3 times and heated for reflux for a reaction overnight. Cooling was conducted to room temperature, and concentration was conducted to remove a solvent. Then, a resulting crude product was purified by SGC (PE:EA=2:1) to obtain a white solid compound 22-5 (18 mg, yield: 75%). LCMS $(M+H)^+$ = 295.0.

**Step 3**

The compound 22-5 (18 mg, 0.06 mmol), a compound 22-6 (47 mg, 0.09 mmol) and potassium carbonate (25 mg, 0.18 mmol) were dissolved in a mixed solvent of dioxane (4 mL) and water (1 mL), and Pd(dppf)$Cl_2$ (5 mg, 0.006 mmol) was added. The system was replaced with nitrogen for 3 times and heated to 100°C and reacted for 3 h. Concentration was conducted to remove the solvent. Then, a resulting crude product was purified by prep-HPLC to obtain a white solid compound 22-7 (30 mg, yield: 81%). LCMS $(M+H)^+$ = 603.0.

**Step 4**

The compound 22-7 (30 mg, 0.05 mmol) was dissolved in dichloromethane (2 mL), trifluoroacetic acid (1 mL) was added and stirred for a reaction at room temperature for 1 h, and concentration was conducted to remove a solvent. Then, a resulting crude residue was purified by prep-HPLC to obtain a white solid compound 22 (5.5 mg, yield: 22%. LCMS $(M+H)^+$ = 503.0. $^1$H NMR (400 MHz, DMSO) δ 8.17 - 8.01 (m, 3H), 7.95 - 7.89 (m, 1H), 7.73 - 7.65 (m, 1H), 7.56 - 7.50 (m, 1H), 7.49 - 7.41 (m, 2H), 7.40 - 7.28 (m, 3H), 5.14 - 4.83 (m, 2H), 3.43 - 3.28 (m, 2H), 2.87 - 2.77 (m, 2H), 2.37 - 2.22 (m, 1H), 2.14 - 2.02 (m, 1H), 1.97 - 1.84 (m, 1H), 1.76 - 1.63 (m, 2H), 1.15 (s, 6H). $^{19}$F NMR (376 MHz, DMSO) δ -73.836 (s, 3F), -107.991 (s, 1F), -114.191 (s, 1F).

**Embodiment 23**

Synthesis of (*S*)-4-(8-(3-aminopiperidin-1-yl)-3-(6-fluoro-1-(2-hydroxy-2-methylpropyl)-1H-benzo[d][1,2,3]triazol-5-yl)imidazo[1,2-a]pyrazin-2-yl)-2-fluorobenzonitrile (compound 23)

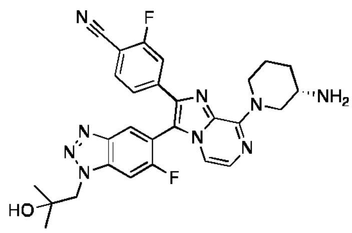

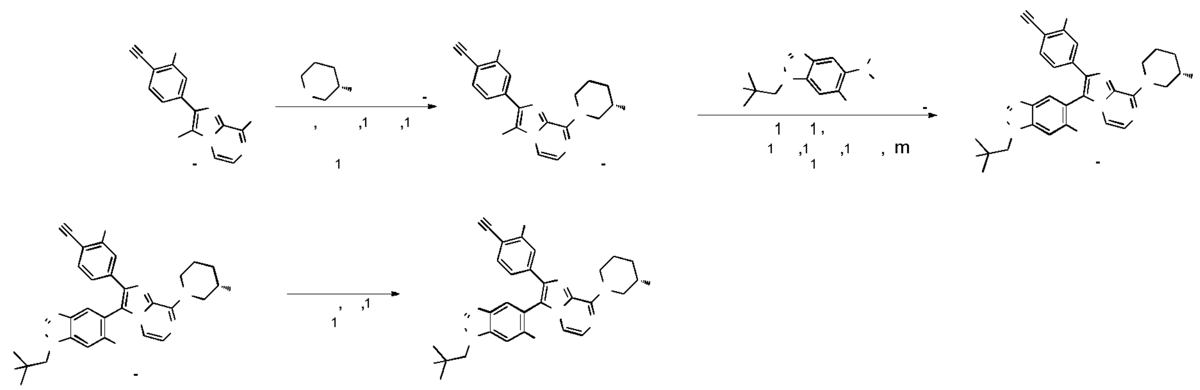

**Step 1**

A mixture of a compound 23-1 (225 mg, 0.64 mmol), a compound 23-2 (155 mg, 0.77 mmol), DIPA (166 mg, 1.3 mmol) and NMP (5 mL) was heated to 100°C and stirred for a reaction for 1 h. Cooling was conducted to room temperature, a reaction solution was dropped into ice water, extraction was conducted with ethyl acetate, and concentration was conducted to obtain a crude product. Then, the crude product was purified by SGC (with a mixture of DCM and MeOH at a ratio of 20:1) to obtain a white solid compound 23-3 (50 mg, yield: 15%). LCMS $(M+H)^+$ = 515.0.

**Step 2**

The compound 23-3 (25 mg, 0.05 mmol), a compound 23-4 (13 mg, 0.05 mmol) and an aqueous solution (0.5 mL) of potassium carbonate (71 mg, 0.5 mmol) were dissolved in dioxane (1 mL), and tetratriphenylphosphine palladium (6 mg, 0.005 mmol) was added. The system was replaced with nitrogen for 3 times and heated to 100°C for a microwave reaction for 15 min. Concentration was conducted to remove a solvent. Then, a resulting crude product was purified by SGC (PE:EA=1:1) to obtain a white solid compound 23-5 (20 mg, yield: 64%). LCMS $(M+H)^+$ = 644.0.

**Step 3**

The compound 23-5 (20 mg, 0.03 mmol) was dissolved in dichloromethane (2 mL), trifluoroacetic acid (1 mL) was added and stirred for a reaction at room temperature for 1 h, and concentration was conducted to remove a solvent. Then, a resulting crude residue was purified by prep-HPLC to obtain a white solid compound 23 (5 mg, yield: 30%. LCMS $(M+H)^+$ = 544.0. $^1H$ NMR (400 MHz, DMSO) δ 8.47- 8.43 (m, 1H), 8.07 (s, 4H), 7.88 - 7.83 (m, 1H), 7.82 - 7.77 (m, 1H), 7.43 (s, 3H), 5.12 - 4.95 (m, 2H), 4.69 (s, 2H), 3.48 - 3.28 (m, 3H), 2.12 - 2.07 (m, 1H), 1.94 - 1.88 (m, 1H), 1.75 - 1.66 (m, 2H), 1.23 (d, J = 25.0 Hz, 6H). $^{19}F$ NMR (376 MHz, DMSO) δ -74.185 (s, 4.26F), -107.739 (s, 1F), -114.368 (s, 1F).

**Embodiment 24**

Synthesis of (*R*)-4-(8-(3-aminopyrrolidin-1-yl)-3-(6-fluoro-1-(2-hydroxy-2-methylpropyl)-1H-benzo[d][1,2,3]triazol-5-yl)imidazo[1,2-a]pyrazin-2-yl)-2-fluorobenzonitrile (compound 24)

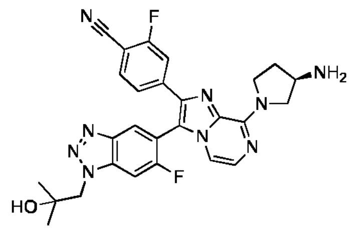

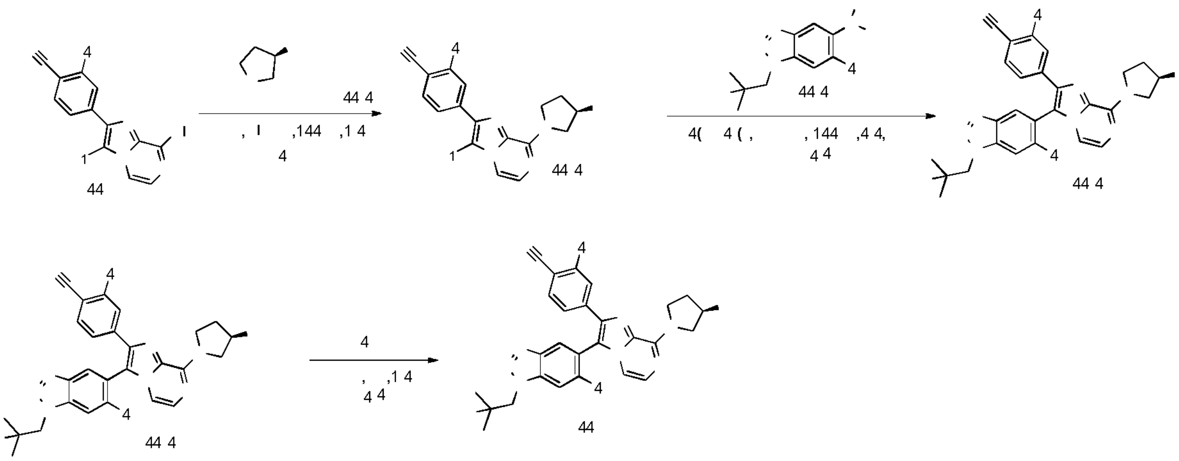

**Step 1**

A compound 24-1 (300 mg, 0.857 mmol) was dissolved in NMP (3 mL), and a compound 24-2 (192 mg, 1.0 mmol) and DIPA (222 mg, 1.714 mmol) were added, heated to 120°C and stirred for 2 h. Cooling was conducted to room temperature, a reaction solution was added to ice water for quenching under an ice water bath, extraction was conducted with ethyl acetate, and an organic phase was back-washed with a saturated salt solution, dried with anhydrous sodium sulfate and concentrated. Then, a resulting crude product was purified by SGC (PE:EA=1:1) to obtain a white solid compound 24-3 (110 mg, yield: 26%). LCMS $(M+H)^+$ = 501.0.

**Step 2**

The compound 24-3 (70 mg, 0.14 mmol), a compound 24-4 (54 mg, 0.21 mmol) and potassium carbonate (39 mg, 0.28 mmol) were dissolved in a mixed solvent of dioxane (2 mL) and water (0.5 mL), and tetratriphenylphosphine palladium (17 mg, 0.014 mmol) was added. The system was replaced with nitrogen for 3 times and heated to 130°C for a microwave reaction for 2 h. Concentration was conducted to remove the solvent. Then, a resulting crude product was purified by prep-HPLC to obtain a white solid compound 24-5 (10 mg, yield: 11%). LCMS $(M+H)^+$ = 630.0.

**Step 3**

The compound 24-5 (10 mg, 0.1 mmol) was dissolved in dichloromethane (2 mL), trifluoroacetic acid (1 mL) was added and stirred for a reaction at room temperature for 1 h, and concentration was conducted to remove a solvent. Then, a resulting crude residue was purified by prep-HPLC to obtain a white solid compound 24 (0.9 mg, yield: 9%. LCMS $(M+H)^+$ = 530.0. $^1H$ NMR (400 MHz, DMSO) $\delta$ 8.51 - 8.41 (m, 1H), 8.26 - 8.03 (m, 4H), 7.93 - 7.83 (m, 1H), 7.78 - 7.69 (m, 1H), 7.45 - 7.41 (m, 1H), 7.41 - 7.28 (m, 2H), 4.99 - 4.86 (m, 1H), 4.73 - 4.66 (m, 2H), 4.09 - 3.96 (m, 2H), 2.62 - 2.56 (m, 1H), 2.43 - 2.31 (m, 2H), 2.21 - 2.10 (m, 1H), 1.29 - 1.17 (m, 6H). $^{19}F$ NMR (376 MHz, DMSO) $\delta$ -73.659 (s, 6F), -107.878 (s, 1F), -114.446 (s, 1F).

**Embodiment 25**

Synthesis of (*R*)-4-(8-(3-aminopiperidin-1-yl)-3-(6-fluoro-1-((1-hydroxycyclobutyl) methyl)-1H-benzo[d][1,2,3]triazol-5-yl)imidazo[1,2-a]pyrazin-2-yl)-2-fluorobenzonitrile (compound 25)

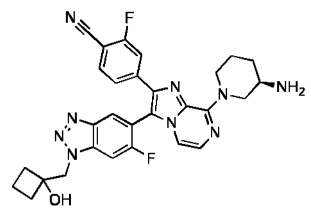

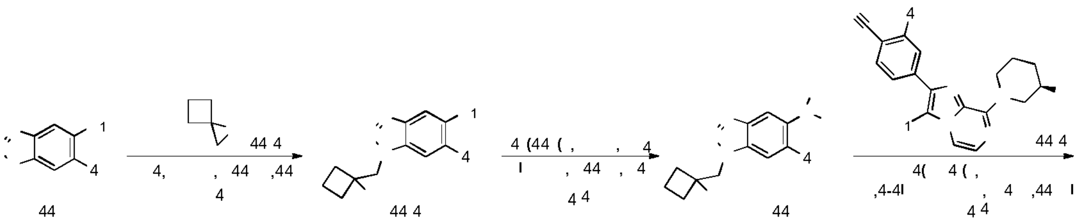

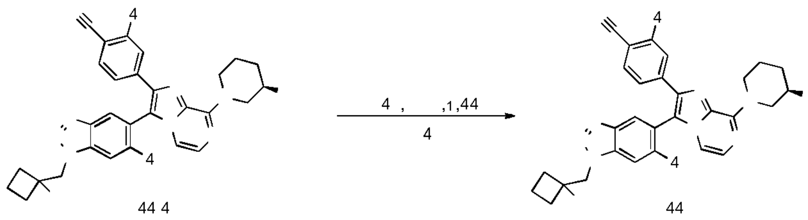

**Step 1**

A compound 25-1 (268 mg, 1.25 mmol), a compound 25 -2 (210 mg, 2.5 mmol) and $K_2CO_3$ (345 mg, 2.5 mmol) were dissolved in DMF (5 mL), and the reaction system was heated to 100°C and reacted for 2 h. The reaction system was quenched with water. Extraction was conducted with ethyl acetate (3*50 mL), drying was conducted with anhydrous sodium sulfate, and spin-drying was conducted. Then, column chromatography was conducted (PE:EA=1:1) to obtain of a white solid compound 25-3 (140 mg, yield: 38%). LCMS $(M+H)^+$ = 300.1.

**Step 2**

The compound 25-3 (140 mg, 0.47 mmol), bis(pinacolato)diboron (238 mg, 0.94 mmol), $Pd_2(dba)_3$ (129 mg, 0.14 mmol), tricyclohexylphosphine (78 mg, 0.28 mmol) and potassium acetate (135 mg, 1.4 mmol) were dissolved in 1,4-dioxane (5 mL), the reaction system was heated to 125°C, and a reaction solution was subjected to a reaction under the protection of nitrogen for 1 h. The reaction solution was concentrated to obtain a crude product. Then, the crude product was separated by a high performance liquid chromatography column to obtain a white solid compound 25-4 (45 mg, yield: 36%). LCMS $(M+H)^+$ = 266.0.

**Step 3**

The compound 25-4 (45 mg, 0.17 mmol), a compound 25-5 (87 mg, 0.17 mmol), $Pd(PPh_3)_4$ (39 mg, 0.03 mmol) and a 1 M $K_2CO_3$ aqueous solution (1 mL) were dissolved in 1,4-dioxane (2 mL), the reaction system was heated to 110°C, and a reaction solution was subjected to a reaction under the protection of nitrogen for 30 min and then concentrated. Then, a resulting crude product was separated by a high performance liquid chromatography column to obtain a white solid compound 25-6 (15 mg, yield: 13%). LCMS $(M+H)^+$ = 656.2.

**Step 4**

The compound 25-6 (15 mg, 0.02 mmol) was dissolved in DCM (2 mL), and TFA (0.5 mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 25 (12 mg, yield: 100%. LCMS $(M+H)^+$ = 556.3. $^1H$ NMR (400 MHz, DMSO) δ 8.45 (d, J = 5.9 Hz, 1H), 8.08 - 8.00 (m, 3H), 7.89 - 7.83 (m, 1H), 7.80 (d, J = 8.6 Hz, 1H), 7.47 - 7.36 (m, 3H), 5.60 (s, 1H), 5.08 (s, 2H), 4.90 (dd, J = 25.0, 10.6 Hz, 3H), 3.70 (d, J = 20.8 Hz, 2H), 2.24 (d, J = 5.3 Hz, 2H), 2.06 (d, J = 12.2 Hz, 2H), 1.91 (s, 2H), 1.78 - 1.66 (m, 4H). $^{19}F$ NMR (376 MHz, DMSO) δ -73.87 (s, 3F), -107.68 (s, 1F), -114.31 (s, 1F).

**Compoud 26** [00508] **Synthesis of (*S*)-4-(8-(3-aminopiperidin-1-yl)-3-(1-(2-ethyl-2-hydroxybutyl)-6-fluoro-1H-benzo [d] [1,2,3] triazol-5-yl)imidazo [1,2-a] pyrazin-2-yl)-2-fluorobenzonitrile (compound 26)**

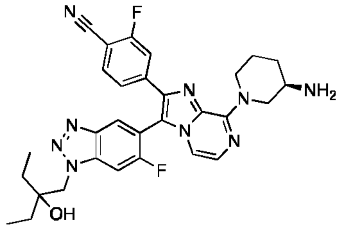

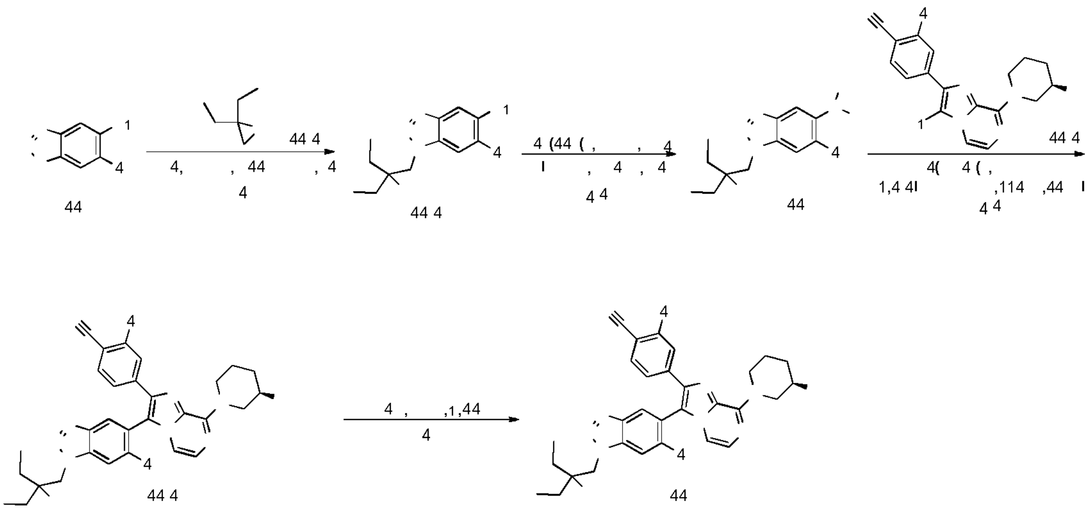

**Step 1**

A compound 26-1 (71 mg, 0.33 mmol), a compound 26 -2 (110 mg, 1.10 mmol) and $K_2CO_3$ (91 mg, 0.66 mmol) were dissolved in DMF (2 mL), and the reaction system was heated to 100°C for a microwave reaction for 1 h. The reaction system was quenched with water. Extraction was conducted with ethyl acetate (3*50 mL), drying was conducted with anhydrous sodium sulfate, and spin-drying was conducted. Then, column chromatography was conducted (PE:EA=1:1) to obtain a white solid compound 26-3 (60 mg, yield: 57%). LCMS $(M+H)^+$ = 316.1.

**Step 2**

The compound 26-3 (50 mg, 0.16 mmol), bis(pinacolato)diboron (81 mg, 0.32 mmol), $Pd_2(dba)_3$ (27 mg, 0.03 mmol), tricyclohexylphosphine (17 mg, 0.06 mmol) and potassium acetate (47 mg, 0.48 mmol) were dissolved in 1,4-dioxane (5 mL), the reaction system was heated to 110°C, and a reaction solution was subjected to a microwave reaction under the protection of nitrogen for 1 h. The reaction solution was concentrated to obtain a crude product. Then, the crude product was separated by a high performance liquid chromatography column to obtain a white solid compound 26-4 (20 mg, yield: 44%). LCMS $(M+H)^+$ = 288.0.

**Step 3**

The compound 26-4 (20 mg, 0.07 mmol), a compound 26-5 (36 mg, 0.07 mmol), $Pd(PPh_3)_4$ (2 mg, 0.02 mmol) and a 1 M $K_2CO_3$ aqueous solution (1 mL) were dissolved in 1,4-dioxane (2 mL), the reaction system was heated to 110°C, and a reaction solution was subjected to a reaction under the protection of nitrogen for 15 min and then concentrated. Then, a resulting crude product was separated by a high performance liquid chromatography column to obtain a white solid compound 26-6 (5 mg, yield: 13%). LCMS $(M+H)^+$ = 672.2.

**Step 4**

The compound 26-6 (5 mg, 0.01 mmol) was dissolved in DCM (2 mL), and TFA (0.5 mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 26 (2.7 mg, yield: 63%. LCMS $(M+H)^+$ = 572.3. $^1H$ NMR (400

MHz, DMSO) δ 8.45 (s, 1H), 8.09 (s, 4H), 7.86 (s, 1H), 7.81 (s, 1H), 7.42 (s, 3H), 5.10 (s, 1H), 4.96 (s, 1H), 4.69 (s, 2H), 3.41 - 3.31 (m, 3H), 2.09 (s, 2H), 1.91 (s, 2H), 1.70 (s, 2H), 1.48 (s, 2H), 1.39 (s, 2H), 0.94 (d, J = 7.1 Hz, 6H). $^{19}F$ NMR (376 MHz, DMSO) δ -73.83 (s, 7F), -107.69 (s, 1F), -114.52 (s, 1F).

**Compound 27**

Synthesis of 4-(8-((*1S,2S,4R*)-2-amino-7-azabicyclo[2.2.1]heptane-7-yl)-3-(6-fluoro-1-(2-hydroxy-2-methylpropyl)-1H-benzo[d][1,2,3]triazol-5-yl)imidazo[1,2-a]pyrazin-2-yl )-2-fluorobenzonitrile (compound 27)

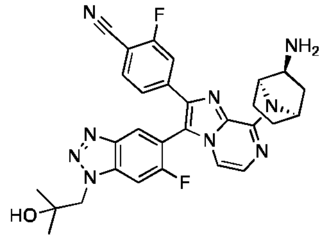

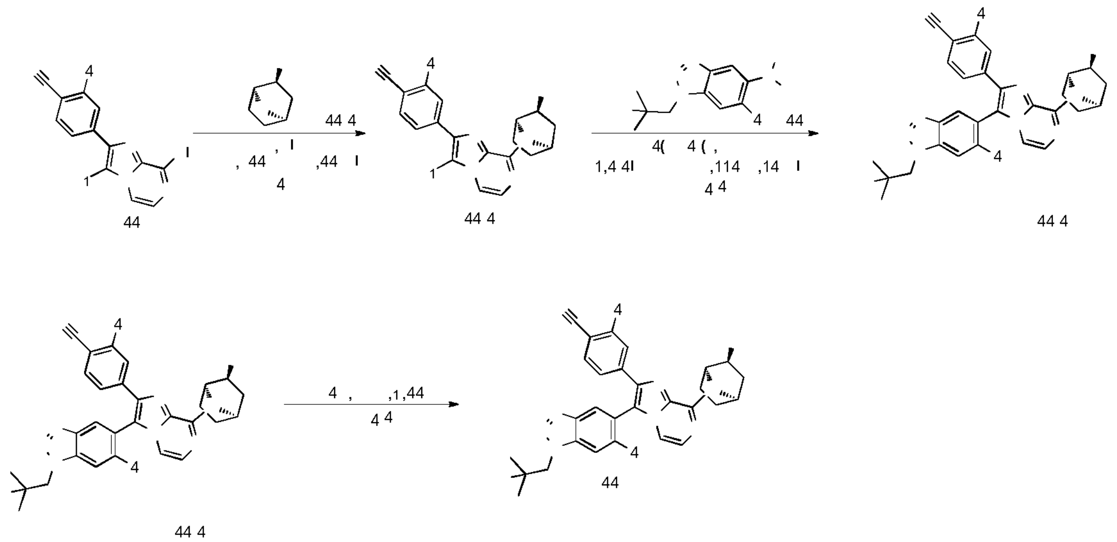

**Step 1**

A compound 27-1 (400 mg, 1.14 mmol), a compound 27 -2 (240 mg, 0.76 mmol), DMAP (12 mg, 0.1 mmol) and DIPEA(196 mg, 1.52 mmol) were dissolved in NMP (8 mL), and the reaction system was heated to 120°C for a microwave reaction for 30 min. The reaction system was quenched with water. Extraction was conducted with ethyl acetate (3*100 mL), drying was conducted with anhydrous sodium sulfate, and spin-drying was conducted. Then, column chromatography was conducted (PE:EA=1:2) to obtain a white solid compound 27-3 (100 mg, yield: 25%). LCMS $(M+H)^+$ = 527.1.

**Step 2**

The compound 27-3 (100 mg, 0.19 mmol), a compound 27-4 (70 mg, 0.28 mmol), $Pd(PPh_3)_4$ (23 mg, 0.02 mmol) and a 1 M $K_2CO_3$ aqueous solution (1 mL) were dissolved in 1,4-dioxane (2 mL), the reaction system was heated to 110°C, and a reaction solution was subjected to a reaction under the protection of nitrogen for 15 min. The reaction solution was concentrated to obtain a crude product. Then, the crude product was separated by a high performance liquid chromatography column to obtain a white solid compound 27-5 (30 mg, yield: 24%). LCMS $(M+H)^+$ = 656.2.

**Step 3**

The compound 27-5 (30 mg, 0.04 mmol) was dissolved in DCM (2 mL), and TFA (0.5 mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 27 (20.5 mg, yield: 82%. LCMS $(M+H)^+$ = 556.3. $^1H$ NMR (400 MHz, DMSO) δ 8.43 (t, J = 6.0 Hz, 1H), 8.22 (s, 2H), 8.14 (d, J = 9.7 Hz, 1H), 7.91 - 7.84 (m, 1H), 7.79 (d, J = 10.8 Hz, 1H), 7.60 (d, J = 4.6 Hz, 1H), 7.45 (d, J = 4.8 Hz, 2H), 5.78 (s, 1H), 4.73 (s, 2H), 3.98 (s, 2H), 3.71 (s, 1H), 2.32 (d, J = 7.5 Hz, 1H), 1.95 (d, J = 11.1 Hz, 1H), 1.85 (s, 2H), 1.73 (d, J = 11.6 Hz, 1H), 1.45 (d, J = 11.8 Hz, 1H), 1.25 (s, 6H). $^{19}F$ NMR (376 MHz, DMSO) δ -74.23 (s, 4F), -107.77 (s, 1F), -115.39 (s, 1F).

**Embodiment 28**

Synthesis of ((*1R*,3r,*5S*)-8-(2-(4-cyano-3-fluorophenyl)-3-(6-fluoro-1-(2-hydroxy-2-methylpropyl)-1H-benzo[d][1,2,3]triazol-5-yl)imidazo[1,2-a]pyrazin-8-yl)-8-azabicyclo[3.2.1] octane-3-yl)carbamate (compound 28)

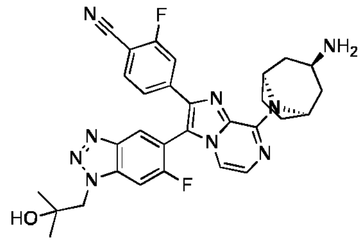

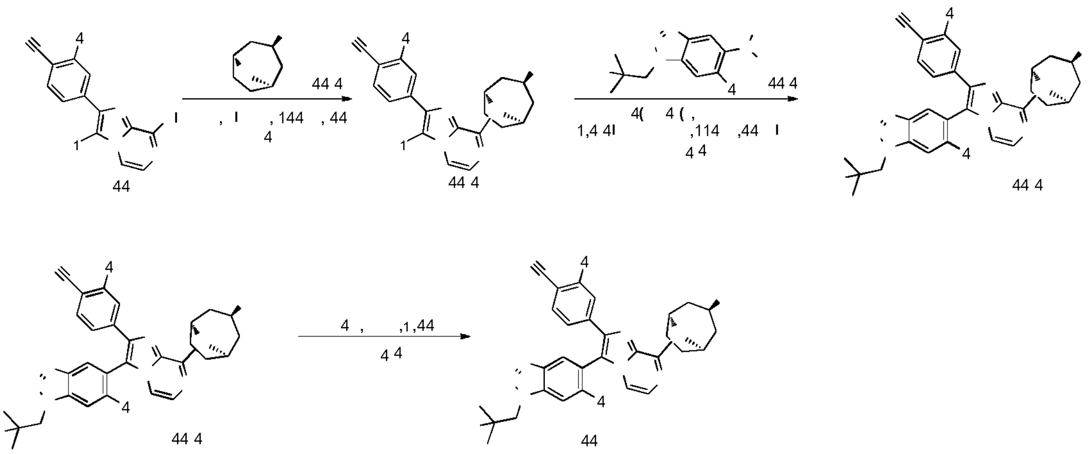

**Step 1**

A compound 28-1 (387 mg, 1.11 mmol), a compound 28 -2 (250 mg, 1.11 mmol) and $K_2CO_3$ (306 mg, 2.22 mmol) were dissolved in DMF (8 mL), and the reaction system was heated to 120°C and reacted for 2 h. The reaction system was quenched with water. Extraction was conducted with ethyl acetate (3*100 mL), drying was conducted with anhydrous sodium sulfate, and spin-drying was conducted. Then, column chromatography was conducted (PE:EA=1:2) to obtain a white solid compound 28-3 (100 mg, yield: 17%). LCMS $(M+H)^+$ = 541.1.

**Step 2**

The compound 28-3 (100 mg, 0.19 mmol), a compound 28-4 (70 mg, 0.28 mmol), $Pd(PPh_3)_4$ (23 mg, 0.02 mmol) and a 1 M $K_2CO_3$ aqueous solution (1 mL) were dissolved in 1,4-dioxane (2 mL), the reaction system was heated to 110°C, and a reaction solution was subjected to a reaction under the protection of nitrogen for 30 min. The reaction solution was concentrated to obtain a crude product. Then, the crude product was separated by a high performance liquid chromatography column to obtain a white solid compound 28-5 (12 mg, yield: 9%). LCMS $(M+H)^+$ = 670.2.

**Step 3**

The compound 28-5 (12 mg, 0.02 mmol) was dissolved in DCM (2 mL), and TFA (0.5 mL) was dropped under an ice water bath. A reaction solution was subjected to a reaction at room temperature for 2 h. The reaction solution was concentrated to obtain a crude product. Then, the crude product was separated by a high performance liquid chromatography column to obtain a white solid compound 28 (8.9 mg, yield: 87%. LCMS $(M+H)^+$ = 570.3. $^1$H NMR (400 MHz, DMSO) $\delta$ 8.43 (d, J = 6.3 Hz, 1H), 8.08 (d, J = 9.2 Hz, 1H), 7.85 (d, J = 7.4 Hz, 3H), 7.74 (d, J = 11.4 Hz, 1H), 7.45 (d, J = 8.6 Hz, 1H), 7.39 (dd, J = 10.8, 4.7 Hz, 2H), 4.96 (s, 1H), 4.69 (s, 2H), 3.28 - 3.24 (m, 1H), 2.67 (s, 1H), 2.33 (s, 1H), 2.19 (s, 2H), 2.02 (s, 2H), 1.76 (d, J = 14.8 Hz, 2H), 1.26 (s, 3H), 1.20 (s, 3H). $^{19}$F NMR (376 MHz, DMSO) $\delta$ -73.8 (s, 9F), -107.73 (s, 1F), -114.27 (s, 1F).

**Embodiment 29**

Synthesis of (*R*)-4-(8-(3-aminopiperidin-1-yl)-3-(6-fluoro-1-methyl-1H-benzo[d][1,2,3]triazol-5-yl)imidazo[1,2-a]pyrazin-2-yl)-2-fluorobenzonitrile (compound 30)

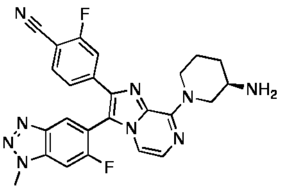

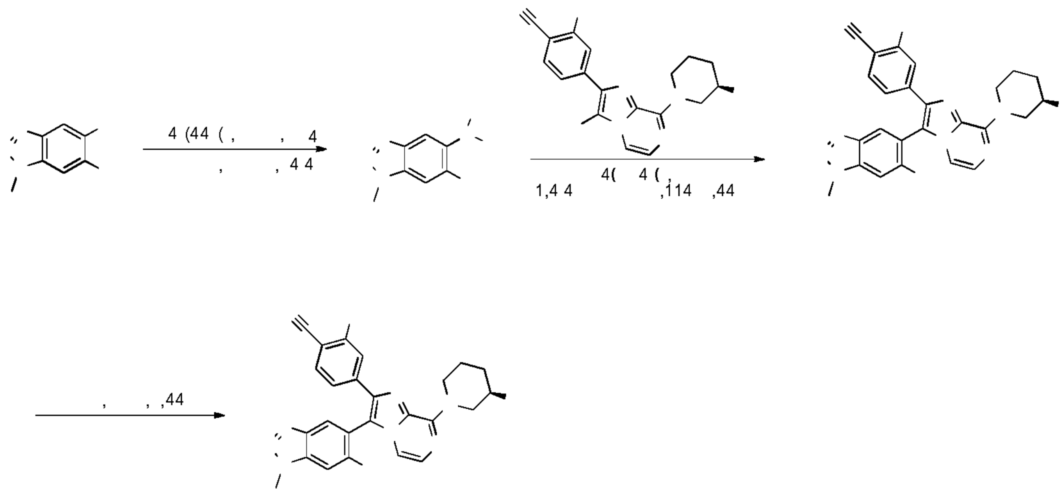

**Step 1**

A compound 30-1 (130 mg, 0.56 mmol), bis(pinacolato)diboron (286 mg, 1.12 mmol), $Pd_2(dba)_3$ (64 mg, 0.06 mmol), tricyclohexylphosphine (40 mg, 0.12 mmol) and potassium acetate (117 mg, 1.12 mmol) were dissolved in 1,4-dioxane (15 mL), the reaction system was heated to 110°C, and a reaction solution was subjected to a reaction under the protection of nitrogen for 12 h. The reaction solution was concentrated to obtain a crude product. Then, the crude product was separated by a high performance liquid chromatography column to obtain a white solid compound 30-2 (85 mg, yield: 78%). LCMS $(M+H)^+$ = 196.0.

**Step 2**

The compound 30-2 (85 mg, 0.45 mmol), a compound 30-3 (154 mg, 0.3 mmol), $Pd(PPh_3)_4$ (35 mg, 0.03 mmol) and $K_2CO_3$ (83 mg, 0.6 mmol) were dissolved in 1,4-dioxane/$H_2O$ (4/0.5 mL), the reaction system was heated to 110°C, and a reaction solution was subjected to a reaction under the protection of nitrogen for 30 min. The reaction solution was concentrated to obtain a crude product. Then, the crude product was separated by a high performance liquid

chromatography column to obtain a white solid compound 30-4 (25 mg, yield: 14%). LCMS $(M+H)^+$ = 586.2.

**Step 3**

The compound 30-4 (25 mg, 0.04 mmol) was dissolved in DCM (2 mL), and TFA (0.5 mL) was dropped under an ice water bath. A reaction solution was subjected to a reaction at room temperature for 2 h. The reaction solution was concentrated to obtain a crude product. Then, the crude product was separated by a high performance liquid chromatography column to obtain of a white solid compound 30 (18 mg, yield: 93%). LCMS $(M+H)^+$ = 486.2. $^1$H NMR (400 MHz, DMSO) δ 8.48 - 8.43 (m, 1H), 8.17 (d, J = 9.1 Hz, 1H), 8.07 (s, 2H), 7.82 (dd, J = 16.7, 9.2 Hz, 2H), 7.42 (d, J = 4.9 Hz, 3H), 5.03 (dd, J = 43.4, 15.6 Hz, 2H), 4.39 (s, 3H), 3.68 (d, J = 10.6 Hz, 2H), 3.39 (s, 1H), 2.09 (s, 1H), 1.92 (s, 1H), 1.71 (s, 2H). $^{19}$F NMR (376 MHz, DMSO) δ -73.87 (s, 3F), -107.68 (s, 1F), -114.31 (s, 1F).

**Activity test embodiments**

In the following embodiments, the LSD1 inhibitory activity and pharmacokinetic properties of compounds of this application are tested by the applicant with some of the compounds of this application as examples.

**Embodiment A: LSD1 inhibitory activity**

This experiment was carried out to test the in vitro inhibitory activity of the compounds of this application against LSD1.

Experimental steps and a method: In this experiment, the in vitro inhibitory activity of the compounds of this application against LSD1 on an enzyme level was tested by using an AlphaScreen method. The experimental steps are described as follows:

a) preparation of a 1x buffer:
preparing a 1x buffer (improved trihydroxymethylaminomethane buffer);
b) continuous dilution of the compounds:
transferring the compounds of this application to a porous plate by using Echo at a final DMSO concentration of 1%;
c) preparation of an enzyme solution:
preparing an enzyme solution in the 1x buffer;
d) preparation of a substrate solution:
adding polypeptide to the 1x buffer to prepare a substrate solution;
e) transferring 5 μL of the enzyme solution or the 1x buffer to the porous plate;
f) conducting incubation at room temperature for 15 min;
g) adding 5 μL of the substrate solution to each well to induce a reaction;
h) conducting incubation at room temperature for 40 min;
i) preparing a 1x Alphalisa buffer;
j) preparation of a 1x Alphalisa buffer solution of receptors and donors:
adding 15 μL of a receptor and donor solution in dark light environment, and conducting incubation at room temperature for 60 min;
k) reading an endpoint in an EnSpire Alpha mode; and
l) data processing:

calculating an inhibitory value according to an equation 1:

$$\text{equation 1: Inh\%=( Max-Signal)/(Max-Min)*100;}$$

and
calculating an $IC_{50}$ value by using XL-Fit according to an equation 2:

$$\text{equation 2: Y=Bottom+(Top-Bottom)/(1+(IC50/X)*HillSlope),}$$

where Y indicates the inhibitory rate, and X indicates the concentration of a compound.

Table 1 shows experimental data of the LSD1 inhibitory activity of some compounds of this application.

Table 1:

| Compound | LSD1 $IC_{50}$ (nM) |
|---|---|
| 1 | 7.4 |
| 2 | 1.3 |
| 3 | 2.6 |
| 4 | 1.0 |
| 5 | 2.3 |
| 6 | 45 |
| 7 | 12 |
| 8 | 1.7 |
| 9 | 0.88 |
| 10 | 7.5 |
| 11 | 0.97 |
| 12 | 1.6 |
| 13 | 56 |
| 14 | 37 |
| 15 | 11 |
| 16 | 15 |
| 17 | 6.3 |
| 18 | 2.9 |
| 19 | 0.71 |
| 20 | 2.6 |
| 21 | 2.3 |
| 22 | 2.5 |
| 23 | 4.4 |
| 26 | 0.7 |
| 27 | 1.0 |
| 28 | 5.5 |
| 30 | 1.0 |

The experimental results show that the compounds of this application have good LSD1 inhibitory activity.

**Embodiment B: Evaluation of pharmacokinetics of the compound of this application after intravenous injection or oral quantitative administration in mice**

This experiment was carried out to test pharmacokinetic properties of the compounds of this application in mice.

Experimental steps and a method:

A tested compound was dissolved in a mixture of 10% DMSO, 10% Solutol HS15 and 80% normal saline, and subjected to vortex treatment and ultrasonic treatment to obtain a corresponding concentration of a clarified solution. Then, the clarified solution was filtered with a microporous filter membrane for later use. A resulting tested compound solution was injected intravenously to CD-1 mice with a body weight of 21 to 27 g at a dose of 5 mg/kg. A tested compound

was dissolved in a mixture of 10% DMSO, 10% Solutol HS15 and 80% normal saline, and subjected to vortex treatment and ultrasonic treatment to obtain a corresponding concentration of a clarified solution. Then, the clarified solution was filtered with a microporous filter membrane for later use. A resulting tested compound solution was administered orally to CD-1 mice with a body weight of 21 to 27 g at a dose of 5 mg/kg. Whole blood was collected at certain time points, and plasma was prepared. Drug concentration was analyzed by an LC-MS/MS method, and pharmacokinetic parameters were calculated by Phoenix WinNonlin software.

Experimental results of the compound 27 are as shown in Table 2.

Table 2:

| Administration mode | Blood drug concentratio n | Curve area | Half-lif e | Clearance rate | Apparent volume of distribution |
|---|---|---|---|---|---|
| | Cmax (ng/mL) | $AUC_{0-t}$ (ng/mL* h) | $T_{1/2}$ (h) | CL (ml/min/kg) | V (L/kg) |
| Oral administration | 648 | 6528 | 5.86 | n/a | n/a |
| Administration by intravenous injection | n/a | 11502 | 8.33 | 7.25 | 5.04 |
| Note: n/a indicates undetected. | | | | | |

The experimental results show that the compound of this application has high exposure in to-be-tested animals, good absorption and obvious advantages in pharmacokinetic properties.

**Embodiment C: hERG inhibitory activity**

This experiment was carried out to test the in vitro inhibitory activity of the compounds of this application against hERG.

Experimental steps and method:

In this method, whole cell recording was conducted by using a HEKA EPC 10 USB patch clamp amplifier (HEKA Elektronik, Germany). A cover slide with a large number of individual CHO hERG cells on the surface was removed and placed in a recording tank which was treated with continuous perfusion (about 1 ml/min) and loaded into an inverted microscope. Currents of hERG channels of the individual cells were recorded by using a standard whole cell recording method. A fixed voltage of -80 mV was applied to the cells by a voltage clamp. The hERG currents were activated by depolarization at +20 mV for 5 s, and then the currents were changed back at -50 mV for 5 s to remove a passivation effect and observe deactivated tail currents. Potassium ion tail currents observed in the hERG channels were stabilized by continuous perfusion in this step. Then, the cells were treated with a drug of this application until a stable state was reached. A stable state was considered as being reached when three consecutive superimposed currents were recorded. At this time point, the cells were treated again with an extracellular solvent until the current amplitude was changed back to near the level before administration. Cisapride was used in a control experiment to verify that the mass and response of hERG cells were in a normal state.

Table 3 shows experimental data of the hERG inhibitory activity of some compounds of this application.

Table 3:

| Compound | hERG inhibitory rate (%@10 μM) |
|---|---|
| 12 | 27.01 |
| 18 | 43.72 |
| 20 | 26.74 |

The experimental results show that the compounds of this application have low hERG inhibitory activity.

In addition, the terms "first" and "second" are used merely for the purpose of description, and shall not be construed as indicating or implying relative importance or implying a quantity of indicated technical features. Therefore, a feature restricted by "first" or "second" may explicitly indicate or implicitly include at least one of such features. In

description of this application, "a variety of" means at least two, such as two and three unless it is specifically defined otherwise.

In descriptions of this specification, a description of a reference term such as "an embodiment", "some embodiments", "an example", "a specific example", or "some examples" means that a specific feature, structure, material, or characteristic that is described with reference to the embodiment or the example is included in at least one embodiment or example of this application. In this specification, schematic descriptions of the foregoing terms are not necessarily directed at the same embodiment or example. Besides, the specific feature, structure, material or characteristic described may be integrated in a proper manner in any one or more embodiments or examples. In addition, different embodiments or examples described in the specification and features of the different embodiments or examples may be integrated or combined by a person skilled in the art without being contradictory to each other.

Although the embodiments of this application are shown and described above, it may be understood that, the foregoing embodiments are exemplary, and may not be construed as limitations to this application. Within the scope of this application, a person of ordinary skill in the art may make changes, modifications, substitutions and variations to the foregoing embodiments.

## Claims

1. A compound, being a compound as shown in Formula (I) or a stereoisomer, tautomer, deuterated compound, oxynitride, solvate, metabolite, pharmaceutically acceptable salt or prodrug of the compound as shown in Formula (I):

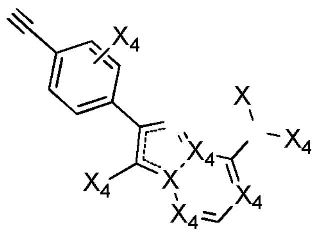

(I)

wherein

X is C, N, or O;

$X_1$, $X_2$, $X_3$, and $X_4$ are respectively selected from C or N, provided that $X_1$ is different from $X_2$, $X_1$ is different from $X_4$, and $X_2$ is different from $X_3$;

$R_1$ and $R_2$ are independently H, deuterium, $C_{1-6}$ alkyl, $C_{3-12}$ carbocyclyl, $C_{3-12}$ carbocyclyl-$C_{1-4}$ alkylene, heterocyclyl composed of 3-12 atoms, (heterocyclyl composed of 3-12 atoms)-$C_{1-4}$ alkylene, $C_{6-10}$ aryl, $C_{6-10}$ aryl-$C_{1-4}$ alkylene, heteroaryl composed of 5-14 atoms, or (heteroaryl composed of 5-14 atoms)-$C_{1-4}$ alkylene, or $R_1$, $R_1$ and X connected thereto form a $C_{3-12}$ carbon ring or a heterocyclic ring composed of 3-12 atoms, wherein the $C_{1-6}$ alkyl, the $C_{3-12}$ carbocyclyl, the $C_{3-12}$ carbocyclyl-$C_{1-4}$ alkylene, the heterocyclyl composed of 3-12 atoms, the (heterocyclyl composed of 3-12 atoms)-$C_{1-4}$ alkylene, the $C_{6-10}$ aryl, the $C_{6-10}$ aryl-$C_{1-4}$ alkylene, the heteroaryl composed of 5-14 atoms, the (heteroaryl composed of 5-14 atoms)-$C_{1-4}$ alkylene, or the $C_{3-12}$ carbon ring or the heterocyclic ring composed of 3-12 atoms formed by $R_1$, $R_2$ and X connected thereto are independently unsubstituted or substituted with 1, 2, 3, 4, or 5 R';

$R_3$ is $C_{6-10}$ aryl or heteroaryl composed of 5-10 atoms, wherein the $C_{6-10}$ aryl and the heteroaryl composed of 5-10 atoms are independently unsubstituted or substituted with 1, 2, 3, or 4R";

$R_4$ is H, deuterium, F, Cl, Br, CN, $NO_2$, $-OR^a$, $-NR^bR^c$, or $C_{1-6}$ alkyl;

R' and R" are independently H, deuterium, F, Cl, Br, CN, $NO_2$, =O, $-OR^a$, $-NR^bR^c$, $-S(=O)_2R^a$, $C_{1-6}$ alkyl, $C_{3-8}$ cycloalkyl, $C_{3-8}$ cycloalkenyl, or heterocyclyl composed of 3-12 atoms, wherein the $C_{1-6}$ alkyl, the $C_{3-8}$ cycloalkyl, the $C_{3-8}$ cycloalkenyl and the heterocyclyl composed of 3-12 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$; and

$R^a$, $R^b$, and $R^c$ are independently H, deuterium, $C_{1-6}$ alkyl, $C_{1-6}$ haloalkyl, or heterocyclyl composed of 3-6 atoms, or $R^b$, $R^c$ and nitrogen atoms connected thereto form a heterocyclic ring composed of 3-6 atoms, wherein the $C_{1-6}$ alkyl and the heterocyclic ring composed of 3-6 atoms are independently unsubstituted or substituted with 1, 2, 3, or 4 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

**2.** The compound according to claim 1, having a structure as shown in Formula (II) or (III):

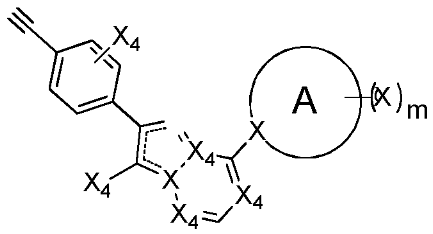 (II), 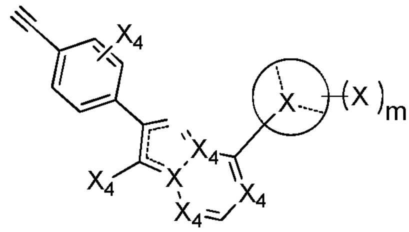 (III),

wherein ring A is a $C_{3-6}$ carbon ring or a heterocyclic ring composed of 3-6 atoms; ring B is a bridge ring composed of 6-8 atoms; and
m is 0, 1, 2, 3, 4, or 5.

**3.** The compound according to claim 1, having a structure as shown in Formula (IV), Formula (V), Formula (VI), or Formula (VII):

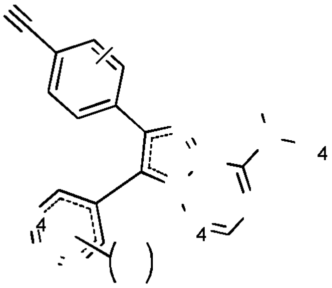 (IV), 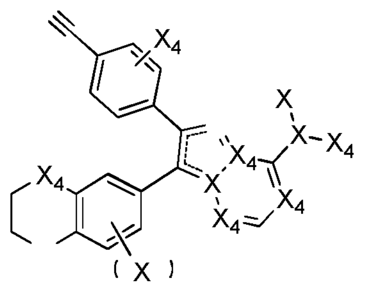 (V),

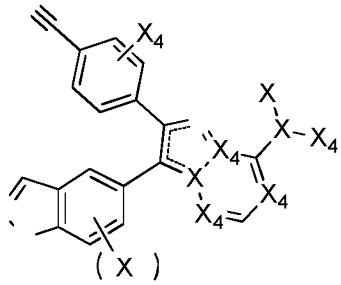 (VI), 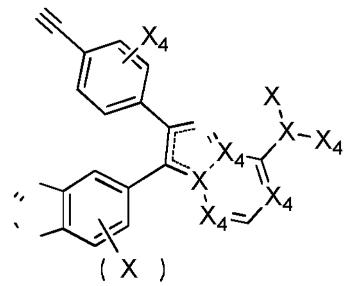 (VII),

wherein $X_5$ is empty or C; $X_6$ and $X_7$ are independently selected from C or N respectively; $X_8$ is O, S, C, or N; and n is 0, 1, 2, 3, or 4.

**4.** The compound according to claim 1, having a structure as shown in Formula (VIII) or Formula (IX):

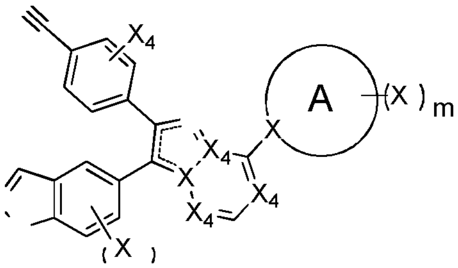 (VIII), 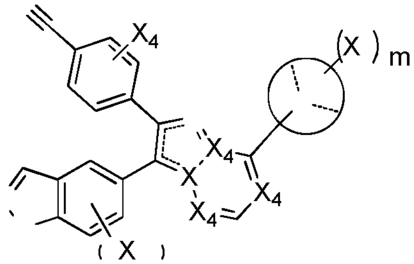 (IX),

wherein ring A is a $C_{3-6}$ carbon ring or a heterocyclic ring composed of 3-6 atoms;

ring B is a bridge ring composed of 6-8 atoms;
m is 0, 1, 2, 3, 4, or 5;
n is 1, 2, 3, or 4; and
R" is $C_{1-6}$ alkyl or $C_{3-8}$ cycloalkyl, wherein the $C_{1-6}$ alkyl and the $C_{3-8}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

**5.** The compound according to claim 1, having a structure as shown in Formula (X) or Formula (XI):

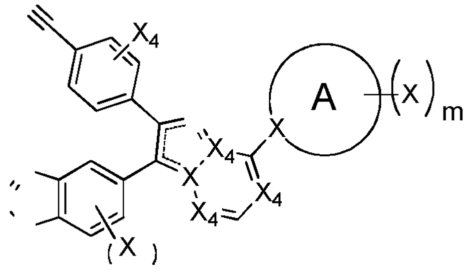
(X),
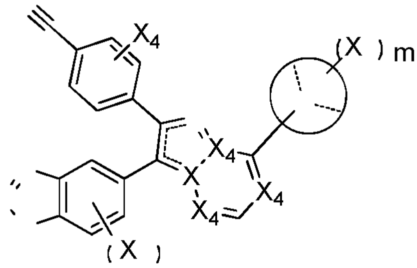
(XI),

wherein ring A is a $C_{3-6}$ carbon ring or a heterocyclic ring composed of 3-6 atoms;
ring B is a bridge ring composed of 6-8 atoms;
m is 0, 1, 2, 3, 4, or 5; and
n is 0, 1, 2, 3, or 4.

**6.** The compound according to any one of claims 1 to 5, wherein X is N.

**7.** The compound according to claim 1 or 3, wherein $R_1$ and $R_2$ are independently H, deuterium, $C_{1-6}$ alkyl, $C_{3-6}$ carbocyclyl, $C_{3-6}$ carbocyclyl-$C_{1-4}$ alkylene, heterocyclyl composed of 3-6 atoms, (heterocyclyl composed of 3-6 atoms)-$C_{1-4}$ alkylene, $C_{6-8}$ aryl, $C_{6-8}$ aryl-$C_{1-4}$ alkylene, heteroaryl composed of 5-8 atoms, or (heteroaryl composed of 5-8 atoms)-$C_{1-4}$ alkylene, or $R_1$, $R_2$ and X connected thereto form a $C_{3-8}$ carbon ring or a heterocyclic ring composed of 3-8 atoms, wherein the $C_{1-6}$ alkyl, the $C_{3-6}$ carbocyclyl, the $C_{3-6}$ carbocyclyl-$C_{1-4}$ alkylene, the heterocyclyl composed of 3-6 atoms, the (heterocyclyl composed of 3-6 atoms)-$C_{1-4}$ alkylene, the $C_{6-8}$ aryl, the $C_{6-8}$ aryl-$C_{1-4}$ alkylene, the heteroaryl composed of 5-8 atoms, the (heteroaryl composed of 5-8 atoms)-$C_{1-4}$ alkylene, or the $C_{3-8}$ carbon ring or the heterocyclic ring composed of 3-8 atoms formed by $R_1$, $R_2$ and X connected thereto are independently unsubstituted or substituted with 1, 2, or 3 R'.

**8.** The compound according to claim 1 or 2, wherein $R_3$ is $C_{6-9}$ aryl or heteroaryl composed of 5-9 atoms, and wherein the $C_{6-9}$ aryl and the heteroaryl composed of 5-9 atoms are independently unsubstituted or substituted with 1 or 2 R".

**9.** The compound according to any one of claims 1 to 5, wherein $R_4$ is H, deuterium, F, Cl, Br, CN, $NO_2$, -OH, $-NH_2$, or $C_{1-3}$ alkyl.

**10.** The compound according to any one of claims 1 to 5, wherein R' is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, or heterocyclyl composed of 3-6 atoms, wherein the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{1-6}$ haloalkyl, or $-CO-NR^bR^c$.

**11.** The compound according to any one of claims 1 to 3 and claim 5, whereinR" is independently H, deuterium, F, Cl, Br, CN, $NO_2$, =O, $-OR^a$, $-NR^bR^c$, $-S(=O)OR^a$, $C_{1-3}$ alkyl, $C_{3-6}$ cycloalkyl, or heterocyclyl composed of 3-6 atoms, wherein the $C_{1-3}$ alkyl, the $C_{3-6}$ cycloalkyl and the heterocyclyl composed of 3-6 atoms are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

**12.** The compound according to claim 4, wherein R" is $C_{1-3}$ alkyl or $C_{3-6}$ cycloalkyl, wherein the $C_{1-3}$ alkyl and the $C_{3-6}$ cycloalkyl are independently unsubstituted or substituted with 1, 2, 3, or 4 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl,

or -CO-NR^bR^c.

**13.** The compound according to claim 1 or 3, wherein $R_1$ and $R_2$ are independently H, deuterium, $C_{1-6}$ alkyl, $C_{3-6}$ carbocyclyl, or heterocyclyl composed of 3-6 atoms, or $R_1$, $R_2$ and X connected thereto form a $C_{3-8}$ carbon ring or a heterocyclic ring composed of 3-8 atoms, wherein the $C_{1-6}$ alkyl, the $C_{3-6}$ carbocyclyl, the heterocyclyl composed of 3-6 atoms, or the $C_{3-8}$ carbon ring or the heterocyclic ring composed of 3-8 atoms formed by $R_1$, $R_2$ and X connected thereto are independently unsubstituted or substituted with 1, 2, or 3 R'.

**14.** The compound according to claim 1 or 2, wherein $R_3$ is phenyl, naphthyl, pyrrolyl, pyridyl, pyrazolyl, imidazolyl, triazolyl, tetrazolyl, oxazolyl, oxadiazolyl, 1,3,5-triazinyl, thiazolyl, thienyl, pyrazinyl, pyridazinyl, pyrimidinyl, indolyl, purinyl, quinolinyl, isoquinolinyl, or phenothianyl, wherein the phenyl, the naphthyl, the pyrrolyl, the pyridyl, the pyrazolyl, the imidazolyl, the triazolyl, the tetrazolyl, the oxazolyl, the oxadiazolyl, the 1,3,5-triazinyl, the thiazolyl, the thienyl, the pyrazinyl, the pyridazinyl, the pyrimidinyl, the indolyl, the purinyl, the quinolinyl, the isoquinolinyl and the phenothianyl are independently unsubstituted or substituted with 1 or 2 R".

**15.** The compound according to any one of claims 1 to 5, wherein $R_4$ is F, Cl, or Br.

**16.** The compound according to any one of claims 1 to 5, wherein R' is independently H, deuterium, F, Cl, Br, or $-NR^bR^c$.

**17.** The compound according to any one of claims 1 to 3 and 5, wherein R" is independently H, deuterium, F, Cl, Br, CN, $NO_2$, =O, $-OR^a$, $-NR^bR^c$, $C_{1-3}$ alkyl, or $C_{3-6}$ cycloalkyl, wherein the $C_{1-3}$ alkyl and the $C_{3-6}$ cycloalkyl are independently unsubstituted or substituted with 1, 2, or 3 substituents, and the substituents are independently selected from deuterium, F, Cl, Br, CN, =O, $-OR^a$, $-NR^bR^c$, $C_{1-6}$ alkyl, $C_{3-6}$ cycloalkyl, $C_{1-6}$ haloalkyl, or $-CO-NR^bR^c$.

**18.** The compound according to claim 4, wherein R" is $C_{1-3}$ alkyl or $C_{3-6}$ cycloalkyl,
wherein the $C_{1-3}$ alkyl and the $C_{3-6}$ cycloalkyl are independently unsubstituted or substituted with 1 or 2 substituents, and the substituents are independently selected from deuterium, F, Cl, Br, CN, =O, $--OR^a$, $-NR^bR^c$, $C_{1-6}$ alkyl, $C_{3-6}$ cycloalkyl, $C_{1-6}$ haloalkyl, or $-CO-NR^bR^c$.

**19.** The compound according to any one of claims 1 to 18, wherein $R^a$, $R^b$, and $R^c$ are independently H, deuterium, methyl, ethyl, isopropyl, n-propyl, n-butyl, tert-butyl, $C_{1-3}$ haloalkyl, or heterocyclyl composed of 3-6 atoms, or $R^b$, $R^c$ and nitrogen atoms connected thereto form a heterocyclic ring composed of 3-6 atoms, wherein the methyl, the ethyl, the isopropyl, the n-propyl, the n-butyl, the tert-butyl and the heterocyclic ring composed of 3-6 atoms are independently unsubstituted or substituted with 1, 2, or 3 substituents, and the substituents are independently selected from deuterium, F, Cl, CN, OH, $NH_2$, $C_{1-6}$ alkyl, $C_{1-6}$ haloalkyl, $C_{1-6}$ alkoxyl, or $C_{1-6}$ alkylamino.

**20.** The compound according to claim 1, having one of the following structures:

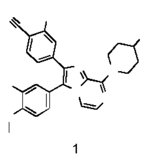
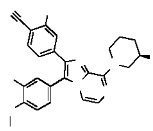
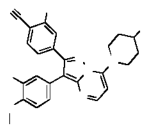
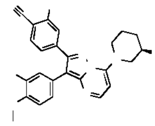
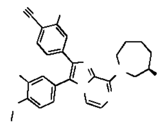
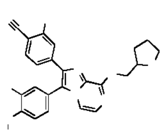
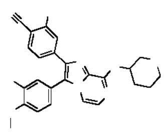
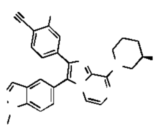
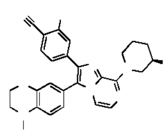
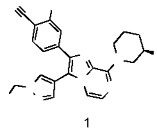
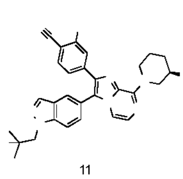
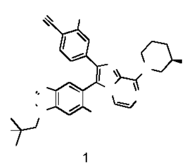
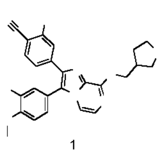
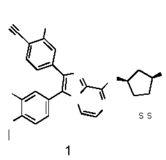
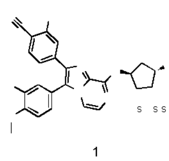

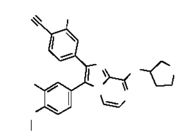

1

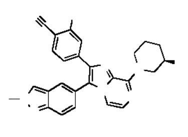

1

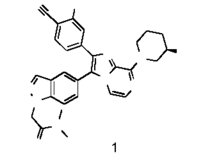

1

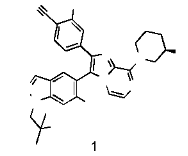

1

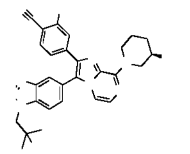

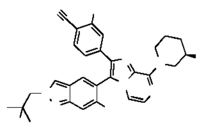

1

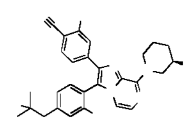

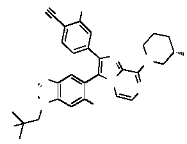

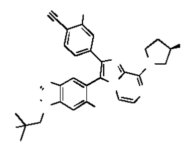

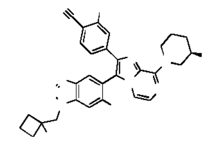

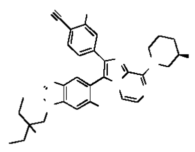

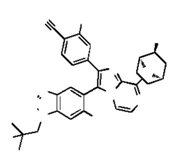

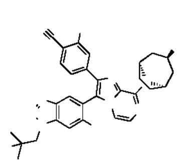

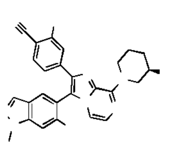

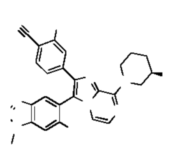

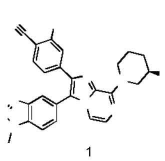

1

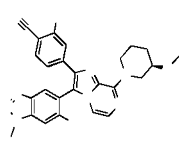

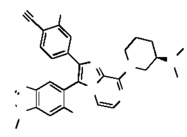

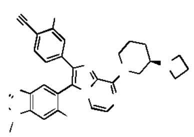

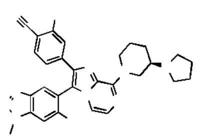

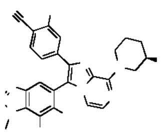

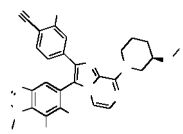

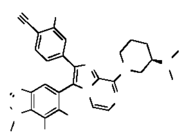

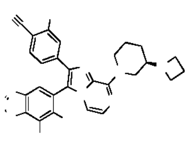

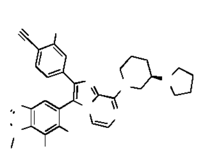

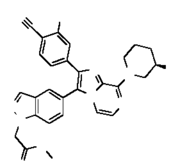

1

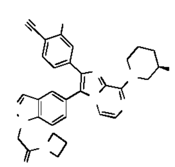

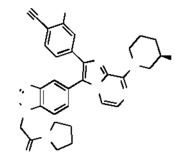

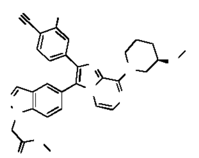

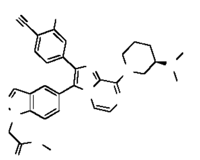

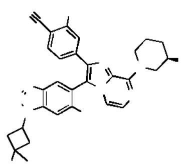

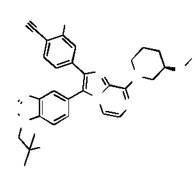

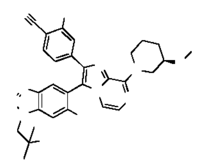

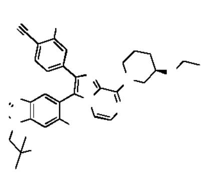

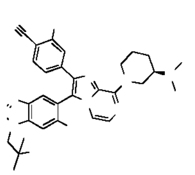

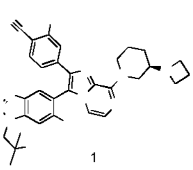

1

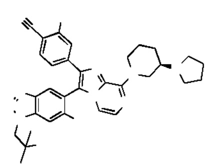

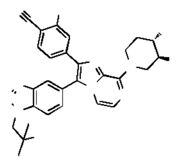

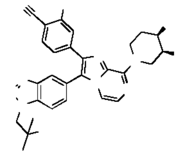 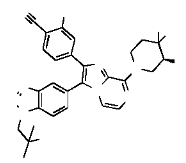 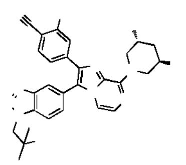 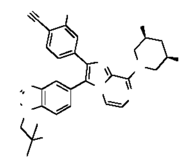 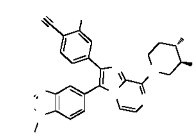

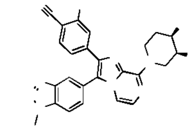 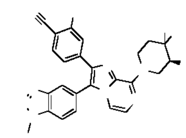 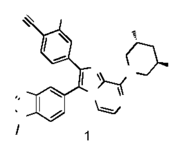 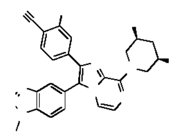 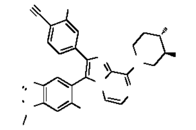

1

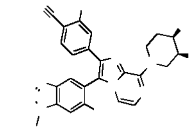 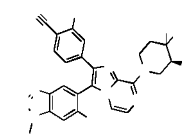 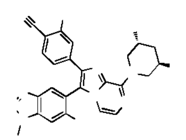 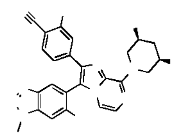 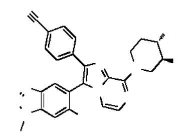

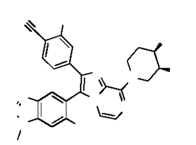 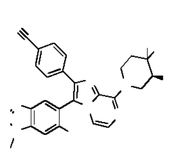 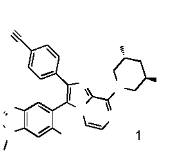 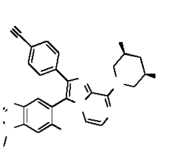 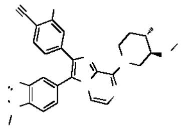

1 ,

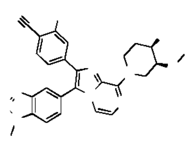 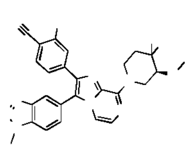 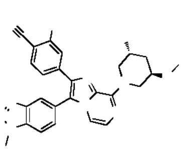 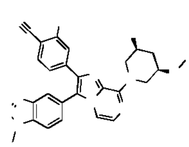 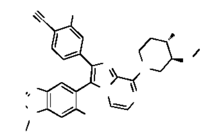

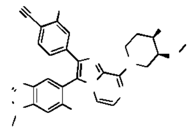 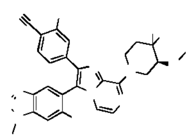 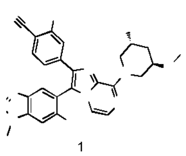 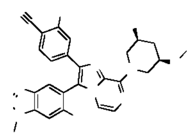 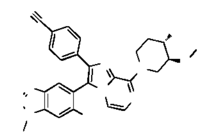

1

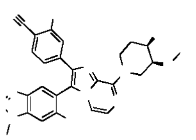 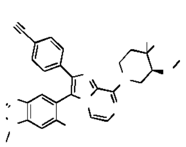 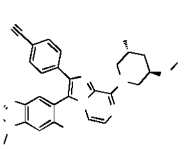 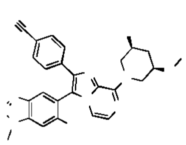 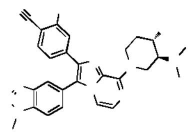

,

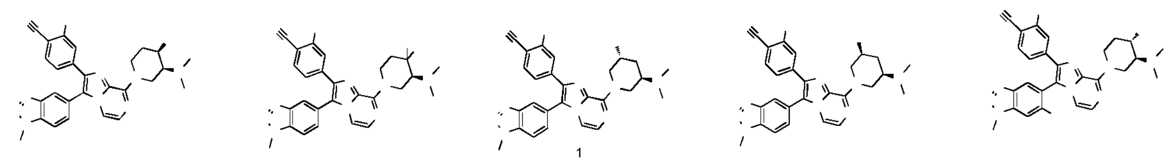

1

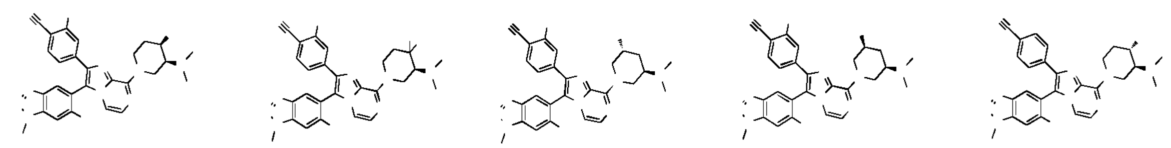

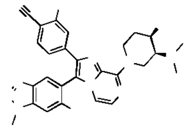 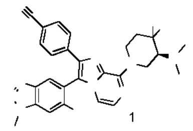 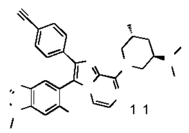 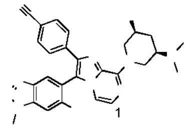 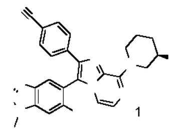 ,

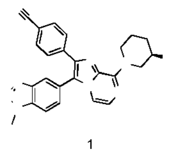 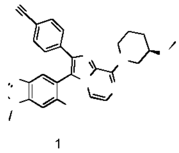 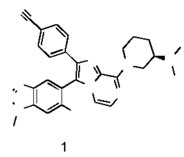 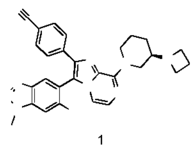 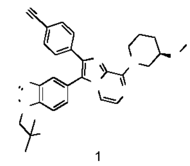

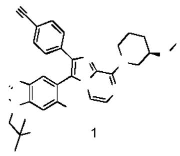 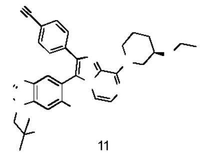 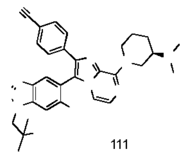 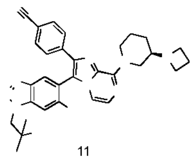 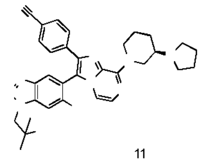 ,

or being a stereoisomer, tautomer, oxynitride, solvate, metabolite, pharmaceutically acceptable salt or prodrug thereof.

**21.** A pharmaceutical composition, comprising an effective amount of the compound according to any one of claims 1 to 20.

**22.** The pharmaceutical composition according to claim 21, wherein the pharmaceutical composition further comprises: a pharmaceutically acceptable carrier, an adjuvant, a medium or a combination thereof.

**23.** The pharmaceutical composition according to claim 21 or 22, wherein the pharmaceutical composition further comprises one or more therapeutic agents, and the therapeutic agent is selected from other anti-tumor drugs.

**24.** The pharmaceutical composition according to claim 23, wherein the therapeutic agent is an anti-mitotic drug, an alkylating agent, an anti-metabolic drug, a topoisomerase inhibitor, an estrogen receptor regulator, an androgen receptor regulator, a small-molecule inhibitor targeting protein kinase, and an antibody drug targeting protein kinase.

**25.** The pharmaceutical composition according to claim 24, wherein the anti-mitotic drug is paclitaxel, vincristine.

**26.** The pharmaceutical composition according to claim 24, wherein the alkylating agent is cisplatin, oxaliplatin, carboplatin, or cyclophosphamide.

**27.** The pharmaceutical composition according to claim 24, wherein the anti-metabolic drug is gemcitabine, 5-fluorouracil, or methotrexate.

**28.** The pharmaceutical composition according to claim 24, wherein the topoisomerase inhibitor is epipodophyllotoxin, etoposide, topotecan, or camptothecin.

**29.** The pharmaceutical composition according to claim 24, wherein the estrogen receptor regulator is tamoxifen or fulvestrant.

**30.** The pharmaceutical composition according to claim 24, wherein the androgen receptor regulator is bicalutamide.

**31.** The pharmaceutical composition according to claim 24, wherein the small-molecule inhibitor targeting protein kinase is dasatinib, bosutinib, gefitinib, erlotinib, lapatinib, imatinib, nilotinib, sorafenib, tipifarnib, sunitinib, axitinib.

**32.** The pharmaceutical composition according to claim 24, wherein the antibody drug targeting protein kinase is trastuzumab, panitumumab, cetuximab.

**33.** Use of the compound according to any one of claims 1 to 20 or the pharmaceutical composition according to any one of claims 21 to 32 in preparation of a medicine, wherein the medicine is used for preventing, disposing, treating, or alleviating a disease associated with overexpression or hyperactivity of LSD1 in a patient.

**34.** The use according to claim 34, wherein the disease associated with overexpression or hyperactivity of LSD1 is a tumor.

**35.** The use according to claim 34, wherein the tumor is papillary thyroid carcinoma, breast cancer, gastric cancer, bronchogenic carcinoma, lung cancer, acute myeloid leukemia, small cell lung cancer, prostate cancer, or non-hodgkin lymphoma.

**36.** Use of the compound according to any one of claims 1 to 20 or the pharmaceutical composition according to any one of claims 21 to 32 in preparation of a medicine, wherein the medicine is used for inhibiting LSD1.

**37.** A compound according to any one of claims 1 to 20 or a pharmaceutical composition according to any one of claims 21 to 32, being used for preventing, disposing, treating, or alleviating a disease associated with overexpression or hyperactivity of LSD1 in a patient.

**38.** A compound according to any one of claims 1 to 20 or a pharmaceutical composition according to any one of claims 21 to 32, being used for preventing, disposing, treating, or alleviating a tumor.

**39.** A compound according to any one of claims 1 to 20 or a pharmaceutical composition according to any one of claims 21 to 32, being used for preventing, disposing, treating, or alleviating papillary thyroid carcinoma, breast cancer, gastric cancer, bronchogenic carcinoma, lung cancer, acute myeloid leukemia, small cell lung cancer, prostate cancer, or non-hodgkin lymphoma.

**40.** A compound according to any one of claims 1 to 20 or a pharmaceutical composition according to any one of claims 21 to 32, being used for inhibiting LSD1.

**41.** A method for preventing, disposing, treating, or alleviating a disease associated with overexpression or hyperactivity of LSD1 in a patient, comprising administering an effective amount of the compound according to any one of claims 1 to 20 or the pharmaceutical composition according to any one of claims 21 to 32 to the patient.

**42.** The method according to claim 41, wherein the disease associated with overexpression or hyperactivity of LSD1 is a tumor.

**43.** The method according to claim 42, wherein the tumor is papillary thyroid carcinoma, breast cancer, gastric cancer, bronchogenic carcinoma, lung cancer, acute myeloid leukemia, small cell lung cancer, prostate cancer, or non-hodgkin lymphoma.

**44.** A method for inhibiting LSD1, comprising administering the compound according to any one of claims 1 to 20 or the pharmaceutical composition according to any one of claims 21 to 32 to a cell to be treated, wherein the cell to be treated expresses LSD1.

**INTERNATIONAL SEARCH REPORT**

International application No.
**PCT/CN2022/135152**

**A. CLASSIFICATION OF SUBJECT MATTER**

C07D487/04(2006.01)i;A61K31/52(2006.01)i;A61P35/00(2006.01)i

According to International Patent Classification (IPC) or to both national classification and IPC

**B. FIELDS SEARCHED**

Minimum documentation searched (classification system followed by classification symbols)

IPC: C07D487/-， A61K31/-

Documentation searched other than minimum documentation to the extent that such documents are included in the fields searched

Electronic data base consulted during the international search (name of data base and, where practicable, search terms used)

CNKI, CNTXT, VEN, STN: 咪唑并, 吡唑并, 吡嗪, 嘧啶, 肿瘤, 癌, LSD1, imidazo, pyrazine, pyridine, 根据式(I)进行了结构式检索, structural formula search based on formula (I)

**C. DOCUMENTS CONSIDERED TO BE RELEVANT**

| Category* | Citation of document, with indication, where appropriate, of the relevant passages | Relevant to claim No. |
|---|---|---|
| A | TW 201609726 A (INCYTE CORP.) 16 March 2016 (2016-03-16)<br>entire document | 1-40 |
| A | KUMAR, R. et al. "Base-Promoted Transition-Metal-Free Arylation of Imidazo-Fused Heterocycles with Diaryliodonium Salts"<br>*European Journal of Organic Chemistry (2018),* No. no. 14, 31 December 2018 (2018-12-31), pp. 1665-1673 | 1-40 |

☐ Further documents are listed in the continuation of Box C.

☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 February 2023** | **01 March 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/135152**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **41-44**
because they relate to subject matter not required to be searched by this Authority, namely:

   The subject matter of claims 41-44 relates to a treatment method implemented on the human/animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/135152**

| Patent document cited in search report | Publication date (day/month/year) | Patent family member(s) | Publication date (day/month/year) |
|---|---|---|---|
| TW 201609726 A | 16 March 2016 | TWI 687419 B | 11 March 2020 |
| | | WO 2016007731 A1 | 14 January 2016 |
| | | AR 101175 A1 | 30 November 2016 |
| | | US 2019040058 A1 | 07 February 2019 |
| | | US 10640503 B2 | 05 May 2020 |
| | | US 2016009712 A1 | 14 January 2016 |
| | | US 9695168 B2 | 04 July 2017 |
| | | US 2017369487 A1 | 28 December 2017 |
| | | US 10047086 B2 | 14 August 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Chemical Handbook. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007
- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **JERRY MARCH.** Advanced Organic Chemistry. Wiley Interscience
- *Syn. Comm.,* 1977, vol. 7, 509-514
- *J. Pharmaceutical Sciences,* 1977, vol. 66, 1-19
- **T. HIGUCHI ; V STELLA.** Pro-drugs as Novel Delivery Systems. *A. C. S Symposium Series,* vol. 14
- Bioreversible Carriers in Drug Design. American Pharmaceutical Association and Pergamon Press, 1987
- **J. RAUTIO et al.** Prodrugs: Design and Clinical Applications. *Nature Review Drug Discovery,* 2008, vol. 7, 255-270
- **S. J. HECKER et al.** Prodrugs of Phosphates and Phosphonates. *Journal of Medicinal Chemistry,* 2008, vol. 51, 2328-2345
- **JACQUES et al.** Enantiomers, Racemates and Resolutions. Wiley Interscience, 1981
- **ROBERT E. GAWLEY ; JEFFREY AUBÉ.** Principles of Asymmetric Synthesis. Elsevier, 2012
- **ELIEL, E. L.** Stereochemistry of Carbon Compounds. McGraw-Hill, 1962
- **WILEN, S.H.** Tables of Resolving Agents and Optical Resolutions. Univ. of Notre Dame Press, 1972, 268
- Remington's Pharmaceutical Sciences. Mack Printing Company, 1990, 1289-1329
- **T W. GREENE.** Protective Groups in Organic Synthesis. John Wiley & Sons, 1991
- **P. J. KOCIENSKI.** Protecting Groups. Thieme, 2005
- Remington's Pharmaceutical Sciences. Mack Publishing Company, 1985
- **STAHL ; WERMUTH.** Handbook of Medicinal Salts: Properties, Selection, and Use. Wiley-VCH, 2002
- Remington: The Science and Practice of Pharmacy. Lippincott Williams & Wilkins, 2005
- Encyclopedia of Pharmaceutical Technology. Marcel Dekker, 1988